# EUROPEAN PATENT SPECIFICATION

(11) **EP 3 268 024 B1**
(45) Date of publication and mention of the grant of the patent: **09.09.2020**
(21) Application number: 16762530.0
(22) Date of filing: 10.03.2016
(51) Int. Cl.: A61K 38/16, A61K 48/00, A61P 25/00, A61P 25/28

(54) **RECOMBINANT GLUT1 ADENO-ASSOCIATED VIRAL VECTOR CONSTRUCTS AND RELATED METHODS FOR RESTORING GLUT1 EXPRESSION**
REKOMBINANTE GLUT1-ADENO-ASSOZIIERTE VIRUSVEKTORKONSTRUKTE UND ZUGEHÖRIGE VERFAHREN ZUR WIEDERHERSTELLUNG DER GLUT1-EXPRESSION
CONSTRUCTIONS DE VECTEUR VIRAL ADÉNO-ASSOCIÉ GLUT1 DE RECOMBINAISON ET PROCÉDÉS ASSOCIÉS PERMETTANT DE RESTAURER L'EXPRESSION DE GLUT1

(30) Priority: 10.03.2015 US 201562130899 P
(43) Date of publication of application: 17.01.2018
(73) Proprietor: The Trustees of Columbia University in the City of New York, New York, NY 10027 (US); University of Massachusetts, Boston, MA 02108 (US)
(72) Inventor: DE VIVO, Darryl, New York, NY 10065 (US); MONANI, Umrao, Oradell, NJ 07649 (US); GAO, Guangping, Westborough, MA 01581 (US); ENGELSTAD, Kristin, Bronx, NY 10471 (US)
(74) Representative: Elkington and Fife LLP
(86) International application number: PCT/US2016/021810
(87) International publication number: WO 2016/145217

(56) References cited:
- US-A1- 2010 199 362
- US-A1- 2012 177 605
- US-A1- 2012 232 130
- US-B2- 8 945 583
- JÖRG KLEPPER: "Glucose transporter deficiency syndrome (GLUT1DS) and the ketogenic diet", EPILEPSIA, vol. 49, 1 November 2008 (2008-11-01), pages 46-49, XP055485407, NEW YORK, US ISSN: 0013-9580, DOI: 10.1111/j.1528-1167.2008.01833.x
- MAOXUE TANG ET AL: "Brain microvasculature defects and Glut1 deficiency syndrome averted by early repletion of the glucose transporter-1 protein", NATURE COMMUNICATIONS, vol. 8, 20 January 2017 (2017-01-20), page 14152, XP055485145, DOI: 10.1038/ncomms14152
- SACHIE NAKAMURA ET AL: "Gene therapy for a mouse model of glucose transporter-1 deficiency syndrome", MOLECULAR GENETICS AND METABOLISM REPORTS, vol. 10, 15 January 2017 (2017-01-15), pages 67-74, XP055485142, ISSN: 2214-4269, DOI: 10.1016/j.ymgmr.2016.12.008
- DATABASE GENPEPT [Online] 02 February 2008 'GI 166795299: solute carrier family 2, facilitated glucose transporter member 1 [Homo sapiens', XP055310425 Database accession no. NP_006507.2
- SHAH ET AL.: 'The Role of Glucose Transporters in Brain Disease: Diabetes and Alzheimer's Disease' INTERNATIONAL JOURNAL OF MOLECULAR SCIENCES vol. 13, no. 12, 03 October 2012, pages 12629 - 12655, XP055310427

### SEQUENCE LISTING

<110> THE TRUSTEES OF COLUMBIA UNIVERSITY IN THE CITY OF NEW YORK UNIVERSITY OF MASSACHUSETTS MEDICAL CENTER
<120> RECOMBINANT GLUT1 ADENO-ASSOCIATED VIRAL VECTOR CONSTRUCTS AND RELATED METHODS FOR RESTORING GLUT1 EXPRESSION
<130> 01001/003887-WO0
<140>
   <141>
<150> 62/130,899
   <151> 2015-03-10
<160> 97
<170> PatentIn version 3.5
<210> 1
   <211> 7109
   <212> DNA
   <213> Artificial Sequence
<220>
   <223> Description of Artificial Sequence: Synthetic polynucleotide
<220>
   <223> pAAV CB6 PI EGFP-2A-hGlut1
<400> 1
   gccttaatta ggctgcgcgc tcgctcgctc actgaggccg cccgggcaaa gcccgggcgt      60
   cgggcgacct ttggtcgccc ggcctcagtg agcgagcgag cgcgcagaga gggagtggcc     120
   aactccatca ctaggggttc cttgtagtta atgattaacc cgccatgcta cttatctacc     180
   agggtaatgg ggatcctcta gaactatagc tagtcgacat tgattattga ctagttatta     240
   atagtaatca attacggggt cattagttca tagcccatat atggagttcc gcgttacata     300
   acttacggta aatggcccgc ctggctgacc gcccaacgac ccccgcccat tgacgtcaat     360
   aatgacgtat gttcccatag taacgccaat agggactttc cattgacgtc aatgggtgga     420
   gtatttacgg taaactgccc acttggcagt acatcaagtg tatcatatgc caagtacgcc     480
   ccctattgac gtcaatgacg gtaaatggcc cgcctggcat tatgcccagt acatgacctt     540
   atgggacttt cctacttggc agtacatcta cgtattagtc atcgctatta ccatgtcgag     600
   gccacgttct gcttcactct ccccatctcc cccccctccc cacccccaat tttgtattta     660
   tttatttttt aattattttg tgcagcgatg ggggcggggg gggggggcgc gcgccaggcg     720
   gggcggggcg gggcgagggg cggggcgggg cgaggcggag aggtgcggcg gcagccaatc     780
   agagcggcgc gctccgaaag tttcctttta tggcgaggcg gcggcggcgg cggccctata     840
   aaaagcgaag cgcgcggcgg gcgggagcaa gctttattgc ggtagtttat cacagttaaa     900
   ttgctaacgc agtcagtgct tctgacacaa cagtctcgaa cttaagctgc agaagttggt 960
   cgtgaggcac tgggcaggta agtatcaagg ttacaagaca ggtttaagga gaccaataga 1020
   aactgggctt gtcgagacag agaagactct tgcgtttctg ataggcacct attggtctta 1080
   ctgacatcca ctttgccttt ctctccacag gtgtccactc ccagttcaat tacagctctt 1140
   aaggctagag tacttaatac gactcactat aggctagtaa tacgactcac tatagatggt 1200
   gagcaagggc gaggagctgt tcaccggggt ggtgcccatc ctggtcgagc tggacggcga 1260
   cgtaaacggc cacaagttca gcgtgtccgg cgagggcgag ggcgatgcca cctacggcaa 1320
   gctgaccctg aagttcatct gcaccaccgg caagctgccc gtgccctggc ccaccctcgt 1380
   gaccaccctg acctacggcg tgcagtgctt cagccgctac cccgaccaca tgaagcagca 1440
   cgacttcttc aagtccgcca tgcccgaagg ctacgtccag gagcgcacca tcttcttcaa 1500
   ggacgacggc aactacaaga cccgcgccga ggtgaagttc gagggcgaca ccctggtgaa 1560
   ccgcatcgag ctgaagggca tcgacttcaa ggaggacggc aacatcctgg ggcacaagct 1620
   ggagtacaac tacaacagcc acaacgtcta tatcatggcc gacaagcaga agaacggcat 1680
   caaggtgaac ttcaagatcc gccacaacat cgaggacggc agcgtgcagc tcgccgacca 1740
   ctaccagcag aacaccccca tcggcgacgg ccccgtgctg ctgcccgaca accactacct 1800
   gagcacccag tccgccctga gcaaagaccc caacgagaag cgcgatcaca tggtcctgct 1860
   ggagttcgtg accgccgccg ggatcactct cggcatggac gagctgtaca agaattttga 1920
   ccttcttaag cttgcgggag acgtcgagtc caaccctggg cccatggagc ccagcagcaa 1980
   gaagctgacg ggtcgcctca tgctggccgt gggaggagca gtgcttggct ccctgcagtt 2040
   tggctacaac actggagtca tcaatgcccc ccagaaggtg atcgaggagt tctacaacca 2100
   gacatgggtc caccgctatg gggagagcat cctgcccacc acgctcacca cgctctggtc 2160
   cctctcagtg gccatctttt ctgttggggg catgattggc tccttctctg tgggcctttt 2220
   cgttaaccgc tttggccggc ggaattcaat gctgatgatg aacctgctgg ccttcgtgtc 2280
   cgccgtgctc atgggcttct cgaaactggg caagtccttt gagatgctga tcctgggccg 2340
   cttcatcatc ggtgtgtact gtggcctgac cacaggcttc gtgcccatgt atgtgggtga 2400
   agtgtcaccc acagcccttc gtggggccct gggcaccctg caccagctgg gcatcgtcgt 2460
   cggcatcctc atcgcccagg tgttcggcct ggactccatc atgggcaaca aggacctgtg 2520
   gcccctgctg ctgagcatca tcttcatccc ggccctgctg cagtgcatcg tgctgccctt 2580
   ctgccccgag agtccccgct tcctgctcat caaccgcaac gaggagaacc gggccaagag 2640
   tgtgctaaag aagctgcgcg ggacagctga cgtgacccat gacctgcagg agatgaagga 2700
   agagagtcgg cagatgatgc gggagaagaa ggtcaccatc ctggagctgt tccgctcccc 2760
   cgcctaccgc cagcccatcc tcatcgctgt ggtgctgcag ctgtcccagc agctgtctgg 2820
   catcaacgct gtcttctatt actccacgag catcttcgag aaggcggggg tgcagcagcc 2880
   tgtgtatgcc accattggct ccggtatcgt caacacggcc ttcactgtcg tgtcgctgtt 2940
   tgtggtggag cgagcaggcc ggcggaccct gcacctcata ggcctcgctg gcatggcggg 3000
   ttgtgccata ctcatgacca tcgcgctagc actgctggag cagctacccc ggatgtccta 3060
   tctgagcatc gtggccatct ttggctttgt ggccttcttt gaagtgggtc ctggccccat 3120
   cccatggttc atcgtggctg aactcttcag ccagggtcca cgtccagctg ccattgccgt 3180
   tgcaggcttc tccaactgga cctcaaattt cattgtgggc atgtgcttcc agtatgtgga 3240
   gcaactgtgt ggtccctacg tcttcatcat cttcactgtg ctcctggttc tgttcttcat 3300
   cttcacctac ttcaaagttc ctgagactaa aggccggacc ttcgatgaga tcgcttccgg 3360
   cttccggcag gggggagcca gccaaagtga caagacaccc gaggagctgt tccatcccct 3420
   gggggctgat tcccaagtgt gagtcgcccc agatcaccag cccggcctgc tcccagcagc 3480
   cctaaggatc tctcaggagc acaggcagct ggatgagact tccaaacctg acagatgtca 3540
   gccgagccgg gcctggggct cctttctcca gccagcaatg atgtccagaa gaatattcag 3600
   gacttaacgg ctccaggatt ttaacaaaag caagactgtt gctcaaatct attcagacaa 3660
   gcaacaggtt ttataatttt tttattactg attttgttat ttttatatca gcctgagtct 3720
   cctgtgccca catcccaggc ttcaccctga atggttccat gcctgagggt ggagactaag 3780
   ccctgtcgag acacttgcct tcttcaccca gctaatctgt agggctggac ctatgtccta 3840
   aggacacact aatcgaacta tgaactacaa agcttctatc ccaggaggtg gctatggcca 3900
   cccgttctgc tggcctggat ctctcgagga cggggtgaac tacgcctgag gatccgatct 3960
   ttttccctct gccaaaaatt atggggacat catgaagccc cttgagcatc tgacttctgg 4020
   ctaataaagg aaatttattt tcattgcaat agtgtgttgg aattttttgt gtctctcact 4080
   cggaagcaat tcgttgatct gaatttcgac cacccataat acccattacc ctggtagata 4140
   agtagcatgg cgggttaatc attaactaca aggaacccct agtgatggag ttggccactc 4200
   cctctctgcg cgctcgctcg ctcactgagg ccgggcgacc aaaggtcgcc cgacgcccgg 4260
   gctttgcccg ggcggcctca gtgagcgagc gagcgcgcag ccttaattaa cctaattcac 4320
   tggccgtcgt tttacaacgt cgtgactggg aaaaccctgg cgttacccaa cttaatcgcc 4380
   ttgcagcaca tccccctttc gccagctggc gtaatagcga agaggcccgc accgatcgcc 4440
   cttcccaaca gttgcgcagc ctgaatggcg aatgggacgc gccctgtagc ggcgcattaa 4500
   gcgcggcggg tgtggtggtt acgcgcagcg tgaccgctac acttgccagc gccctagcgc 4560
   ccgctccttt cgctttcttc ccttcctttc tcgccacgtt cgccggcttt ccccgtcaag 4620
   ctctaaatcg ggggctccct ttagggttcc gatttagtgc tttacggcac ctcgacccca 4680
   aaaaacttga ttagggtgat ggttcacgta gtgggccatc gccctgatag acggtttttc 4740
   gccctttgac gttggagtcc acgttcttta atagtggact cttgttccaa actggaacaa 4800
   cactcaaccc tatctcggtc tattcttttg atttataagg gattttgccg atttcggcct 4860
   attggttaaa aaatgagctg atttaacaaa aatttaacgc gaattttaac aaaatattaa 4920
   cgcttacaat ttaggtggca cttttcgggg aaatgtgcgc ggaaccccta tttgtttatt 4980
   tttctaaata cattcaaata tgtatccgct catgagacaa taaccctgat aaatgcttca 5040
   ataatattga aaaaggaaga gtatgagtat tcaacatttc cgtgtcgccc ttattccctt 5100
   ttttgcggca ttttgccttc ctgtttttgc tcacccagaa acgctggtga aagtaaaaga 5160
   tgctgaagat cagttgggtg cacgagtggg ttacatcgaa ctggatctca acagcggtaa 5220
   gatccttgag agttttcgcc ccgaagaacg ttttccaatg atgagcactt ttaaagttct 5280
   gctatgtggc gcggtattat cccgtattga cgccgggcaa gagcaactcg gtcgccgcat 5340
   acactattct cagaatgact tggttgagta ctcaccagtc acagaaaagc atcttacgga 5400
   tggcatgaca gtaagagaat tatgcagtgc tgccataacc atgagtgata acactgcggc 5460
   caacttactt ctgacaacga tcggaggacc gaaggagcta accgcttttt tgcacaacat 5520
   gggggatcat gtaactcgcc ttgatcgttg ggaaccggag ctgaatgaag ccataccaaa 5580
   cgacgagcgt gacaccacga tgcctgtagc aatggcaaca acgttgcgca aactattaac 5640
   tggcgaacta cttactctag cttcccggca acaattaata gactggatgg aggcggataa 5700
   agttgcagga ccacttctgc gctcggccct tccggctggc tggtttattg ctgataaatc 5760
   tggagccggt gagcgtgggt ctcgcggtat cattgcagca ctggggccag atggtaagcc 5820
   ctcccgtatc gtagttatct acacgacggg gagtcaggca actatggatg aacgaaatag 5880
   acagatcgct gagataggtg cctcactgat taagcattgg taactgtcag accaagttta 5940
   ctcatatata ctttagattg atttaaaact tcatttttaa tttaaaagga tctaggtgaa 6000
   gatccttttt gataatctca tgaccaaaat cccttaacgt gagttttcgt tccactgagc 6060
   gtcagacccc gtagaaaaga tcaaaggatc ttcttgagat cctttttttc tgcgcgtaat 6120
   ctgctgcttg caaacaaaaa aaccaccgct accagcggtg gtttgtttgc cggatcaaga 6180
   gctaccaact ctttttccga aggtaactgg cttcagcaga gcgcagatac caaatactgt 6240
   tcttctagtg tagccgtagt taggccacca cttcaagaac tctgtagcac cgcctacata 6300
   cctcgctctg ctaatcctgt taccagtggc tgctgccagt ggcgataagt cgtgtcttac 6360
   cgggttggac tcaagacgat agttaccgga taaggcgcag cggtcgggct gaacgggggg 6420
   ttcgtgcaca cagcccagct tggagcgaac gacctacacc gaactgagat acctacagcg 6480
   tgagctatga gaaagcgcca cgcttcccga agggagaaag gcggacaggt atccggtaag 6540
   cggcagggtc ggaacaggag agcgcacgag ggagcttcca gggggaaacg cctggtatct 6600
   ttatagtcct gtcgggtttc gccacctctg acttgagcgt cgatttttgt gatgctcgtc 6660
   aggggggcgg agcctatgga aaaacgccag caacgcggcc tttttacggt tcctggcctt 6720
   ttgctggcct tttgctcaca tgttctttcc tgcgttatcc cctgattctg tggataaccg 6780
   tattaccgcc tttgagtgag ctgataccgc tcgccgcagc cgaacgaccg agcgcagcga 6840
   gtcagtgagc gaggaagcgg aagagcgccc aatacgcaaa ccgcctctcc ccgcgcgttg 6900
   gccgattcat taatgcagct ggcacgacag gtttcccgac tggaaagcgg gcagtgagcg 6960
   caacgcaatt aatgtgagtt agctcactca ttaggcaccc caggctttac actttatgct 7020
   tccggctcgt atgttgtgtg gaattgtgag cggataacaa tttcacacag gaaacagcta 7080
   tgaccatgat tacgccagat ttaattaag 7109
<210> 2
   <211> 130
   <212> DNA
   <213> Artificial Sequence
<220>
   <223> Description of Artificial Sequence: Synthetic polynucleotide
<220>
   <223> 5'ITR
<400> 2
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 3
   <211> 382
   <212> DNA
   <213> Artificial Sequence
<220>
   <223> Description of Artificial Sequence: Synthetic polynucleotide
<220>
   <223> CMV IE enhancer
<400> 3
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 4
   <211> 382
   <212> DNA
   <213> Artificial Sequence
<220>
   <223> Description of Artificial Sequence: Synthetic polynucleotide
<220>
   <223> CB promoter
<400> 4
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 5
   <211> 717
   <212> DNA
   <213> Artificial Sequence
<220>
   <223> Description of Artificial Sequence: Synthetic polynucleotide
<220>
   <223> eGFP
<400> 5
   atggtgagca agggcgagga gctgttcacc ggggtggtgc ccatcctggt cgagctggac 60
   ggcgacgtaa acggccacaa gttcagcgtg tccggcgagg gcgagggcga tgccacctac 120
   ggcaagctga ccctgaagtt catctgcacc accggcaagc tgcccgtgcc ctggcccacc 180
   ctcgtgacca ccctgaccta cggcgtgcag tgcttcagcc gctaccccga ccacatgaag 240
   cagcacgact tcttcaagtc cgccatgccc gaaggctacg tccaggagcg caccatcttc 300
   ttcaaggacg acggcaacta caagacccgc gccgaggtga agttcgaggg cgacaccctg 360
   gtgaaccgca tcgagctgaa gggcatcgac ttcaaggagg acggcaacat cctggggcac 420
   aagctggagt acaactacaa cagccacaac gtctatatca tggccgacaa gcagaagaac 480
   ggcatcaagg tgaacttcaa gatccgccac aacatcgagg acggcagcgt gcagctcgcc 540
   gaccactacc agcagaacac ccccatcggc gacggccccg tgctgctgcc cgacaaccac 600
   tacctgagca cccagtccgc cctgagcaaa gaccccaacg agaagcgcga tcacatggtc 660
   ctgctggagt tcgtgaccgc cgccgggatc actctcggca tggacgagct gtacaag 717
<210> 6
   <211> 51
   <212> DNA
   <213> Artificial Sequence
<220>
   <223> Description of Artificial Sequence: Synthetic oligonucleotide
<220>
   <223> 2A-linker
<400> 6
   aattttgacc ttcttaagct tgcgggagac gtcgagtcca accctgggcc c 51
<210> 7
   <211> 1959
   <212> DNA
   <213> Artificial Sequence
<220>
   <223> Description of Artificial Sequence: Synthetic polynucleotide
<220>
   <223> hGlut1 cDNA and 3'UTR
<400> 7
   atggagccca gcagcaagaa gctgacgggt cgcctcatgc tggccgtggg aggagcagtg      60
   cttggctccc tgcagtttgg ctacaacact ggagtcatca atgcccccca gaaggtgatc     120
   gaggagttct acaaccagac atgggtccac cgctatgggg agagcatcct gcccaccacg     180
   ctcaccacgc tctggtccct ctcagtggcc atcttttctg ttgggggcat gattggctcc     240
   ttctctgtgg gccttttcgt taaccgcttt ggccggcgga attcaatgct gatgatgaac     300
   ctgctggcct tcgtgtccgc cgtgctcatg ggcttctcga aactgggcaa gtcctttgag     360
   atgctgatcc tgggccgctt catcatcggt gtgtactgtg gcctgaccac aggcttcgtg     420
   cccatgtatg tgggtgaagt gtcacccaca gcccttcgtg gggccctggg caccctgcac     480
   cagctgggca tcgtcgtcgg catcctcatc gcccaggtgt tcggcctgga ctccatcatg     540
   ggcaacaagg acctgtggcc cctgctgctg agcatcatct tcatcccggc cctgctgcag     600
   tgcatcgtgc tgcccttctg ccccgagagt ccccgcttcc tgctcatcaa ccgcaacgag     660
   gagaaccggg ccaagagtgt gctaaagaag ctgcgcggga cagctgacgt gacccatgac     720
   ctgcaggaga tgaaggaaga gagtcggcag atgatgcggg agaagaaggt caccatcctg     780
   gagctgttcc gctcccccgc ctaccgccag cccatcctca tcgctgtggt gctgcagctg 840
   tcccagcagc tgtctggcat caacgctgtc ttctattact ccacgagcat cttcgagaag 900
   gcgggggtgc agcagcctgt gtatgccacc attggctccg gtatcgtcaa cacggccttc 960
   actgtcgtgt cgctgtttgt ggtggagcga gcaggccggc ggaccctgca cctcataggc 1020
   ctcgctggca tggcgggttg tgccatactc atgaccatcg cgctagcact gctggagcag 1080
   ctaccccgga tgtcctatct gagcatcgtg gccatctttg gctttgtggc cttctttgaa 1140
   gtgggtcctg gccccatccc atggttcatc gtggctgaac tcttcagcca gggtccacgt 1200
   ccagctgcca ttgccgttgc aggcttctcc aactggacct caaatttcat tgtgggcatg 1260
   tgcttccagt atgtggagca actgtgtggt ccctacgtct tcatcatctt cactgtgctc 1320
   ctggttctgt tcttcatctt cacctacttc aaagttcctg agactaaagg ccggaccttc 1380
   gatgagatcg cttccggctt ccggcagggg ggagccagcc aaagtgacaa gacacccgag 1440
   gagctgttcc atcccctggg ggctgattcc caagtgtgag tcgccccaga tcaccagccc 1500
   ggcctgctcc cagcagccct aaggatctct caggagcaca ggcagctgga tgagacttcc 1560
   aaacctgaca gatgtcagcc gagccgggcc tggggctcct ttctccagcc agcaatgatg 1620
   tccagaagaa tattcaggac ttaacggctc caggatttta acaaaagcaa gactgttgct 1680
   caaatctatt cagacaagca acaggtttta taattttttt attactgatt ttgttatttt 1740
   tatatcagcc tgagtctcct gtgcccacat cccaggcttc accctgaatg gttccatgcc 1800
   tgagggtgga gactaagccc tgtcgagaca cttgccttct tcacccagct aatctgtagg 1860
   gctggaccta tgtcctaagg acacactaat cgaactatga actacaaagc ttctatccca 1920
   ggaggtggct atggccaccc gttctgctgg cctggatct 1959
<210> 8
   <211> 127
   <212> DNA
   <213> Artificial Sequence
<220>
   <223> Description of Artificial Sequence: Synthetic polynucleotide
<220>
   <223> Poly A signal
<400> 8
   gatctttttc cctctgccaa aaattatggg gacatcatga agccccttga gcatctgact 60
   tctggctaat aaaggaaatt tattttcatt gcaatagtgt gttggaattt tttgtgtctc 120
   tcactcg 127
<210> 9
   <211> 130
   <212> DNA
   <213> Artificial Sequence
<220>
   <223> Description of Artificial Sequence: Synthetic polynucleotide
<220>
   <223> 3' ITR
<400> 9
   aggaacccct agtgatggag ttggccactc cctctctgcg cgctcgctcg ctcactgagg 60
   ccgggcgacc aaaggtcgcc cgacgcccgg gctttgcccg ggcggcctca gtgagcgagc 120
   gagcgcgcag 130
<210> 10
   <211> 7503
   <212> DNA
   <213> Artificial Sequence
<220>
   <223> Description of Artificial Sequence: Synthetic polynucleotide
<220>
   <223> pAAV CB6 PI EGFP-2A-mGlut1
<400> 10
   gccttaatta ggctgcgcgc tcgctcgctc actgaggccg cccgggcaaa gcccgggcgt      60
   cgggcgacct ttggtcgccc ggcctcagtg agcgagcgag cgcgcagaga gggagtggcc     120
   aactccatca ctaggggttc cttgtagtta atgattaacc cgccatgcta cttatctacc     180
   agggtaatgg ggatcctcta gaactatagc tagtcgacat tgattattga ctagttatta     240
   atagtaatca attacggggt cattagttca tagcccatat atggagttcc gcgttacata     300
   acttacggta aatggcccgc ctggctgacc gcccaacgac ccccgcccat tgacgtcaat     360
   aatgacgtat gttcccatag taacgccaat agggactttc cattgacgtc aatgggtgga     420
   gtatttacgg taaactgccc acttggcagt acatcaagtg tatcatatgc caagtacgcc     480
   ccctattgac gtcaatgacg gtaaatggcc cgcctggcat tatgcccagt acatgacctt     540
   atgggacttt cctacttggc agtacatcta cgtattagtc atcgctatta ccatgtcgag     600
   gccacgttct gcttcactct ccccatctcc cccccctccc cacccccaat tttgtattta     660
   tttatttttt aattattttg tgcagcgatg ggggcggggg gggggggcgc gcgccaggcg     720
   gggcggggcg gggcgagggg cggggcgggg cgaggcggag aggtgcggcg gcagccaatc     780
   agagcggcgc gctccgaaag tttcctttta tggcgaggcg gcggcggcgg cggccctata     840
   aaaagcgaag cgcgcggcgg gcgggagcaa gctttattgc ggtagtttat cacagttaaa     900
   ttgctaacgc agtcagtgct tctgacacaa cagtctcgaa cttaagctgc agaagttggt     960
   cgtgaggcac tgggcaggta agtatcaagg ttacaagaca ggtttaagga gaccaataga 1020
   aactgggctt gtcgagacag agaagactct tgcgtttctg ataggcacct attggtctta 1080
   ctgacatcca ctttgccttt ctctccacag gtgtccactc ccagttcaat tacagctctt 1140
   aaggctagag tacttaatac gactcactat aggctagtaa tacgactcac tatagatggt 1200
   gagcaagggc gaggagctgt tcaccggggt ggtgcccatc ctggtcgagc tggacggcga 1260
   cgtaaacggc cacaagttca gcgtgtccgg cgagggcgag ggcgatgcca cctacggcaa 1320
   gctgaccctg aagttcatct gcaccaccgg caagctgccc gtgccctggc ccaccctcgt 1380
   gaccaccctg acctacggcg tgcagtgctt cagccgctac cccgaccaca tgaagcagca 1440
   cgacttcttc aagtccgcca tgcccgaagg ctacgtccag gagcgcacca tcttcttcaa 1500
   ggacgacggc aactacaaga cccgcgccga ggtgaagttc gagggcgaca ccctggtgaa 1560
   ccgcatcgag ctgaagggca tcgacttcaa ggaggacggc aacatcctgg ggcacaagct 1620
   ggagtacaac tacaacagcc acaacgtcta tatcatggcc gacaagcaga agaacggcat 1680
   caaggtgaac ttcaagatcc gccacaacat cgaggacggc agcgtgcagc tcgccgacca 1740
   ctaccagcag aacaccccca tcggcgacgg ccccgtgctg ctgcccgaca accactacct 1800
   gagcacccag tccgccctga gcaaagaccc caacgagaag cgcgatcaca tggtcctgct 1860
   ggagttcgtg accgccgccg ggatcactct cggcatggac gagctgtaca agaattttga 1920
   ccttcttaag cttgcgggag acgtcgagtc caaccctggg cccatggatc ccagcagcaa 1980
   gaaggtgacg ggccgcctca tgttggctgt gggaggagca gtgctcggat cactgcagtt 2040
   cggctataac actggtgtca tcaacgcccc ccagaaggtt attgaggagt tctacaatca 2100
   aacatggaac caccgctacg gagagcccat cccatccacc acactcacca cgctttggtc 2160
   tctctccgtg gccatcttct ctgtcggggg catgattggt tccttctctg tcggcctctt 2220
   tgttaatcgc tttggcaggc ggaactccat gctgatgatg aacctgttgg cctttgtggc 2280
   tgctgtgctt atgggcttct ccaaactggg caagtccttt gagatgctga tcctgggccg 2340
   cttcatcatc ggtgtgtact gcggcctgac tactggcttt gtgcccatgt atgtgggaga 2400
   ggtgtcacct acagctctac gtggagccct aggcacactg caccagctgg gaatcgtcgt 2460
   tggcatcctt attgcccagg tgtttggctt agactccatc atgggcaatg cagacttgtg 2520
   gcctctgctg ctcagtgtca tcttcatccc agccctgcta cagtgtatcc tgttgccctt 2580
   ctgccccgag agcccccgct tcctgctcat caatcgtaac gaggagaacc gggccaagag 2640
   tgtgctgaag aagcttcgag ggacagccga tgtgacccga gacctgcagg agatgaaaga 2700
   agagggtcgg cagatgatgc gggagaagaa ggtcaccatc ttggagctgt tccgctcacc 2760
   cgcctaccgc cagcccatcc tcatcgctgt ggtgctgcag ctgtcccagc agctgtcggg 2820
   tatcaatgct gtgttctact actcaacgag catcttcgag aaggcaggtg tgcagcagcc 2880
   tgtgtacgcc accatcggct ccggtatcgt caacacggcc ttcactgtgg tgtcgctgtt 2940
   tgttgtagag cgagctggac gacggaccct gcacctcatt ggcctggctg gcatggcagg 3000
   ctgtgctgtg ctcatgacca tcgccctggc cttgctggaa cggctgcctt ggatgtccta 3060
   tctgagcatc gtggccatct ttggctttgt ggccttcttt gaagtaggcc ctggtcctat 3120
   tccatggttc attgtggccg agctgttcag ccaggggccc cgtcctgctg ctattgctgt 3180
   ggctggcttc tccaactgga cctcaaactt cattgtgggc atgtgcttcc agtatgtgga 3240
   gcaactgtgc ggcccctacg tcttcatcat cttcacggtg ctcctcgtgc tcttcttcat 3300
   cttcacctac ttcaaagtcc ctgagaccaa aggccgaacc ttcgatgaga tcgcttccgg 3360
   cttccggcag gggggtgcca gccaaagtga caagacaccc gaggagctct tccaccctct 3420
   gggggcggac tcccaagtgt gaggagcccc acacccagcc cggcctgctc cctgcagccc 3480
   aaggatctct ctggagcaca ggcagctaga tgagacctct tccgaaccga cagatctcgg 3540
   gcaagccggg cctgggcgcc tttcctcagc cagcagtgaa gtccaggagg atattcagga 3600
   ctttgatggc tccagaattt ttaatgaaag caagactgct gctcagatct attcagataa 3660
   gcagcaggtt ttataatttt tttattactg attttgttat tttttttttt tatcagccac 3720
   tctcctatct ccacactgta gtcttcacct tgattggccc agtgcctgag ggtggggacc 3780
   acgccctgtc cagacacttg ccttctttgc caagctaatc tgtagggctg gacctatggc 3840
   caaggacaca ctaataccga actctgagct aggaggcttt accgctggag gcggtagctg 3900
   ccacccactt ccgcaggcct ggacctcggc accatagggg tccggactcc attttaggat 3960
   tcgcccattc ctgtctcttc ctacccaacc actcaattaa tctttccttg cctgagacca 4020
   gttggaagca ctggagtgca gggaggagag ggaagggcca ggctgggctg ccaggttcta 4080
   gtctcctgtg cactgagggc cacacaaaca ccatgagaag gacctcggag gctgagaact 4140
   taactgctga agacacggac actcctgccc tgctgtgtat agatggaaga tatttatata 4200
   ttttttggtt gtcaatatta aatacagaca ctaagttata gtatatctgg acaaacccac 4260
   ttgtaaatac accaacaaac tcctgtaact ttacctaagc agatataaat ggctggctcg 4320
   aggacggggt gaactacgcc tgaggatccg atctttttcc ctctgccaaa aattatgggg 4380
   acatcatgaa gccccttgag catctgactt ctggctaata aaggaaattt attttcattg 4440
   caatagtgtg ttggaatttt ttgtgtctct cactcggaag caattcgttg atctgaattt 4500
   cgaccaccca taatacccat taccctggta gataagtagc atggcgggtt aatcattaac 4560
   tacaaggaac ccctagtgat ggagttggcc actccctctc tgcgcgctcg ctcgctcact 4620
   gaggccgggc gaccaaaggt cgcccgacgc ccgggctttg cccgggcggc ctcagtgagc 4680
   gagcgagcgc gcagccttaa ttaacctaat tcactggccg tcgttttaca acgtcgtgac 4740
   tgggaaaacc ctggcgttac ccaacttaat cgccttgcag cacatccccc tttcgccagc 4800
   tggcgtaata gcgaagaggc ccgcaccgat cgcccttccc aacagttgcg cagcctgaat 4860
   ggcgaatggg acgcgccctg tagcggcgca ttaagcgcgg cgggtgtggt ggttacgcgc 4920
   agcgtgaccg ctacacttgc cagcgcccta gcgcccgctc ctttcgcttt cttcccttcc 4980
   tttctcgcca cgttcgccgg ctttccccgt caagctctaa atcgggggct ccctttaggg 5040
   ttccgattta gtgctttacg gcacctcgac cccaaaaaac ttgattaggg tgatggttca 5100
   cgtagtgggc catcgccctg atagacggtt tttcgccctt tgacgttgga gtccacgttc 5160
   tttaatagtg gactcttgtt ccaaactgga acaacactca accctatctc ggtctattct 5220
   tttgatttat aagggatttt gccgatttcg gcctattggt taaaaaatga gctgatttaa 5280
   caaaaattta acgcgaattt taacaaaata ttaacgctta caatttaggt ggcacttttc 5340
   ggggaaatgt gcgcggaacc cctatttgtt tatttttcta aatacattca aatatgtatc 5400
   cgctcatgag acaataaccc tgataaatgc ttcaataata ttgaaaaagg aagagtatga 5460
   gtattcaaca tttccgtgtc gcccttattc ccttttttgc ggcattttgc cttcctgttt 5520
   ttgctcaccc agaaacgctg gtgaaagtaa aagatgctga agatcagttg ggtgcacgag 5580
   tgggttacat cgaactggat ctcaacagcg gtaagatcct tgagagtttt cgccccgaag 5640
   aacgttttcc aatgatgagc acttttaaag ttctgctatg tggcgcggta ttatcccgta 5700
   ttgacgccgg gcaagagcaa ctcggtcgcc gcatacacta ttctcagaat gacttggttg 5760
   agtactcacc agtcacagaa aagcatctta cggatggcat gacagtaaga gaattatgca 5820
   gtgctgccat aaccatgagt gataacactg cggccaactt acttctgaca acgatcggag 5880
   gaccgaagga gctaaccgct tttttgcaca acatggggga tcatgtaact cgccttgatc 5940
   gttgggaacc ggagctgaat gaagccatac caaacgacga gcgtgacacc acgatgcctg 6000
   tagcaatggc aacaacgttg cgcaaactat taactggcga actacttact ctagcttccc 6060
   ggcaacaatt aatagactgg atggaggcgg ataaagttgc aggaccactt ctgcgctcgg 6120
   cccttccggc tggctggttt attgctgata aatctggagc cggtgagcgt gggtctcgcg 6180
   gtatcattgc agcactgggg ccagatggta agccctcccg tatcgtagtt atctacacga 6240
   cggggagtca ggcaactatg gatgaacgaa atagacagat cgctgagata ggtgcctcac 6300
   tgattaagca ttggtaactg tcagaccaag tttactcata tatactttag attgatttaa 6360
   aacttcattt ttaatttaaa aggatctagg tgaagatcct ttttgataat ctcatgacca 6420
   aaatccctta acgtgagttt tcgttccact gagcgtcaga ccccgtagaa aagatcaaag 6480
   gatcttcttg agatcctttt tttctgcgcg taatctgctg cttgcaaaca aaaaaaccac 6540
   cgctaccagc ggtggtttgt ttgccggatc aagagctacc aactcttttt ccgaaggtaa 6600
   ctggcttcag cagagcgcag ataccaaata ctgttcttct agtgtagccg tagttaggcc 6660
   accacttcaa gaactctgta gcaccgccta catacctcgc tctgctaatc ctgttaccag 6720
   tggctgctgc cagtggcgat aagtcgtgtc ttaccgggtt ggactcaaga cgatagttac 6780
   cggataaggc gcagcggtcg ggctgaacgg ggggttcgtg cacacagccc agcttggagc 6840
   gaacgaccta caccgaactg agatacctac agcgtgagct atgagaaagc gccacgcttc 6900
   ccgaagggag aaaggcggac aggtatccgg taagcggcag ggtcggaaca ggagagcgca 6960
   cgagggagct tccaggggga aacgcctggt atctttatag tcctgtcggg tttcgccacc 7020
   tctgacttga gcgtcgattt ttgtgatgct cgtcaggggg gcggagccta tggaaaaacg 7080
   ccagcaacgc ggccttttta cggttcctgg ccttttgctg gccttttgct cacatgttct 7140
   ttcctgcgtt atcccctgat tctgtggata accgtattac cgcctttgag tgagctgata 7200
   ccgctcgccg cagccgaacg accgagcgca gcgagtcagt gagcgaggaa gcggaagagc 7260
   gcccaatacg caaaccgcct ctccccgcgc gttggccgat tcattaatgc agctggcacg 7320
   acaggtttcc cgactggaaa gcgggcagtg agcgcaacgc aattaatgtg agttagctca 7380
   ctcattaggc accccaggct ttacacttta tgcttccggc tcgtatgttg tgtggaattg 7440
   tgagcggata acaatttcac acaggaaaca gctatgacca tgattacgcc agatttaatt 7500
   aag 7503
<210> 11
   <211> 130
   <212> DNA
   <213> Artificial Sequence
<220>
   <223> Description of Artificial Sequence: Synthetic polynucleotide
<220>
   <223> 5'ITR
<400> 11
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 12
   <211> 382
   <212> DNA
   <213> Artificial Sequence
<220>
   <223> Description of Artificial Sequence: Synthetic polynucleotide
<220>
   <223> CMV IE enhancer
<400> 12
   ctagtcgaca ttgattattg actagttatt aatagtaatc aattacgggg tcattagttc        60
   atagcccata tatggagttc cgcgttacat aacttacggt aaatggcccg cctggctgac       120
   cgcccaacga cccccgccca ttgacgtcaa taatgacgta tgttcccata gtaacgccaa       180
   tagggacttt ccattgacgt caatgggtgg agtatttacg gtaaactgcc cacttggcag       240
   tacatcaagt gtatcatatg ccaagtacgc cccctattga cgtcaatgac ggtaaatggc       300
   ccgcctggca ttatgcccag tacatgacct tatgggactt tcctacttgg cagtacatct       360
   acgtattagt catcgctatt ac                                                382
<210> 13
   <211> 382
   <212> DNA
   <213> Artificial Sequence
<220>
   <223> Description of Artificial Sequence: Synthetic polynucleotide
<220>
   <223> CB promoter
<400> 13
   ctagtcgaca ttgattattg actagttatt aatagtaatc aattacgggg tcattagttc        60
   atagcccata tatggagttc cgcgttacat aacttacggt aaatggcccg cctggctgac       120
   cgcccaacga cccccgccca ttgacgtcaa taatgacgta tgttcccata gtaacgccaa       180
   tagggacttt ccattgacgt caatgggtgg agtatttacg gtaaactgcc cacttggcag       240
   tacatcaagt gtatcatatg ccaagtacgc cccctattga cgtcaatgac ggtaaatggc       300
   ccgcctggca ttatgcccag tacatgacct tatgggactt tcctacttgg cagtacatct       360
   acgtattagt catcgctatt ac                                                382
<210> 14
   <211> 717
   <212> DNA
   <213> Artificial Sequence
<220>
   <223> Description of Artificial Sequence: Synthetic polynucleotide
<220>
   <223> eGFP
<400> 14
   atggtgagca agggcgagga gctgttcacc ggggtggtgc ccatcctggt cgagctggac      60
   ggcgacgtaa acggccacaa gttcagcgtg tccggcgagg gcgagggcga tgccacctac     120
   ggcaagctga ccctgaagtt catctgcacc accggcaagc tgcccgtgcc ctggcccacc     180
   ctcgtgacca ccctgaccta cggcgtgcag tgcttcagcc gctaccccga ccacatgaag     240
   cagcacgact tcttcaagtc cgccatgccc gaaggctacg tccaggagcg caccatcttc     300
   ttcaaggacg acggcaacta caagacccgc gccgaggtga agttcgaggg cgacaccctg     360
   gtgaaccgca tcgagctgaa gggcatcgac ttcaaggagg acggcaacat cctggggcac     420
   aagctggagt acaactacaa cagccacaac gtctatatca tggccgacaa gcagaagaac     480
   ggcatcaagg tgaacttcaa gatccgccac aacatcgagg acggcagcgt gcagctcgcc     540
   gaccactacc agcagaacac ccccatcggc gacggccccg tgctgctgcc cgacaaccac     600
   tacctgagca cccagtccgc cctgagcaaa gaccccaacg agaagcgcga tcacatggtc     660
   ctgctggagt tcgtgaccgc cgccgggatc actctcggca tggacgagct gtacaag        717
<210> 15
   <211> 51
   <212> DNA
   <213> Artificial Sequence
<220>
   <223> Description of Artificial Sequence: Synthetic oligonucleotide
<220>
   <223> 2A-linker
<400> 15
   aattttgacc ttcttaagct tgcgggagac gtcgagtcca accctgggcc c 51
<210> 16
   <211> 2353
   <212> DNA
   <213> Artificial Sequence
<220>
   <223> Description of Artificial Sequence: Synthetic polynucleotide
<220>
   <223> mGlut1 cDNA and 3'UTR
<400> 16 tataaatggc tgg 2353
<210> 17
   <211> 127
   <212> DNA
   <213> Artificial Sequence
<220>
   <223> Description of Artificial Sequence: Synthetic polynucleotide
<220>
   <223> Poly A signal
<400> 17
   gatctttttc cctctgccaa aaattatggg gacatcatga agccccttga gcatctgact 60
   tctggctaat aaaggaaatt tattttcatt gcaatagtgt gttggaattt tttgtgtctc 120
   tcactcg 127
<210> 18
   <211> 130
   <212> DNA
   <213> Artificial Sequence
<220>
   <223> Description of Artificial Sequence: Synthetic polynucleotide
<220>
   <223> 3' ITR
<400> 18
   aggaacccct agtgatggag ttggccactc cctctctgcg cgctcgctcg ctcactgagg 60
   ccgggcgacc aaaggtcgcc cgacgcccgg gctttgcccg ggcggcctca gtgagcgagc 120
   gagcgcgcag 130
<210> 19
   <211> 6844
   <212> DNA
   <213> Artificial Sequence
<220>
   <223> Description of Artificial Sequence: Synthetic polynucleotide
<220>
   <223> pAAV CB6 PI hGlutl-2A-EGFP
<400> 19
   cttaattagg ctgcgcgctc gctcgctcac tgaggccgcc cgggcaaagc ccgggcgtcg 60
   ggcgaccttt ggtcgcccgg cctcagtgag cgagcgagcg cgcagagagg gagtggccaa 120
   ctccatcact aggggttcct tgtagttaat gattaacccg ccatgctact tatctaccag 180
   ggtaatgggg atcctctaga actatagcta gtcgacattg attattgact agttattaat 240
   agtaatcaat tacggggtca ttagttcata gcccatatat ggagttccgc gttacataac 300
   ttacggtaaa tggcccgcct ggctgaccgc ccaacgaccc ccgcccattg acgtcaataa 360
   tgacgtatgt tcccatagta acgccaatag ggactttcca ttgacgtcaa tgggtggagt 420
   atttacggta aactgcccac ttggcagtac atcaagtgta tcatatgcca agtacgcccc 480
   ctattgacgt caatgacggt aaatggcccg cctggcatta tgcccagtac atgaccttat 540
   gggactttcc tacttggcag tacatctacg tattagtcat cgctattacc atgtcgaggc 600
   cacgttctgc ttcactctcc ccatctcccc cccctcccca cccccaattt tgtatttatt 660
   tattttttaa ttattttgtg cagcgatggg ggcggggggg gggggcgcgc gccaggcggg 720
   gcggggcggg gcgaggggcg gggcggggcg aggcggagag gtgcggcggc agccaatcag 780
   agcggcgcgc tccgaaagtt tccttttatg gcgaggcggc ggcggcggcg gccctataaa 840
   aagcgaagcg cgcggcgggc gggagcaagc tttattgcgg tagtttatca cagttaaatt 900
   gctaacgcag tcagtgcttc tgacacaaca gtctcgaact taagctgcag aagttggtcg 960
   tgaggcactg ggcaggtaag tatcaaggtt acaagacagg tttaaggaga ccaatagaaa 1020
   ctgggcttgt cgagacagag aagactcttg cgtttctgat aggcacctat tggtcttact 1080
   gacatccact ttgcctttct ctccacaggt gtccactccc agttcaatta cagctcttaa 1140
   ggctagagta cttaatacga ctcactatag gctagcgcgc cgaattgatc cactagtaac 1200
   ggccgccagt gtgctggaag caggagacca aacgacgggg gtcggagtca gagtcgcagt 1260
   gggagtcccc ggaccggagc acgagcctga gcgggagagc gccgctcgca cgcccgtcgc 1320
   cacccgcgta cccggcgcag ccagagccac cagcgcagcg ctgccatgga gcccagcagc 1380
   aagaagctga cgggtcgcct catgctggcc gtgggaggag cagtgcttgg ctccctgcag 1440
   tttggctaca acactggagt catcaatgcc ccccagaagg tgatcgagga gttctacaac 1500
   cagacatggg tccaccgcta tggggagagc atcctgccca ccacgctcac cacgctctgg 1560
   tccctctcag tggccatctt ttctgttggg ggcatgattg gctccttctc tgtgggcctt 1620
   ttcgttaacc gctttggccg gcggaattca atgctgatga tgaacctgct ggccttcgtg 1680
   tccgccgtgc tcatgggctt ctcgaaactg ggcaagtcct ttgagatgct gatcctgggc 1740
   cgcttcatca tcggtgtgta ctgtggcctg accacaggct tcgtgcccat gtatgtgggt 1800
   gaagtgtcac ccacagccct tcgtggggcc ctgggcaccc tgcaccagct gggcatcgtc 1860
   gtcggcatcc tcatcgccca ggtgttcggc ctggactcca tcatgggcaa caaggacctg 1920
   tggcccctgc tgctgagcat catcttcatc ccggccctgc tgcagtgcat cgtgctgccc 1980
   ttctgccccg agagtccccg cttcctgctc atcaaccgca acgaggagaa ccgggccaag 2040
   agtgtgctaa agaagctgcg cgggacagct gacgtgaccc atgacctgca ggagatgaag 2100
   gaagagagtc ggcagatgat gcgggagaag aaggtcacca tcctggagct gttccgctcc 2160
   cccgcctacc gccagcccat cctcatcgct gtggtgctgc agctgtccca gcagctgtct 2220
   ggcatcaacg ctgtcttcta ttactccacg agcatcttcg agaaggcggg ggtgcagcag 2280
   cctgtgtatg ccaccattgg ctccggtatc gtcaacacgg ccttcactgt cgtgtcgctg 2340
   tttgtggtgg agcgagcagg ccggcggacc ctgcacctca taggcctcgc tggcatggcg 2400
   ggttgtgcca tactcatgac catcgcgcta gcactgctgg agcagctacc ccggatgtcc 2460
   tatctgagca tcgtggccat ctttggcttt gtggccttct ttgaagtggg tcctggcccc 2520
   atcccatggt tcatcgtggc tgaactcttc agccagggtc cacgtccagc tgccattgcc 2580
   gttgcaggct tctccaactg gacctcaaat ttcattgtgg gcatgtgctt ccagtatgtg 2640
   gagcaactgt gtggtcccta cgtcttcatc atcttcactg tgctcctggt tctgttcttc 2700
   atcttcacct acttcaaagt tcctgagact aaaggccgga ccttcgatga gatcgcttcc 2760
   ggcttccggc aggggggagc cagccaaagt gacaagacac ccgaggagct gttccatccc 2820
   ctgggggctg attcccaagt gaccggtaat tttgaccttc ttaagcttgc gggagacgtc 2880
   gagtccaacc ctgggcccgc catggtgagc aagggcgagg agctgttcac cggggtggtg 2940
   cccatcctgg tcgagctgga cggcgacgta aacggccaca agttcagcgt gtccggcgag 3000
   ggcgagggcg atgccaccta cggcaagctg accctgaagt tcatctgcac caccggcaag 3060
   ctgcccgtgc cctggcccac cctcgtgacc accctgacct acggcgtgca gtgcttcagc 3120
   cgctaccccg accacatgaa gcagcacgac ttcttcaagt ccgccatgcc cgaaggctac 3180
   gtccaggagc gcaccatctt cttcaaggac gacggcaact acaagacccg cgccgaggtg 3240
   aagttcgagg gcgacaccct ggtgaaccgc atcgagctga agggcatcga cttcaaggag 3300
   gacggcaaca tcctggggca caagctggag tacaactaca acagccacaa cgtctatatc 3360
   atggccgaca agcagaagaa cggcatcaag gtgaacttca agatccgcca caacatcgag 3420
   gacggcagcg tgcagctcgc cgaccactac cagcagaaca cccccatcgg cgacggcccc 3480
   gtgctgctgc ccgacaacca ctacctgagc acccagtccg ccctgagcaa agaccccaac 3540
   gagaagcgcg atcacatggt cctgctggag ttcgtgaccg ccgccgggat cactctcggc 3600
   atggacgagc tgtacaagta aagcggccat caagcttatc ggccgttact agtggatcga 3660
   ggacggggtg aactacgcct gaggatccga tctttttccc tctgccaaaa attatgggga 3720
   catcatgaag ccccttgagc atctgacttc tggctaataa aggaaattta ttttcattgc 3780
   aatagtgtgt tggaattttt tgtgtctctc actcggaagc aattcgttga tctgaatttc 3840
   gaccacccat aatacccatt accctggtag ataagtagca tggcgggtta atcattaact 3900
   acaaggaacc cctagtgatg gagttggcca ctccctctct gcgcgctcgc tcgctcactg 3960
   aggccgggcg accaaaggtc gcccgacgcc cgggctttgc ccgggcggcc tcagtgagcg 4020
   agcgagcgcg cagccttaat taacctaatt cactggccgt cgttttacaa cgtcgtgact 4080
   gggaaaaccc tggcgttacc caacttaatc gccttgcagc acatccccct ttcgccagct 4140
   ggcgtaatag cgaagaggcc cgcaccgatc gcccttccca acagttgcgc agcctgaatg 4200
   gcgaatggga cgcgccctgt agcggcgcat taagcgcggc gggtgtggtg gttacgcgca 4260
   gcgtgaccgc tacacttgcc agcgccctag cgcccgctcc tttcgctttc ttcccttcct 4320
   ttctcgccac gttcgccggc tttccccgtc aagctctaaa tcgggggctc cctttagggt 4380
   tccgatttag tgctttacgg cacctcgacc ccaaaaaact tgattagggt gatggttcac 4440
   gtagtgggcc atcgccctga tagacggttt ttcgcccttt gacgttggag tccacgttct 4500
   ttaatagtgg actcttgttc caaactggaa caacactcaa ccctatctcg gtctattctt 4560
   ttgatttata agggattttg ccgatttcgg cctattggtt aaaaaatgag ctgatttaac 4620
   aaaaatttaa cgcgaatttt aacaaaatat taacgcttac aatttaggtg gcacttttcg 4680
   gggaaatgtg cgcggaaccc ctatttgttt atttttctaa atacattcaa atatgtatcc 4740
   gctcatgaga caataaccct gataaatgct tcaataatat tgaaaaagga agagtatgag 4800
   tattcaacat ttccgtgtcg cccttattcc cttttttgcg gcattttgcc ttcctgtttt 4860
   tgctcaccca gaaacgctgg tgaaagtaaa agatgctgaa gatcagttgg gtgcacgagt 4920
   gggttacatc gaactggatc tcaacagcgg taagatcctt gagagttttc gccccgaaga 4980
   acgttttcca atgatgagca cttttaaagt tctgctatgt ggcgcggtat tatcccgtat 5040
   tgacgccggg caagagcaac tcggtcgccg catacactat tctcagaatg acttggttga 5100
   gtactcacca gtcacagaaa agcatcttac ggatggcatg acagtaagag aattatgcag 5160
   tgctgccata accatgagtg ataacactgc ggccaactta cttctgacaa cgatcggagg 5220
   accgaaggag ctaaccgctt ttttgcacaa catgggggat catgtaactc gccttgatcg 5280
   ttgggaaccg gagctgaatg aagccatacc aaacgacgag cgtgacacca cgatgcctgt 5340
   agcaatggca acaacgttgc gcaaactatt aactggcgaa ctacttactc tagcttcccg 5400
   gcaacaatta atagactgga tggaggcgga taaagttgca ggaccacttc tgcgctcggc 5460
   ccttccggct ggctggttta ttgctgataa atctggagcc ggtgagcgtg ggtctcgcgg 5520
   tatcattgca gcactggggc cagatggtaa gccctcccgt atcgtagtta tctacacgac 5580
   ggggagtcag gcaactatgg atgaacgaaa tagacagatc gctgagatag gtgcctcact 5640
   gattaagcat tggtaactgt cagaccaagt ttactcatat atactttaga ttgatttaaa 5700
   acttcatttt taatttaaaa ggatctaggt gaagatcctt tttgataatc tcatgaccaa 5760
   aatcccttaa cgtgagtttt cgttccactg agcgtcagac cccgtagaaa agatcaaagg 5820
   atcttcttga gatccttttt ttctgcgcgt aatctgctgc ttgcaaacaa aaaaaccacc 5880
   gctaccagcg gtggtttgtt tgccggatca agagctacca actctttttc cgaaggtaac 5940
   tggcttcagc agagcgcaga taccaaatac tgttcttcta gtgtagccgt agttaggcca 6000
   ccacttcaag aactctgtag caccgcctac atacctcgct ctgctaatcc tgttaccagt 6060
   ggctgctgcc agtggcgata agtcgtgtct taccgggttg gactcaagac gatagttacc 6120
   ggataaggcg cagcggtcgg gctgaacggg gggttcgtgc acacagccca gcttggagcg 6180
   aacgacctac accgaactga gatacctaca gcgtgagcta tgagaaagcg ccacgcttcc 6240
   cgaagggaga aaggcggaca ggtatccggt aagcggcagg gtcggaacag gagagcgcac 6300
   gagggagctt ccagggggaa acgcctggta tctttatagt cctgtcgggt ttcgccacct 6360
   ctgacttgag cgtcgatttt tgtgatgctc gtcagggggg cggagcctat ggaaaaacgc 6420
   cagcaacgcg gcctttttac ggttcctggc cttttgctgg ccttttgctc acatgttctt 6480
   tcctgcgtta tcccctgatt ctgtggataa ccgtattacc gcctttgagt gagctgatac 6540
   cgctcgccgc agccgaacga ccgagcgcag cgagtcagtg agcgaggaag cggaagagcg 6600
   cccaatacgc aaaccgcctc tccccgcgcg ttggccgatt cattaatgca gctggcacga 6660
   caggtttccc gactggaaag cgggcagtga gcgcaacgca attaatgtga gttagctcac 6720
   tcattaggca ccccaggctt tacactttat gcttccggct cgtatgttgt gtggaattgt 6780
   gagcggataa caatttcaca caggaaacag ctatgaccat gattacgcca gatttaatta 6840
   aggc 6844
<210> 20
   <211> 130
   <212> DNA
   <213> Artificial Sequence
<220>
   <223> Description of Artificial Sequence: Synthetic polynucleotide
<220>
   <223> 5'ITR
<400> 20
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 21
   <211> 382
   <212> DNA
   <213> Artificial Sequence
<220>
   <223> Description of Artificial Sequence: Synthetic polynucleotide
<220>
   <223> CMV IE enhancer
<400> 21
   ctagtcgaca ttgattattg actagttatt aatagtaatc aattacgggg tcattagttc      60
   atagcccata tatggagttc cgcgttacat aacttacggt aaatggcccg cctggctgac     120
   cgcccaacga cccccgccca ttgacgtcaa taatgacgta tgttcccata gtaacgccaa     180
   tagggacttt ccattgacgt caatgggtgg agtatttacg gtaaactgcc cacttggcag     240
   tacatcaagt gtatcatatg ccaagtacgc cccctattga cgtcaatgac ggtaaatggc     300
   ccgcctggca ttatgcccag tacatgacct tatgggactt tcctacttgg cagtacatct     360
   acgtattagt catcgctatt ac                                              382
<210> 22
   <211> 382
   <212> DNA
   <213> Artificial Sequence
<220>
   <223> Description of Artificial Sequence: Synthetic polynucleotide
<220>
   <223> CB promoter
<400> 22
   ctagtcgaca ttgattattg actagttatt aatagtaatc aattacgggg tcattagttc      60
   atagcccata tatggagttc cgcgttacat aacttacggt aaatggcccg cctggctgac     120
   cgcccaacga cccccgccca ttgacgtcaa taatgacgta tgttcccata gtaacgccaa     180
   tagggacttt ccattgacgt caatgggtgg agtatttacg gtaaactgcc cacttggcag     240
   tacatcaagt gtatcatatg ccaagtacgc cccctattga cgtcaatgac ggtaaatggc     300
   ccgcctggca ttatgcccag tacatgacct tatgggactt tcctacttgg cagtacatct     360
   acgtattagt catcgctatt ac                                              382
<210> 23
   <211> 1479
   <212> DNA
   <213> Artificial Sequence
<220>
   <223> Description of Artificial Sequence: Synthetic polynucleotide
<220>
   <223> hGlut1 cDNA
<400> 23
   atggagccca gcagcaagaa gctgacgggt cgcctcatgc tggccgtggg aggagcagtg 60
   cttggctccc tgcagtttgg ctacaacact ggagtcatca atgcccccca gaaggtgatc 120
   gaggagttct acaaccagac atgggtccac cgctatgggg agagcatcct gcccaccacg 180
   ctcaccacgc tctggtccct ctcagtggcc atcttttctg ttgggggcat gattggctcc 240
   ttctctgtgg gccttttcgt taaccgcttt ggccggcgga attcaatgct gatgatgaac 300
   ctgctggcct tcgtgtccgc cgtgctcatg ggcttctcga aactgggcaa gtcctttgag 360
   atgctgatcc tgggccgctt catcatcggt gtgtactgtg gcctgaccac aggcttcgtg 420
   cccatgtatg tgggtgaagt gtcacccaca gcccttcgtg gggccctggg caccctgcac 480
   cagctgggca tcgtcgtcgg catcctcatc gcccaggtgt tcggcctgga ctccatcatg 540
   ggcaacaagg acctgtggcc cctgctgctg agcatcatct tcatcccggc cctgctgcag 600
   tgcatcgtgc tgcccttctg ccccgagagt ccccgcttcc tgctcatcaa ccgcaacgag 660
   gagaaccggg ccaagagtgt gctaaagaag ctgcgcggga cagctgacgt gacccatgac 720
   ctgcaggaga tgaaggaaga gagtcggcag atgatgcggg agaagaaggt caccatcctg 780
   gagctgttcc gctcccccgc ctaccgccag cccatcctca tcgctgtggt gctgcagctg 840
   tcccagcagc tgtctggcat caacgctgtc ttctattact ccacgagcat cttcgagaag 900
   gcgggggtgc agcagcctgt gtatgccacc attggctccg gtatcgtcaa cacggccttc 960
   actgtcgtgt cgctgtttgt ggtggagcga gcaggccggc ggaccctgca cctcataggc 1020
   ctcgctggca tggcgggttg tgccatactc atgaccatcg cgctagcact gctggagcag 1080
   ctaccccgga tgtcctatct gagcatcgtg gccatctttg gctttgtggc cttctttgaa 1140
   gtgggtcctg gccccatccc atggttcatc gtggctgaac tcttcagcca gggtccacgt 1200
   ccagctgcca ttgccgttgc aggcttctcc aactggacct caaatttcat tgtgggcatg 1260
   tgcttccagt atgtggagca actgtgtggt ccctacgtct tcatcatctt cactgtgctc 1320
   ctggttctgt tcttcatctt cacctacttc aaagttcctg agactaaagg ccggaccttc 1380
   gatgagatcg cttccggctt ccggcagggg ggagccagcc aaagtgacaa gacacccgag 1440
   gagctgttcc atcccctggg ggctgattcc caagtgtga 1479
<210> 24
   <211> 51
   <212> DNA
   <213> Artificial Sequence
<220>
   <223> Description of Artificial Sequence: Synthetic oligonucleotide
<220>
   <223> 2A-linker
<400> 24
   aattttgacc ttcttaagct tgcgggagac gtcgagtcca accctgggcc c 51
<210> 25
   <211> 717
   <212> DNA
   <213> Artificial Sequence
<220>
   <223> Description of Artificial Sequence: Synthetic polynucleotide
<220>
   <223> eGFP
<400> 25
   atggtgagca agggcgagga gctgttcacc ggggtggtgc ccatcctggt cgagctggac       60
   ggcgacgtaa acggccacaa gttcagcgtg tccggcgagg gcgagggcga tgccacctac      120
   ggcaagctga ccctgaagtt catctgcacc accggcaagc tgcccgtgcc ctggcccacc      180
   ctcgtgacca ccctgaccta cggcgtgcag tgcttcagcc gctaccccga ccacatgaag      240
   cagcacgact tcttcaagtc cgccatgccc gaaggctacg tccaggagcg caccatcttc      300
   ttcaaggacg acggcaacta caagacccgc gccgaggtga agttcgaggg cgacaccctg      360
   gtgaaccgca tcgagctgaa gggcatcgac ttcaaggagg acggcaacat cctggggcac      420
   aagctggagt acaactacaa cagccacaac gtctatatca tggccgacaa gcagaagaac      480
   ggcatcaagg tgaacttcaa gatccgccac aacatcgagg acggcagcgt gcagctcgcc      540
   gaccactacc agcagaacac ccccatcggc gacggccccg tgctgctgcc cgacaaccac      600
   tacctgagca cccagtccgc cctgagcaaa gaccccaacg agaagcgcga tcacatggtc      660
   ctgctggagt tcgtgaccgc cgccgggatc actctcggca tggacgagct gtacaag         717
<210> 26
   <211> 127
   <212> DNA
   <213> Artificial Sequence
<220>
   <223> Description of Artificial Sequence: Synthetic polynucleotide
<220>
   <223> Poly A signal
<400> 26
   gatctttttc cctctgccaa aaattatggg gacatcatga agccccttga gcatctgact 60
   tctggctaat aaaggaaatt tattttcatt gcaatagtgt gttggaattt tttgtgtctc 120
   tcactcg 127
<210> 27
   <211> 130
   <212> DNA
   <213> Artificial Sequence
<220>
   <223> Description of Artificial Sequence: Synthetic polynucleotide
<220>
   <223> 3' ITR
<400> 27
   aggaacccct agtgatggag ttggccactc cctctctgcg cgctcgctcg ctcactgagg 60
   ccgggcgacc aaaggtcgcc cgacgcccgg gctttgcccg ggcggcctca gtgagcgagc 120
   gagcgcgcag 130
<210> 28
   <211> 6568
   <212> DNA
   <213> Artificial Sequence
<220>
   <223> Description of Artificial Sequence: Synthetic polynucleotide
<220>
   <223> pAAV CB6 PI hGlut1
<400> 28
   cttaattagg ctgcgcgctc gctcgctcac tgaggccgcc cgggcaaagc ccgggcgtcg       60
   ggcgaccttt ggtcgcccgg cctcagtgag cgagcgagcg cgcagagagg gagtggccaa      120
   ctccatcact aggggttcct tgtagttaat gattaacccg ccatgctact tatctaccag      180
   ggtaatgggg atcctctaga actatagcta gtcgacattg attattgact agttattaat      240
   agtaatcaat tacggggtca ttagttcata gcccatatat ggagttccgc gttacataac      300
   ttacggtaaa tggcccgcct ggctgaccgc ccaacgaccc ccgcccattg acgtcaataa      360
   tgacgtatgt tcccatagta acgccaatag ggactttcca ttgacgtcaa tgggtggagt      420
   atttacggta aactgcccac ttggcagtac atcaagtgta tcatatgcca agtacgcccc      480
   ctattgacgt caatgacggt aaatggcccg cctggcatta tgcccagtac atgaccttat      540
   gggactttcc tacttggcag tacatctacg tattagtcat cgctattacc atgtcgaggc      600
   cacgttctgc ttcactctcc ccatctcccc cccctcccca cccccaattt tgtatttatt      660
   tattttttaa ttattttgtg cagcgatggg ggcggggggg gggggcgcgc gccaggcggg      720
   gcggggcggg gcgaggggcg gggcggggcg aggcggagag gtgcggcggc agccaatcag      780
   agcggcgcgc tccgaaagtt tccttttatg gcgaggcggc ggcggcggcg gccctataaa      840
   aagcgaagcg cgcggcgggc gggagcaagc tttattgcgg tagtttatca cagttaaatt      900
   gctaacgcag tcagtgcttc tgacacaaca gtctcgaact taagctgcag aagttggtcg      960
   tgaggcactg ggcaggtaag tatcaaggtt acaagacagg tttaaggaga ccaatagaaa 1020
   ctgggcttgt cgagacagag aagactcttg cgtttctgat aggcacctat tggtcttact 1080
   gacatccact ttgcctttct ctccacaggt gtccactccc agttcaatta cagctcttaa 1140
   ggctagagta cttaatacga ctcactatag gctagcgcgc cgaattggcc gccagtgtga 1200
   tggatatctg cagaattcgc ccttagcagg agaccaaacg acgggggtcg gagtcagagt 1260
   cgcagtggga gtccccggac cggagcacga gcctgagcgg gagagcgccg ctcgcacgcc 1320
   cgtcgccacc cgcgtacccg gcgcagccag agccaccagc gcagcgctgc catggagccc 1380
   agcagcaaga agctgacggg tcgcctcatg ctggccgtgg gaggagcagt gcttggctcc 1440
   ctgcagtttg gctacaacac tggagtcatc aatgcccccc agaaggtgat cgaggagttc 1500
   tacaaccaga catgggtcca ccgctatggg gagagcatcc tgcccaccac gctcaccacg 1560
   ctctggtccc tctcagtggc catcttttct gttgggggca tgattggctc cttctctgtg 1620
   ggccttttcg ttaaccgctt tggccggcgg aattcaatgc tgatgatgaa cctgctggcc 1680
   ttcgtgtccg ccgtgctcat gggcttctcg aaactgggca agtcctttga gatgctgatc 1740
   ctgggccgct tcatcatcgg tgtgtactgt ggcctgacca caggcttcgt gcccatgtat 1800
   gtgggtgaag tgtcacccac agcccttcgt ggggccctgg gcaccctgca ccagctgggc 1860
   atcgtcgtcg gcatcctcat cgcccaggtg ttcggcctgg actccatcat gggcaacaag 1920
   gacctgtggc ccctgctgct gagcatcatc ttcatcccgg ccctgctgca gtgcatcgtg 1980
   ctgcccttct gccccgagag tccccgcttc ctgctcatca accgcaacga ggagaaccgg 2040
   gccaagagtg tgctaaagaa gctgcgcggg acagctgacg tgacccatga cctgcaggag 2100
   atgaaggaag agagtcggca gatgatgcgg gagaagaagg tcaccatcct ggagctgttc 2160
   cgctcccccg cctaccgcca gcccatcctc atcgctgtgg tgctgcagct gtcccagcag 2220
   ctgtctggca tcaacgctgt cttctattac tccacgagca tcttcgagaa ggcgggggtg 2280
   cagcagcctg tgtatgccac cattggctcc ggtatcgtca acacggcctt cactgtcgtg 2340
   tcgctgtttg tggtggagcg agcaggccgg cggaccctgc acctcatagg cctcgctggc 2400
   atggcgggtt gtgccatact catgaccatc gcgctagcac tgctggagca gctaccccgg 2460
   atgtcctatc tgagcatcgt ggccatcttt ggctttgtgg ccttctttga agtgggtcct 2520
   ggccccatcc catggttcat cgtggctgaa ctcttcagcc agggtccacg tccagctgcc 2580
   attgccgttg caggcttctc caactggacc tcaaatttca ttgtgggcat gtgcttccag 2640
   tatgtggagc aactgtgtgg tccctacgtc ttcatcatct tcactgtgct cctggttctg 2700
   ttcttcatct tcacctactt caaagttcct gagactaaag gccggacctt cgatgagatc 2760
   gcttccggct tccggcaggg gggagccagc caaagtgaca agacacccga ggagctgttc 2820
   catcccctgg gggctgattc ccaagtgtga gtcgccccag atcaccagcc cggcctgctc 2880
   ccagcagccc taaggatctc tcaggagcac aggcagctgg atgagacttc caaacctgac 2940
   agatgtcagc cgagccgggc ctggggctcc tttctccagc cagcaatgat gtccagaaga 3000
   atattcagga cttaacggct ccaggatttt aacaaaagca agactgttgc tcaaatctat 3060
   tcagacaagc aacaggtttt ataatttttt tattactgat tttgttattt ttatatcagc 3120
   ctgagtctcc tgtgcccaca tcccaggctt caccctgaat ggttccatgc ctgagggtgg 3180
   agactaagcc ctgtcgagac acttgccttc ttcacccagc taatctgtag ggctggacct 3240
   atgtcctaag gacacactaa tcgaactatg aactacaaag cttctatccc aggaggtggc 3300
   tatggccacc cgttctgctg gcctggatct ccaagaaaca aagggcgaat tccagcacac 3360
   tggcggccgt tactagtgga tcgaggacgg ggtgaactac gcctgaggat ccgatctttt 3420
   tccctctgcc aaaaattatg gggacatcat gaagcccctt gagcatctga cttctggcta 3480
   ataaaggaaa tttattttca ttgcaatagt gtgttggaat tttttgtgtc tctcactcgg 3540
   aagcaattcg ttgatctgaa tttcgaccac ccataatacc cattaccctg gtagataagt 3600
   agcatggcgg gttaatcatt aactacaagg aacccctagt gatggagttg gccactccct 3660
   ctctgcgcgc tcgctcgctc actgaggccg ggcgaccaaa ggtcgcccga cgcccgggct 3720
   ttgcccgggc ggcctcagtg agcgagcgag cgcgcagcct taattaacct aattcactgg 3780
   ccgtcgtttt acaacgtcgt gactgggaaa accctggcgt tacccaactt aatcgccttg 3840
   cagcacatcc ccctttcgcc agctggcgta atagcgaaga ggcccgcacc gatcgccctt 3900
   cccaacagtt gcgcagcctg aatggcgaat gggacgcgcc ctgtagcggc gcattaagcg 3960
   cggcgggtgt ggtggttacg cgcagcgtga ccgctacact tgccagcgcc ctagcgcccg 4020
   ctcctttcgc tttcttccct tcctttctcg ccacgttcgc cggctttccc cgtcaagctc 4080
   taaatcgggg gctcccttta gggttccgat ttagtgcttt acggcacctc gaccccaaaa 4140
   aacttgatta gggtgatggt tcacgtagtg ggccatcgcc ctgatagacg gtttttcgcc 4200
   ctttgacgtt ggagtccacg ttctttaata gtggactctt gttccaaact ggaacaacac 4260
   tcaaccctat ctcggtctat tcttttgatt tataagggat tttgccgatt tcggcctatt 4320
   ggttaaaaaa tgagctgatt taacaaaaat ttaacgcgaa ttttaacaaa atattaacgc 4380
   ttacaattta ggtggcactt ttcggggaaa tgtgcgcgga acccctattt gtttattttt 4440
   ctaaatacat tcaaatatgt atccgctcat gagacaataa ccctgataaa tgcttcaata 4500
   atattgaaaa aggaagagta tgagtattca acatttccgt gtcgccctta ttcccttttt 4560
   tgcggcattt tgccttcctg tttttgctca cccagaaacg ctggtgaaag taaaagatgc 4620
   tgaagatcag ttgggtgcac gagtgggtta catcgaactg gatctcaaca gcggtaagat 4680
   ccttgagagt tttcgccccg aagaacgttt tccaatgatg agcactttta aagttctgct 4740
   atgtggcgcg gtattatccc gtattgacgc cgggcaagag caactcggtc gccgcataca 4800
   ctattctcag aatgacttgg ttgagtactc accagtcaca gaaaagcatc ttacggatgg 4860
   catgacagta agagaattat gcagtgctgc cataaccatg agtgataaca ctgcggccaa 4920
   cttacttctg acaacgatcg gaggaccgaa ggagctaacc gcttttttgc acaacatggg 4980
   ggatcatgta actcgccttg atcgttggga accggagctg aatgaagcca taccaaacga 5040
   cgagcgtgac accacgatgc ctgtagcaat ggcaacaacg ttgcgcaaac tattaactgg 5100
   cgaactactt actctagctt cccggcaaca attaatagac tggatggagg cggataaagt 5160
   tgcaggacca cttctgcgct cggcccttcc ggctggctgg tttattgctg ataaatctgg 5220
   agccggtgag cgtgggtctc gcggtatcat tgcagcactg gggccagatg gtaagccctc 5280
   ccgtatcgta gttatctaca cgacggggag tcaggcaact atggatgaac gaaatagaca 5340
   gatcgctgag ataggtgcct cactgattaa gcattggtaa ctgtcagacc aagtttactc 5400
   atatatactt tagattgatt taaaacttca tttttaattt aaaaggatct aggtgaagat 5460
   cctttttgat aatctcatga ccaaaatccc ttaacgtgag ttttcgttcc actgagcgtc 5520
   agaccccgta gaaaagatca aaggatcttc ttgagatcct ttttttctgc gcgtaatctg 5580
   ctgcttgcaa acaaaaaaac caccgctacc agcggtggtt tgtttgccgg atcaagagct 5640
   accaactctt tttccgaagg taactggctt cagcagagcg cagataccaa atactgttct 5700
   tctagtgtag ccgtagttag gccaccactt caagaactct gtagcaccgc ctacatacct 5760
   cgctctgcta atcctgttac cagtggctgc tgccagtggc gataagtcgt gtcttaccgg 5820
   gttggactca agacgatagt taccggataa ggcgcagcgg tcgggctgaa cggggggttc 5880
   gtgcacacag cccagcttgg agcgaacgac ctacaccgaa ctgagatacc tacagcgtga 5940
   gctatgagaa agcgccacgc ttcccgaagg gagaaaggcg gacaggtatc cggtaagcgg 6000
   cagggtcgga acaggagagc gcacgaggga gcttccaggg ggaaacgcct ggtatcttta 6060
   tagtcctgtc gggtttcgcc acctctgact tgagcgtcga tttttgtgat gctcgtcagg 6120
   ggggcggagc ctatggaaaa acgccagcaa cgcggccttt ttacggttcc tggccttttg 6180
   ctggcctttt gctcacatgt tctttcctgc gttatcccct gattctgtgg ataaccgtat 6240
   taccgccttt gagtgagctg ataccgctcg ccgcagccga acgaccgagc gcagcgagtc 6300
   agtgagcgag gaagcggaag agcgcccaat acgcaaaccg cctctccccg cgcgttggcc 6360
   gattcattaa tgcagctggc acgacaggtt tcccgactgg aaagcgggca gtgagcgcaa 6420
   cgcaattaat gtgagttagc tcactcatta ggcaccccag gctttacact ttatgcttcc 6480
   ggctcgtatg ttgtgtggaa ttgtgagcgg ataacaattt cacacaggaa acagctatga 6540
   ccatgattac gccagattta attaaggc 6568
<210> 29
   <211> 130
   <212> DNA
   <213> Artificial Sequence
<220>
   <223> Description of Artificial Sequence: Synthetic polynucleotide
<220>
   <223> 5'ITR
<400> 29
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 30
   <211> 382
   <212> DNA
   <213> Artificial Sequence
<220>
   <223> Description of Artificial Sequence: Synthetic polynucleotide
<220>
   <223> CMV IE enhancer
<400> 30
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 31
   <211> 382
   <212> DNA
   <213> Artificial Sequence
<220>
   <223> Description of Artificial Sequence: Synthetic polynucleotide
<220>
   <223> CB promoter
<400> 31
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 32
   <211> 1479
   <212> DNA
   <213> Artificial Sequence
<220>
   <223> Description of Artificial Sequence: Synthetic polynucleotide
<220>
   <223> hGlut1 cDNA
<400> 32
   atggagccca gcagcaagaa gctgacgggt cgcctcatgc tggccgtggg aggagcagtg 60
   cttggctccc tgcagtttgg ctacaacact ggagtcatca atgcccccca gaaggtgatc 120
   gaggagttct acaaccagac atgggtccac cgctatgggg agagcatcct gcccaccacg 180
   ctcaccacgc tctggtccct ctcagtggcc atcttttctg ttgggggcat gattggctcc 240
   ttctctgtgg gccttttcgt taaccgcttt ggccggcgga attcaatgct gatgatgaac 300
   ctgctggcct tcgtgtccgc cgtgctcatg ggcttctcga aactgggcaa gtcctttgag 360
   atgctgatcc tgggccgctt catcatcggt gtgtactgtg gcctgaccac aggcttcgtg 420
   cccatgtatg tgggtgaagt gtcacccaca gcccttcgtg gggccctggg caccctgcac 480
   cagctgggca tcgtcgtcgg catcctcatc gcccaggtgt tcggcctgga ctccatcatg 540
   ggcaacaagg acctgtggcc cctgctgctg agcatcatct tcatcccggc cctgctgcag 600
   tgcatcgtgc tgcccttctg ccccgagagt ccccgcttcc tgctcatcaa ccgcaacgag 660
   gagaaccggg ccaagagtgt gctaaagaag ctgcgcggga cagctgacgt gacccatgac 720
   ctgcaggaga tgaaggaaga gagtcggcag atgatgcggg agaagaaggt caccatcctg 780
   gagctgttcc gctcccccgc ctaccgccag cccatcctca tcgctgtggt gctgcagctg 840
   tcccagcagc tgtctggcat caacgctgtc ttctattact ccacgagcat cttcgagaag 900
   gcgggggtgc agcagcctgt gtatgccacc attggctccg gtatcgtcaa cacggccttc 960
   actgtcgtgt cgctgtttgt ggtggagcga gcaggccggc ggaccctgca cctcataggc 1020
   ctcgctggca tggcgggttg tgccatactc atgaccatcg cgctagcact gctggagcag 1080
   ctaccccgga tgtcctatct gagcatcgtg gccatctttg gctttgtggc cttctttgaa 1140
   gtgggtcctg gccccatccc atggttcatc gtggctgaac tcttcagcca gggtccacgt 1200
   ccagctgcca ttgccgttgc aggcttctcc aactggacct caaatttcat tgtgggcatg 1260
   tgcttccagt atgtggagca actgtgtggt ccctacgtct tcatcatctt cactgtgctc 1320
   ctggttctgt tcttcatctt cacctacttc aaagttcctg agactaaagg ccggaccttc 1380
   gatgagatcg cttccggctt ccggcagggg ggagccagcc aaagtgacaa gacacccgag 1440
   gagctgttcc atcccctggg ggctgattcc caagtgtga 1479
<210> 33
   <211> 127
   <212> DNA
   <213> Artificial Sequence
<220>
   <223> Description of Artificial Sequence: Synthetic polynucleotide
<220>
   <223> Poly A signal
<400> 33
   gatctttttc cctctgccaa aaattatggg gacatcatga agccccttga gcatctgact        60
   tctggctaat aaaggaaatt tattttcatt gcaatagtgt gttggaattt tttgtgtctc       120
   tcactcg                                                                 127
<210> 34
   <211> 130
   <212> DNA
   <213> Artificial Sequence
<220>
   <223> Description of Artificial Sequence: Synthetic polynucleotide
<220>
   <223> 3' ITR
<400> 34
   aggaacccct agtgatggag ttggccactc cctctctgcg cgctcgctcg ctcactgagg        60
   ccgggcgacc aaaggtcgcc cgacgcccgg gctttgcccg ggcggcctca gtgagcgagc       120
   gagcgcgcag                                                              130
<210> 35
   <211> 7057
   <212> DNA
   <213> Artificial Sequence
<220>
   <223> Description of Artificial Sequence: Synthetic polynucleotide
<220>
   <223> pAAV CB6 PI mGlut1
<400> 35
   cttaattagg ctgcgcgctc gctcgctcac tgaggccgcc cgggcaaagc ccgggcgtcg 60
   ggcgaccttt ggtcgcccgg cctcagtgag cgagcgagcg cgcagagagg gagtggccaa 120
   ctccatcact aggggttcct tgtagttaat gattaacccg ccatgctact tatctaccag 180
   ggtaatgggg atcctctaga actatagcta gtcgacattg attattgact agttattaat 240
   agtaatcaat tacggggtca ttagttcata gcccatatat ggagttccgc gttacataac 300
   ttacggtaaa tggcccgcct ggctgaccgc ccaacgaccc ccgcccattg acgtcaataa 360
   tgacgtatgt tcccatagta acgccaatag ggactttcca ttgacgtcaa tgggtggagt 420
   atttacggta aactgcccac ttggcagtac atcaagtgta tcatatgcca agtacgcccc 480
   ctattgacgt caatgacggt aaatggcccg cctggcatta tgcccagtac atgaccttat 540
   gggactttcc tacttggcag tacatctacg tattagtcat cgctattacc atgtcgaggc 600
   cacgttctgc ttcactctcc ccatctcccc cccctcccca cccccaattt tgtatttatt 660
   tattttttaa ttattttgtg cagcgatggg ggcggggggg gggggcgcgc gccaggcggg 720
   gcggggcggg gcgaggggcg gggcggggcg aggcggagag gtgcggcggc agccaatcag 780
   agcggcgcgc tccgaaagtt tccttttatg gcgaggcggc ggcggcggcg gccctataaa 840
   aagcgaagcg cgcggcgggc gggagcaagc tttattgcgg tagtttatca cagttaaatt 900
   gctaacgcag tcagtgcttc tgacacaaca gtctcgaact taagctgcag aagttggtcg 960
   tgaggcactg ggcaggtaag tatcaaggtt acaagacagg tttaaggaga ccaatagaaa 1020
   ctgggcttgt cgagacagag aagactcttg cgtttctgat aggcacctat tggtcttact 1080
   gacatccact ttgcctttct ctccacaggt gtccactccc agttcaatta cagctcttaa 1140
   ggctagagta cttaatacga ctcactatag gctagcgcgc cgaattcggc acgaggaaaa 1200
   aggcagctcc gcgcgctctc ccccaagagc agaggcttgc ttgtagagtg acgatctgag 1260
   ctacggggtc ttaagtgcgt cagggcgtgg aggtctggcg ggagacgcat agttacagcg 1320
   cgtccgttct ccgtctcgca gccggcacag ctagagcttc gagcgcagcg cggccatgga 1380
   tcccagcagc aagaaggtga cgggccgcct catgttggct gtgggaggag cagtgctcgg 1440
   atcactgcag ttcggctata acactggtgt catcaacgcc ccccagaagg ttattgagga 1500
   gttctacaat caaacatgga accaccgcta cggagagccc atcccatcca ccacactcac 1560
   cacgctttgg tctctctccg tggccatctt ctctgtcggg ggcatgattg gttccttctc 1620
   tgtcggcctc tttgttaatc gctttggcag gcggaactcc atgctgatga tgaacctgtt 1680
   ggcctttgtg gctgctgtgc ttatgggctt ctccaaactg ggcaagtcct ttgagatgct 1740
   gatcctgggc cgcttcatca tcggtgtgta ctgcggcctg actactggct ttgtgcccat 1800
   gtatgtggga gaggtgtcac ctacagctct acgtggagcc ctaggcacac tgcaccagct 1860
   gggaatcgtc gttggcatcc ttattgccca ggtgtttggc ttagactcca tcatgggcaa 1920
   tgcagacttg tggcctctgc tgctcagtgt catcttcatc ccagccctgc tacagtgtat 1980
   cctgttgccc ttctgccccg agagcccccg cttcctgctc atcaatcgta acgaggagaa 2040
   ccgggccaag agtgtgctga agaagcttcg agggacagcc gatgtgaccc gagacctgca 2100
   ggagatgaaa gaagagggtc ggcagatgat gcgggagaag aaggtcacca tcttggagct 2160
   gttccgctca cccgcctacc gccagcccat cctcatcgct gtggtgctgc agctgtccca 2220
   gcagctgtcg ggtatcaatg ctgtgttcta ctactcaacg agcatcttcg agaaggcagg 2280
   tgtgcagcag cctgtgtacg ccaccatcgg ctccggtatc gtcaacacgg ccttcactgt 2340
   ggtgtcgctg tttgttgtag agcgagctgg acgacggacc ctgcacctca ttggcctggc 2400
   tggcatggca ggctgtgctg tgctcatgac catcgccctg gccttgctgg aacggctgcc 2460
   ttggatgtcc tatctgagca tcgtggccat ctttggcttt gtggccttct ttgaagtagg 2520
   ccctggtcct attccatggt tcattgtggc cgagctgttc agccaggggc cccgtcctgc 2580
   tgctattgct gtggctggct tctccaactg gacctcaaac ttcattgtgg gcatgtgctt 2640
   ccagtatgtg gagcaactgt gcggccccta cgtcttcatc atcttcacgg tgctcctcgt 2700
   gctcttcttc atcttcacct acttcaaagt ccctgagacc aaaggccgaa ccttcgatga 2760
   gatcgcttcc ggcttccggc aggggggtgc cagccaaagt gacaagacac ccgaggagct 2820
   cttccaccct ctgggggcgg actcccaagt gtgaggagcc ccacacccag cccggcctgc 2880
   tccctgcagc ccaaggatct ctctggagca caggcagcta gatgagacct cttccgaacc 2940
   gacagatctc gggcaagccg ggcctgggcg cctttcctca gccagcagtg aagtccagga 3000
   ggatattcag gactttgatg gctccagaat ttttaatgaa agcaagactg ctgctcagat 3060
   ctattcagat aagcagcagg ttttataatt tttttattac tgattttgtt attttttttt 3120
   tttatcagcc actctcctat ctccacactg tagtcttcac cttgattggc ccagtgcctg 3180
   agggtgggga ccacgccctg tccagacact tgccttcttt gccaagctaa tctgtagggc 3240
   tggacctatg gccaaggaca cactaatacc gaactctgag ctaggaggct ttaccgctgg 3300
   aggcggtagc tgccacccac ttccgcaggc ctggacctcg gcaccatagg ggtccggact 3360
   ccattttagg attcgcccat tcctgtctct tcctacccaa ccactcaatt aatctttcct 3420
   tgcctgagac cagttggaag cactggagtg cagggaggag agggaagggc caggctgggc 3480
   tgccaggttc tagtctcctg tgcactgagg gccacacaaa caccatgaga aggacctcgg 3540
   aggctgagaa cttaactgct gaagacacgg acactcctgc cctgctgtgt atagatggaa 3600
   gatatttata tattttttgg ttgtcaatat taaatacaga cactaagtta tagtatatct 3660
   ggacaaaccc acttgtaaat acaccaacaa actcctgtaa ctttacctaa gcagatataa 3720
   atggctggtt tttagaaaaa aaaaaaaaaa aaaaaaaaaa aaaaaaaaaa aaaaaaaaaa 3780
   aaaaaaaaaa aaaaaaaaaa aaaaaaaaaa actgtctcgc tgcggccgct ctagagtatc 3840
   cctcgactct agagtcgacc cgggcggcct cgaggacggg gtgaactacg cctgaggatc 3900
   cgatcttttt ccctctgcca aaaattatgg ggacatcatg aagccccttg agcatctgac 3960
   ttctggctaa taaaggaaat ttattttcat tgcaatagtg tgttggaatt ttttgtgtct 4020
   ctcactcgga agcaattcgt tgatctgaat ttcgaccacc cataataccc attaccctgg 4080
   tagataagta gcatggcggg ttaatcatta actacaagga acccctagtg atggagttgg 4140
   ccactccctc tctgcgcgct cgctcgctca ctgaggccgg gcgaccaaag gtcgcccgac 4200
   gcccgggctt tgcccgggcg gcctcagtga gcgagcgagc gcgcagcctt aattaaccta 4260
   attcactggc cgtcgtttta caacgtcgtg actgggaaaa ccctggcgtt acccaactta 4320
   atcgccttgc agcacatccc cctttcgcca gctggcgtaa tagcgaagag gcccgcaccg 4380
   atcgcccttc ccaacagttg cgcagcctga atggcgaatg ggacgcgccc tgtagcggcg 4440
   cattaagcgc ggcgggtgtg gtggttacgc gcagcgtgac cgctacactt gccagcgccc 4500
   tagcgcccgc tcctttcgct ttcttccctt cctttctcgc cacgttcgcc ggctttcccc 4560
   gtcaagctct aaatcggggg ctccctttag ggttccgatt tagtgcttta cggcacctcg 4620
   accccaaaaa acttgattag ggtgatggtt cacgtagtgg gccatcgccc tgatagacgg 4680
   tttttcgccc tttgacgttg gagtccacgt tctttaatag tggactcttg ttccaaactg 4740
   gaacaacact caaccctatc tcggtctatt cttttgattt ataagggatt ttgccgattt 4800
   cggcctattg gttaaaaaat gagctgattt aacaaaaatt taacgcgaat tttaacaaaa 4860
   tattaacgct tacaatttag gtggcacttt tcggggaaat gtgcgcggaa cccctatttg 4920
   tttatttttc taaatacatt caaatatgta tccgctcatg agacaataac cctgataaat 4980
   gcttcaataa tattgaaaaa ggaagagtat gagtattcaa catttccgtg tcgcccttat 5040
   tccctttttt gcggcatttt gccttcctgt ttttgctcac ccagaaacgc tggtgaaagt 5100
   aaaagatgct gaagatcagt tgggtgcacg agtgggttac atcgaactgg atctcaacag 5160
   cggtaagatc cttgagagtt ttcgccccga agaacgtttt ccaatgatga gcacttttaa 5220
   agttctgcta tgtggcgcgg tattatcccg tattgacgcc gggcaagagc aactcggtcg 5280
   ccgcatacac tattctcaga atgacttggt tgagtactca ccagtcacag aaaagcatct 5340
   tacggatggc atgacagtaa gagaattatg cagtgctgcc ataaccatga gtgataacac 5400
   tgcggccaac ttacttctga caacgatcgg aggaccgaag gagctaaccg cttttttgca 5460
   caacatgggg gatcatgtaa ctcgccttga tcgttgggaa ccggagctga atgaagccat 5520
   accaaacgac gagcgtgaca ccacgatgcc tgtagcaatg gcaacaacgt tgcgcaaact 5580
   attaactggc gaactactta ctctagcttc ccggcaacaa ttaatagact ggatggaggc 5640
   ggataaagtt gcaggaccac ttctgcgctc ggcccttccg gctggctggt ttattgctga 5700
   taaatctgga gccggtgagc gtgggtctcg cggtatcatt gcagcactgg ggccagatgg 5760
   taagccctcc cgtatcgtag ttatctacac gacggggagt caggcaacta tggatgaacg 5820
   aaatagacag atcgctgaga taggtgcctc actgattaag cattggtaac tgtcagacca 5880
   agtttactca tatatacttt agattgattt aaaacttcat ttttaattta aaaggatcta 5940
   ggtgaagatc ctttttgata atctcatgac caaaatccct taacgtgagt tttcgttcca 6000
   ctgagcgtca gaccccgtag aaaagatcaa aggatcttct tgagatcctt tttttctgcg 6060
   cgtaatctgc tgcttgcaaa caaaaaaacc accgctacca gcggtggttt gtttgccgga 6120
   tcaagagcta ccaactcttt ttccgaaggt aactggcttc agcagagcgc agataccaaa 6180
   tactgttctt ctagtgtagc cgtagttagg ccaccacttc aagaactctg tagcaccgcc 6240
   tacatacctc gctctgctaa tcctgttacc agtggctgct gccagtggcg ataagtcgtg 6300
   tcttaccggg ttggactcaa gacgatagtt accggataag gcgcagcggt cgggctgaac 6360
   ggggggttcg tgcacacagc ccagcttgga gcgaacgacc tacaccgaac tgagatacct 6420
   acagcgtgag ctatgagaaa gcgccacgct tcccgaaggg agaaaggcgg acaggtatcc 6480
   ggtaagcggc agggtcggaa caggagagcg cacgagggag cttccagggg gaaacgcctg 6540
   gtatctttat agtcctgtcg ggtttcgcca cctctgactt gagcgtcgat ttttgtgatg 6600
   ctcgtcaggg gggcggagcc tatggaaaaa cgccagcaac gcggcctttt tacggttcct 6660
   ggccttttgc tggccttttg ctcacatgtt ctttcctgcg ttatcccctg attctgtgga 6720
   taaccgtatt accgcctttg agtgagctga taccgctcgc cgcagccgaa cgaccgagcg 6780
   cagcgagtca gtgagcgagg aagcggaaga gcgcccaata cgcaaaccgc ctctccccgc 6840
   gcgttggccg attcattaat gcagctggca cgacaggttt cccgactgga aagcgggcag 6900
   tgagcgcaac gcaattaatg tgagttagct cactcattag gcaccccagg ctttacactt 6960
   tatgcttccg gctcgtatgt tgtgtggaat tgtgagcgga taacaatttc acacaggaaa 7020
   cagctatgac catgattacg ccagatttaa ttaaggc 7057
<210> 36
   <211> 130
   <212> DNA
   <213> Artificial Sequence
<220>
   <223> Description of Artificial Sequence: Synthetic polynucleotide
<220>
   <223> 5'ITR
<400> 36
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 37
   <211> 382
   <212> DNA
   <213> Artificial Sequence
<220>
   <223> Description of Artificial Sequence: Synthetic polynucleotide
<220>
   <223> CMV IE enhancer
<400> 37
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 38
   <211> 382
   <212> DNA
   <213> Artificial Sequence
<220>
   <223> Description of Artificial Sequence: Synthetic polynucleotide
<220>
   <223> CB promoter
<400> 38
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 39
   <211> 1479
   <212> DNA
   <213> Artificial Sequence
<220>
   <223> Description of Artificial Sequence: Synthetic polynucleotide
<220>
   <223> mGlut1 cDNA
<400> 39
   atggatccca gcagcaagaa ggtgacgggc cgcctcatgt tggctgtggg aggagcagtg      60
   ctcggatcac tgcagttcgg ctataacact ggtgtcatca acgcccccca gaaggttatt     120
   gaggagttct acaatcaaac atggaaccac cgctacggag agcccatccc atccaccaca     180
   ctcaccacgc tttggtctct ctccgtggcc atcttctctg tcgggggcat gattggttcc     240
   ttctctgtcg gcctctttgt taatcgcttt ggcaggcgga actccatgct gatgatgaac     300
   ctgttggcct ttgtggctgc tgtgcttatg ggcttctcca aactgggcaa gtcctttgag     360
   atgctgatcc tgggccgctt catcatcggt gtgtactgcg gcctgactac tggctttgtg     420
   cccatgtatg tgggagaggt gtcacctaca gctctacgtg gagccctagg cacactgcac     480
   cagctgggaa tcgtcgttgg catccttatt gcccaggtgt ttggcttaga ctccatcatg     540
   ggcaatgcag acttgtggcc tctgctgctc agtgtcatct tcatcccagc cctgctacag     600
   tgtatcctgt tgcccttctg ccccgagagc cccggcttcc tgctcatcaa tcgtaacgag     660
   gagaaccggg ccaagagtgt gctgaagaag cttcgaggga cagccgatgt gacccgagac     720
   ctgcaggaga tgaaagaaga gggtcggcag atgatgcggg agaagaaggt caccatcttg     780
   gagctgttcc gctcacccgc ctaccgccag cccatcctca tcgctgtggt gctgcagctg     840
   tcccagcagc tgtcgggtat caatgctgtg ttctactact caacgagcat cttcgagaag     900
   gcaggtgtgc agcagcctgt gtacgccacc atcggctccg gtatcgtcaa cacggccttc     960
   actgtggtgt cgctgtttgt tgtagagcga gctggacgac ggaccctgca cctcattggc    1020
   ctggctggca tggcaggctg tgctgtgctc atgaccatcg ccctggcctt gctggaacgg    1080
   ctgccttgga tgtcctatct gagcatcgtg gccatctttg gctttgtggc cttctttgaa    1140
   gtaggccctg gtcctattcc atggttcatt gtggccgagc tgttcagcca ggggccccgt    1200
   cctgctgcta ttgctgtggc tggcttctcc aactggacct caaacttcat tgtgggcatg    1260
   tgcttccagt atgtggagca actgtgcggc ccctacgtct tcatcatctt cacggtgctc    1320
   ctcgtgctct tcttcatctt cacctacttc aaagtccctg agaccaaagg ccgaaccttc    1380
   gatgagatcg cttccggctt ccggcagggg ggtgccagcc aaagtgacaa gacacccgag    1440
   gagctcttcc accctctggg ggcggactcc caagtgtga                           1479
<210> 40
   <211> 127
   <212> DNA
   <213> Artificial Sequence
<220>
   <223> Description of Artificial Sequence: Synthetic polynucleotide
<220>
   <223> Poly A signal
<400> 40
   gatctttttc cctctgccaa aaattatggg gacatcatga agccccttga gcatctgact 60
   tctggctaat aaaggaaatt tattttcatt gcaatagtgt gttggaattt tttgtgtctc 120
   tcactcg 127
<210> 41
   <211> 130
   <212> DNA
   <213> Artificial Sequence
<220>
   <223> Description of Artificial Sequence: Synthetic polynucleotide
<220>
   <223> 3' ITR
<400> 41
   aggaacccct agtgatggag ttggccactc cctctctgcg cgctcgctcg ctcactgagg 60
   ccgggcgacc aaaggtcgcc cgacgcccgg gctttgcccg ggcggcctca gtgagcgagc 120
   gagcgcgcag 130
<210> 42
   <211> 6641
   <212> DNA
   <213> Artificial Sequence
<220>
   <223> Description of Artificial Sequence: Synthetic polynucleotide
<220>
   <223> pAAV CB6 PI hGlut1-out3xmiR-122 BS
<400> 42
   cttaattagg ctgcgcgctc gctcgctcac tgaggccgcc cgggcaaagc ccgggcgtcg 60
   ggcgaccttt ggtcgcccgg cctcagtgag cgagcgagcg cgcagagagg gagtggccaa 120
   ctccatcact aggggttcct tgtagttaat gattaacccg ccatgctact tatctaccag 180
   ggtaatgggg atcctctaga actatagcta gtcgacattg attattgact agttattaat 240
   agtaatcaat tacggggtca ttagttcata gcccatatat ggagttccgc gttacataac 300
   ttacggtaaa tggcccgcct ggctgaccgc ccaacgaccc ccgcccattg acgtcaataa 360
   tgacgtatgt tcccatagta acgccaatag ggactttcca ttgacgtcaa tgggtggagt 420
   atttacggta aactgcccac ttggcagtac atcaagtgta tcatatgcca agtacgcccc 480
   ctattgacgt caatgacggt aaatggcccg cctggcatta tgcccagtac atgaccttat 540
   gggactttcc tacttggcag tacatctacg tattagtcat cgctattacc atgtcgaggc 600
   cacgttctgc ttcactctcc ccatctcccc cccctcccca cccccaattt tgtatttatt 660
   tattttttaa ttattttgtg cagcgatggg ggcggggggg gggggcgcgc gccaggcggg 720
   gcggggcggg gcgaggggcg gggcggggcg aggcggagag gtgcggcggc agccaatcag 780
   agcggcgcgc tccgaaagtt tccttttatg gcgaggcggc ggcggcggcg gccctataaa 840
   aagcgaagcg cgcggcgggc gggagcaagc tttattgcgg tagtttatca cagttaaatt 900
   gctaacgcag tcagtgcttc tgacacaaca gtctcgaact taagctgcag aagttggtcg 960
   tgaggcactg ggcaggtaag tatcaaggtt acaagacagg tttaaggaga ccaatagaaa 1020
   ctgggcttgt cgagacagag aagactcttg cgtttctgat aggcacctat tggtcttact 1080
   gacatccact ttgcctttct ctccacaggt gtccactccc agttcagctc ttaaggctag 1140
   agtacttaat acgactcact ataggctagc gcgccgaatt ggccgccagt gtgatggata 1200
   tctgcagaat tcgcccttag caggagacca aacgacgggg gtcggagtca gagtcgcagt 1260
   gggagtcccc ggaccggagc acgagcctga gcgggagagc gccgctcgca cgcccgtcgc 1320
   cacccgcgta cccggcgcag ccagagccac cagcgcagcg ctgccatgga gcccagcagc 1380
   aagaagctga cgggtcgcct catgctggcc gtgggaggag cagtgcttgg ctccctgcag 1440
   tttggctaca acactggagt catcaatgcc ccccagaagg tgatcgagga gttctacaac 1500
   cagacatggg tccaccgcta tggggagagc atcctgccca ccacgctcac cacgctctgg 1560
   tccctctcag tggccatctt ttctgttggg ggcatgattg gctccttctc tgtgggcctt 1620
   ttcgttaacc gctttggccg gcggaattca atgctgatga tgaacctgct ggccttcgtg 1680
   tccgccgtgc tcatgggctt ctcgaaactg ggcaagtcct ttgagatgct gatcctgggc 1740
   cgcttcatca tcggtgtgta ctgtggcctg accacaggct tcgtgcccat gtatgtgggt 1800
   gaagtgtcac ccacagccct tcgtggggcc ctgggcaccc tgcaccagct gggcatcgtc 1860
   gtcggcatcc tcatcgccca ggtgttcggc ctggactcca tcatgggcaa caaggacctg 1920
   tggcccctgc tgctgagcat catcttcatc ccggccctgc tgcagtgcat cgtgctgccc 1980
   ttctgccccg agagtccccg cttcctgctc atcaaccgca acgaggagaa ccgggccaag 2040
   agtgtgctaa agaagctgcg cgggacagct gacgtgaccc atgacctgca ggagatgaag 2100
   gaagagagtc ggcagatgat gcgggagaag aaggtcacca tcctggagct gttccgctcc 2160
   cccgcctacc gccagcccat cctcatcgct gtggtgctgc agctgtccca gcagctgtct 2220
   ggcatcaacg ctgtcttcta ttactccacg agcatcttcg agaaggcggg ggtgcagcag 2280
   cctgtgtatg ccaccattgg ctccggtatc gtcaacacgg ccttcactgt cgtgtcgctg 2340
   tttgtggtgg agcgagcagg ccggcggacc ctgcacctca taggcctcgc tggcatggcg 2400
   ggttgtgcca tactcatgac catcgcgcta gcactgctgg agcagctacc ccggatgtcc 2460
   tatctgagca tcgtggccat ctttggcttt gtggccttct ttgaagtggg tcctggcccc 2520
   atcccatggt tcatcgtggc tgaactcttc agccagggtc cacgtccagc tgccattgcc 2580
   gttgcaggct tctccaactg gacctcaaat ttcattgtgg gcatgtgctt ccagtatgtg 2640
   gagcaactgt gtggtcccta cgtcttcatc atcttcactg tgctcctggt tctgttcttc 2700
   atcttcacct acttcaaagt tcctgagact aaaggccgga ccttcgatga gatcgcttcc 2760
   ggcttccggc aggggggagc cagccaaagt gacaagacac ccgaggagct gttccatccc 2820
   ctgggggctg attcccaagt gtgagtcgcc ccagatcacc agcccggcct gctcccagca 2880
   gccctaagga tctctcagga gcacaggcag ctggatgaga cttccaaacc tgacagatgt 2940
   cagccgagcc gggcctgggg ctcctttctc cagccagcaa tgatgtccag aagaatattc 3000
   aggacttaac ggctccagga ttttaacaaa agcaagactg ttgctcaaat ctattcagac 3060
   aagcaacagg ttttataatt tttttattac tgattttgtt atttttatat cagcctgagt 3120
   ctcctgtgcc cacatcccag gcttcaccct gaatggttcc atgcctgagg gtggagacta 3180
   agccctgtcg agacacttgc cttcttcacc cagctaatct gtagggctgg acctatgtcc 3240
   taaggacaca ctaatcgaac tatgaactac aaagcttcta tcccaggagg tggctatggc 3300
   cacccgttct gctggcctgg atctcccaag aaacaaaggg cgaattccag cacactggcg 3360
   gcccgaaaca aacaccattg tcacactcca acaaacacca ttgtcacact ccaacaaaca 3420
   ccattgtcac actccattcg ggttactagt ggatcgagga cggggtgaac tacgcctgag 3480
   gatccgatct tttccctctg gccaaaaatt atggggacat catgaagccc cttgagcatc 3540
   tgacttctgg ctaataaagg aaatttattt tcattgcaat agtgtgttgg aattttttgt 3600
   gtctctcact cggaagcaat tcgttgatct gaatttcgac cacccataat acccattacc 3660
   ctggtagata agtagcatgg cgggttaatc attaactaca aggaacccct agtgatggag 3720
   ttggccactc cctctctgcg cgctcgctcg ctcactgagg ccgggcgacc aaaggtcgcc 3780
   cgacgcccgg gctttgcccg ggcggcctca gtgagcgagc gagcgcgcag ccttaattaa 3840
   cctaattcac tggccgtcgt tttacaacgt cgtgactggg aaaaccctgg cgttacccaa 3900
   cttaatcgcc ttgcagcaca tccccctttc gccagctggc gtaatagcga agaggcccgc 3960
   accgatcgcc cttcccaaca gttgcgcagc ctgaatggcg aatgggacgc gccctgtagc 4020
   ggcgcattaa gcgcggcggg tgtggtggtt acgcgcagcg tgaccgctac acttgccagc 4080
   gccctagcgc ccgctccttt cgctttcttc ccttcctttc tcgccacgtt cgccggcttt 4140
   ccccgtcaag ctctaaatcg ggggctccct ttagggttcc gatttagtgc tttacggcac 4200
   ctcgacccca aaaaacttga ttagggtgat ggttcacgta gtgggccatc gccctgatag 4260
   acggtttttc gccctttgac gttggagtcc acgttcttta atagtggact cttgttccaa 4320
   actggaacaa cactcaaccc tatctcggtc tattcttttg atttataagg gattttgccg 4380
   atttcggcct attggttaaa aaatgagctg atttaacaaa aatttaacgc gaattttaac 4440
   aaaatattaa cgcttacaat ttaggtggca cttttcgggg aaatgtgcgc ggaaccccta 4500
   tttgtttatt tttctaaata cattcaaata tgtatccgct catgagacaa taaccctgat 4560
   aaatgcttca ataatattga aaaaggaaga gtatgagtat tcaacatttc cgtgtcgccc 4620
   ttattccctt ttttgcggca ttttgccttc ctgtttttgc tcacccagaa acgctggtga 4680
   aagtaaaaga tgctgaagat cagttgggtg cacgagtggg ttacatcgaa ctggatctca 4740
   acagcggtaa gatccttgag agttttcgcc ccgaagaacg ttttccaatg atgagcactt 4800
   ttaaagttct gctatgtggc gcggtattat cccgtattga cgccgggcaa gagcaactcg 4860
   gtcgccgcat acactattct cagaatgact tggttgagta ctcaccagtc acagaaaagc 4920
   atcttacgga tggcatgaca gtaagagaat tatgcagtgc tgccataacc atgagtgata 4980
   acactgcggc caacttactt ctgacaacga tcggaggacc gaaggagcta accgcttttt 5040
   tgcacaacat gggggatcat gtaactcgcc ttgatcgttg ggaaccggag ctgaatgaag 5100
   ccataccaaa cgacgagcgt gacaccacga tgcctgtagc aatggcaaca acgttgcgca 5160
   aactattaac tggcgaacta cttactctag cttcccggca acaattaata gactggatgg 5220
   aggcggataa agttgcagga ccacttctgc gctcggccct tccggctggc tggtttattg 5280
   ctgataaatc tggagccggt gagcgtgggt ctcgcggtat cattgcagca ctggggccag 5340
   atggtaagcc ctcccgtatc gtagttatct acacgacggg gagtcaggca actatggatg 5400
   aacgaaatag acagatcgct gagataggtg cctcactgat taagcattgg taactgtcag 5460
   accaagttta ctcatatata ctttagattg atttaaaact tcatttttaa tttaaaagga 5520
   tctaggtgaa gatccttttt gataatctca tgaccaaaat cccttaacgt gagttttcgt 5580
   tccactgagc gtcagacccc gtagaaaaga tcaaaggatc ttcttgagat cctttttttc 5640
   tgcgcgtaat ctgctgcttg caaacaaaaa aaccaccgct accagcggtg gtttgtttgc 5700
   cggatcaaga gctaccaact ctttttccga aggtaactgg cttcagcaga gcgcagatac 5760
   caaatactgt tcttctagtg tagccgtagt taggccacca cttcaagaac tctgtagcac 5820
   cgcctacata cctcgctctg ctaatcctgt taccagtggc tgctgccagt ggcgataagt 5880
   cgtgtcttac cgggttggac tcaagacgat agttaccgga taaggcgcag cggtcgggct 5940
   gaacgggggg ttcgtgcaca cagcccagct tggagcgaac gacctacacc gaactgagat 6000
   acctacagcg tgagctatga gaaagcgcca cgcttcccga agggagaaag gcggacaggt 6060
   atccggtaag cggcagggtc ggaacaggag agcgcacgag ggagcttcca gggggaaacg 6120
   cctggtatct ttatagtcct gtcgggtttc gccacctctg acttgagcgt cgatttttgt 6180
   gatgctcgtc aggggggcgg agcctatgga aaaacgccag caacgcggcc tttttacggt 6240
   tcctggcctt ttgctggcct tttgctcaca tgttctttcc tgcgttatcc cctgattctg 6300
   tggataaccg tattaccgcc tttgagtgag ctgataccgc tcgccgcagc cgaacgaccg 6360
   agcgcagcga gtcagtgagc gaggaagcgg aagagcgccc aatacgcaaa ccgcctctcc 6420
   ccgcgcgttg gccgattcat taatgcagct ggcacgacag gtttcccgac tggaaagcgg 6480
   gcagtgagcg caacgcaatt aatgtgagtt agctcactca ttaggcaccc caggctttac 6540
   actttatgct tccggctcgt atgttgtgtg gaattgtgag cggataacaa tttcacacag 6600
   gaaacagcta tgaccatgat tacgccagat ttaattaagg c 6641
<210> 43
   <211> 130
   <212> DNA
   <213> Artificial Sequence
<220>
   <223> Description of Artificial Sequence: Synthetic polynucleotide
<220>
   <223> 5'ITR
<400> 43
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 44
   <211> 382
   <212> DNA
   <213> Artificial Sequence
<220>
   <223> Description of Artificial Sequence: Synthetic polynucleotide
<220>
   <223> CMV IE enhancer
<400> 44
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 45
   <211> 382
   <212> DNA
   <213> Artificial Sequence
<220>
   <223> Description of Artificial Sequence: Synthetic polynucleotide
<220>
   <223> CB promoter
<400> 45
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 46
   <211> 1479
   <212> DNA
   <213> Artificial Sequence
<220>
   <223> Description of Artificial Sequence: Synthetic polynucleotide
<220>
   <223> hGlut1 cDNA
<400> 46
   atggagccca gcagcaagaa gctgacgggt cgcctcatgc tggccgtggg aggagcagtg      60
   cttggctccc tgcagtttgg ctacaacact ggagtcatca atgcccccca gaaggtgatc     120
   gaggagttct acaaccagac atgggtccac cgctatgggg agagcatcct gcccaccacg     180
   ctcaccacgc tctggtccct ctcagtggcc atcttttctg ttgggggcat gattggctcc     240
   ttctctgtgg gccttttcgt taaccgcttt ggccggcgga attcaatgct gatgatgaac     300
   ctgctggcct tcgtgtccgc cgtgctcatg ggcttctcga aactgggcaa gtcctttgag     360
   atgctgatcc tgggccgctt catcatcggt gtgtactgtg gcctgaccac aggcttcgtg     420
   cccatgtatg tgggtgaagt gtcacccaca gcccttcgtg gggccctggg caccctgcac     480
   cagctgggca tcgtcgtcgg catcctcatc gcccaggtgt tcggcctgga ctccatcatg     540
   ggcaacaagg acctgtggcc cctgctgctg agcatcatct tcatcccggc cctgctgcag     600
   tgcatcgtgc tgcccttctg ccccgagagt ccccgcttcc tgctcatcaa ccgcaacgag     660
   gagaaccggg ccaagagtgt gctaaagaag ctgcgcggga cagctgacgt gacccatgac     720
   ctgcaggaga tgaaggaaga gagtcggcag atgatgcggg agaagaaggt caccatcctg     780
   gagctgttcc gctcccccgc ctaccgccag cccatcctca tcgctgtggt gctgcagctg     840
   tcccagcagc tgtctggcat caacgctgtc ttctattact ccacgagcat cttcgagaag     900
   gcgggggtgc agcagcctgt gtatgccacc attggctccg gtatcgtcaa cacggccttc     960
   actgtcgtgt cgctgtttgt ggtggagcga gcaggccggc ggaccctgca cctcataggc    1020
   ctcgctggca tggcgggttg tgccatactc atgaccatcg cgctagcact gctggagcag    1080
   ctaccccgga tgtcctatct gagcatcgtg gccatctttg gctttgtggc cttctttgaa    1140
   gtgggtcctg gccccatccc atggttcatc gtggctgaac tcttcagcca gggtccacgt    1200
   ccagctgcca ttgccgttgc aggcttctcc aactggacct caaatttcat tgtgggcatg    1260
   tgcttccagt atgtggagca actgtgtggt ccctacgtct tcatcatctt cactgtgctc    1320
   ctggttctgt tcttcatctt cacctacttc aaagttcctg agactaaagg ccggaccttc    1380
   gatgagatcg cttccggctt ccggcagggg ggagccagcc aaagtgacaa gacacccgag    1440
   gagctgttcc atcccctggg ggctgattcc caagtgtga                           1479
<210> 47
   <211> 482
   <212> DNA
   <213> Artificial Sequence
<220>
   <223> Description of Artificial Sequence: Synthetic polynucleotide
<220>
   <223> 3'UTR
<400> 47
   gtcgccccag atcaccagcc cggcctgctc ccagcagccc taaggatctc tcaggagcac      60
   aggcagctgg atgagacttc caaacctgac agatgtcagc cgagccgggc ctggggctcc     120
   tttctccagc cagcaatgat gtccagaaga atattcagga cttaacggct ccaggatttt     180
   aacaaaagca agactgttgc tcaaatctat tcagacaagc aacaggtttt ataatttttt     240
   tattactgat tttgttattt ttatatcagc ctgagtctcc tgtgcccaca tcccaggctt     300
   caccctgaat ggttccatgc ctgagggtgg agactaagcc ctgtcgagac acttgccttc     360
   ttcacccagc taatctgtag ggctggacct atgtcctaag gacacactaa tcgaactatg     420
   aactacaaag cttctatccc aggaggtggc tatggccacc cgttctgctg gcctggatct     480
   cc                                                                    482
<210> 48
   <211> 77
   <212> DNA
   <213> Artificial Sequence
<220>
   <223> Description of Artificial Sequence: Synthetic oligonucleotide
<220>
   <223> 3xmiR-122 BS
<400> 48
   cgaaacaaac accattgtca cactccaaca aacaccattg tcacactcca acaaacacca      60
   ttgtcacact ccattcg                                                     77
<210> 49
   <211> 127
   <212> DNA
   <213> Artificial Sequence
<220>
   <223> Description of Artificial Sequence: Synthetic polynucleotide
<220>
   <223> Poly A signal
<400> 49
   gatctttttc cctctgccaa aaattatggg gacatcatga agccccttga gcatctgact      60
   tctggctaat aaaggaaatt tattttcatt gcaatagtgt gttggaattt tttgtgtctc     120
   tcactcg                                                               127
<210> 50
   <211> 130
   <212> DNA
   <213> Artificial Sequence
<220>
   <223> Description of Artificial Sequence: Synthetic polynucleotide
<220>
   <223> 3' ITR
<400> 50
   aggaacccct agtgatggag ttggccactc cctctctgcg cgctcgctcg ctcactgagg        60
   ccgggcgacc aaaggtcgcc cgacgcccgg gctttgcccg ggcggcctca gtgagcgagc       120
   gagcgcgcag                                                              130
<210> 51
   <211> 7137
   <212> DNA
   <213> Artificial Sequence
<220>
   <223> Description of Artificial Sequence: Synthetic polynucleotide
<220>
   <223> pAAV CB6 PI hGlut1-in3xmiR-122 BS
<400> 51
   cttaattagg ctgcgcgctc gctcgctcac tgaggccgcc cgggcaaagc ccgggcgtcg 60
   ggcgaccttt ggtcgcccgg cctcagtgag cgagcgagcg cgcagagagg gagtggccaa 120
   ctccatcact aggggttcct tgtagttaat gattaacccg ccatgctact tatctaccag 180
   ggtaatgggg atcctctaga actatagcta gtcgacattg attattgact agttattaat 240
   agtaatcaat tacggggtca ttagttcata gcccatatat ggagttccgc gttacataac 300
   ttacggtaaa tggcccgcct ggctgaccgc ccaacgaccc ccgcccattg acgtcaataa 360
   tgacgtatgt tcccatagta acgccaatag ggactttcca ttgacgtcaa tgggtggagt 420
   atttacggta aactgcccac ttggcagtac atcaagtgta tcatatgcca agtacgcccc 480
   ctattgacgt caatgacggt aaatggcccg cctggcatta tgcccagtac atgaccttat 540
   gggactttcc tacttggcag tacatctacg tattagtcat cgctattacc atgtcgaggc 600
   cacgttctgc ttcactctcc ccatctcccc cccctcccca cccccaattt tgtatttatt 660
   tattttttaa ttattttgtg cagcgatggg ggcggggggg gggggcgcgc gccaggcggg 720
   gcggggcggg gcgaggggcg gggcggggcg aggcggagag gtgcggcggc agccaatcag 780
   agcggcgcgc tccgaaagtt tccttttatg gcgaggcggc ggcggcggcg gccctataaa 840
   aagcgaagcg cgcggcgggc gggagcaagc tttattgcgg tagtttatca cagttaaatt 900
   gctaacgcag tcagtgcttc tgacacaaca gtctcgaact taagctgcag aagttggtcg 960
   tgaggcactg ggcaggtaag tatcaaggtt acaagacagg tttaaggaga ccaatagaaa 1020
   ctgggcttgt cgagacagag aagactcttg cgtttctgat aggcacctat tggtcttact 1080
   gacatccact ttgcctttct ctccacaggt gtccactccc agttcaatta cagctcttaa 1140
   ggctagagta cttaatacga ctcactatag gctagcgcgc cgaattcggc acgaggaaaa 1200
   aggcagctcc gcgcgctctc ccccaagagc agaggcttgc ttgtagagtg acgatctgag 1260
   ctacggggtc ttaagtgcgt cagggcgtgg aggtctggcg ggagacgcat agttacagcg 1320
   cgtccgttct ccgtctcgca gccggcacag ctagagcttc gagcgcagcg cggccatgga 1380
   gcccagcagc aagaagctga cgggtcgcct catgctggcc gtgggaggag cagtgcttgg 1440
   ctccctgcag tttggctaca acactggagt catcaatgcc ccccagaagg tgatcgagga 1500
   gttctacaac cagacatggg tccaccgcta tggggagagc atcctgccca ccacgctcac 1560
   cacgctctgg tccctctcag tggccatctt ttctgttggg ggcatgattg gctccttctc 1620
   tgtgggcctt ttcgttaacc gctttggccg gcggaattca atgctgatga tgaacctgct 1680
   ggccttcgtg tccgccgtgc tcatgggctt ctcgaaactg ggcaagtcct ttgagatgct 1740
   gatcctgggc cgcttcatca tcggtgtgta ctgtggcctg accacaggct tcgtgcccat 1800
   gtatgtgggt gaagtgtcac ccacagccct tcgtggggcc ctgggcaccc tgcaccagct 1860
   gggcatcgtc gtcggcatcc tcatcgccca ggtgttcggc ctggactcca tcatgggcaa 1920
   caaggacctg tggcccctgc tgctgagcat catcttcatc ccggccctgc tgcagtgcat 1980
   cgtgctgccc ttctgccccg agagtccccg cttcctgctc atcaaccgca acgaggagaa 2040
   ccgggccaag agtgtgctaa agaagctgcg cgggacagct gacgtgaccc atgacctgca 2100
   ggagatgaag gaagagagtc ggcagatgat gcgggagaag aaggtcacca tcctggagct 2160
   gttccgctcc cccgcctacc gccagcccat cctcatcgct gtggtgctgc agctgtccca 2220
   gcagctgtct ggcatcaacg ctgtcttcta ttactccacg agcatcttcg agaaggcggg 2280
   ggtgcagcag cctgtgtatg ccaccattgg ctccggtatc gtcaacacgg ccttcactgt 2340
   cgtgtcgctg tttgtggtgg agcgagcagg ccggcggacc ctgcacctca taggcctcgc 2400
   tggcatggcg ggttgtgcca tactcatgac catcgcgcta gcactgctgg agcagctacc 2460
   ccggatgtcc tatctgagca tcgtggccat ctttggcttt gtggccttct ttgaagtggg 2520
   tcctggcccc atcccatggt tcatcgtggc tgaactcttc agccagggtc cacgtccagc 2580
   tgccattgcc gttgcaggct tctccaactg gacctcaaat ttcattgtgg gcatgtgctt 2640
   ccagtatgtg gagcaactgt gtggtcccta cgtcttcatc atcttcactg tgctcctggt 2700
   tctgttcttc atcttcacct acttcaaagt tcctgagact aaaggccgga ccttcgatga 2760
   gatcgcttcc ggcttccggc aggggggagc cagccaaagt gacaagacac ccgaggagct 2820
   gttccatccc ctgggggctg attcccaagt gtgaggagcc ccacacccag cccggcctgc 2880
   tccctgcagc ccaaggatct ctctggagca caggcagcta gatgagacct cttccgaacc 2940
   gacagatctc gggcaagccg ggcctgggcg cctttcctca gccagcagtg aagtccagga 3000
   ggatattcag gactttgatg gctccagaat ttttaatgaa agcaagactg ctgctcagat 3060
   ctattcagat aagcagcagg ttttataatt tttttattac tgattttgtt attttttttt 3120
   tttatcagcc actctcctat ctccacactg tagtcttcac cttgattggc ccagtgcctg 3180
   agggtgggga ccacgccctg tccagacact tgccttcttt gccaagctaa tctgtagggc 3240
   tggacctatg gccaaggaca cactaatacc gaactctgag ctaggaggct ttaccgctgg 3300
   aggcggtagc tgccacccac ttccgcaggc ctggacctcg gcaccatagg ggtccggact 3360
   ccattttagg attcgcccat tcctgtctct tcctacccaa ccactcaatt aatctttcct 3420
   tgcctgagac cagttggaag cactggagtg cagggaggag agggaagggc caggctgggc 3480
   tgccaggttc tagtctcctg tgcactgagg gccacacaaa caccatgaga aggaccgaaa 3540
   caaacaccat tgtcacactc caacaaacac cattgtcaca ctccaacaaa caccattgtc 3600
   acactccatt cggacctcgg aggctgagaa cttaactgct gaagacacgg acactcctgc 3660
   cctgctgtgt atagatggaa gatatttata tattttttgg ttgtcaatat taaatacaga 3720
   cactaagtta tagtatatct ggacaaaccc acttgtaaat acaccaacaa actcctgtaa 3780
   ctttacctaa gcagatataa atggctggtt tttagaaaaa aaaaaaaaaa aaaaaaaaaa 3840
   aaaaaaaaaa aaaaaaaaaa aaaaaaaaaa aaaaaaaaaa aaaaaaaaaa actgtctcgc 3900
   tgcggccgct ctagagtatc cctcgactct agagtcgacc cgggcggcct cgaggacggg 3960
   gtgaactacg cctgaggatc cgatcttttt ccctctgcca aaaattatgg ggacatcatg 4020
   aagccccttg agcatctgac ttctggctaa taaaggaaat ttattttcat tgcaatagtg 4080
   tgttggaatt ttttgtgtct ctcactcgga agcaattcgt tgatctgaat ttcgaccacc 4140
   cataataccc attaccctgg tagataagta gcatggcggg ttaatcatta actacaagga 4200
   acccctagtg atggagttgg ccactccctc tctgcgcgct cgctcgctca ctgaggccgg 4260
   gcgaccaaag gtcgcccgac gcccgggctt tgcccgggcg gcctcagtga gcgagcgagc 4320
   gcgcagcctt aattaaccta attcactggc cgtcgtttta caacgtcgtg actgggaaaa 4380
   ccctggcgtt acccaactta atcgccttgc agcacatccc cctttcgcca gctggcgtaa 4440
   tagcgaagag gcccgcaccg atcgcccttc ccaacagttg cgcagcctga atggcgaatg 4500
   ggacgcgccc tgtagcggcg cattaagcgc ggcgggtgtg gtggttacgc gcagcgtgac 4560
   cgctacactt gccagcgccc tagcgcccgc tcctttcgct ttcttccctt cctttctcgc 4620
   cacgttcgcc ggctttcccc gtcaagctct aaatcggggg ctccctttag ggttccgatt 4680
   tagtgcttta cggcacctcg accccaaaaa acttgattag ggtgatggtt cacgtagtgg 4740
   gccatcgccc tgatagacgg tttttcgccc tttgacgttg gagtccacgt tctttaatag 4800
   tggactcttg ttccaaactg gaacaacact caaccctatc tcggtctatt cttttgattt 4860
   ataagggatt ttgccgattt cggcctattg gttaaaaaat gagctgattt aacaaaaatt 4920
   taacgcgaat tttaacaaaa tattaacgct tacaatttag gtggcacttt tcggggaaat 4980
   gtgcgcggaa cccctatttg tttatttttc taaatacatt caaatatgta tccgctcatg 5040
   agacaataac cctgataaat gcttcaataa tattgaaaaa ggaagagtat gagtattcaa 5100
   catttccgtg tcgcccttat tccctttttt gcggcatttt gccttcctgt ttttgctcac 5160
   ccagaaacgc tggtgaaagt aaaagatgct gaagatcagt tgggtgcacg agtgggttac 5220
   atcgaactgg atctcaacag cggtaagatc cttgagagtt ttcgccccga agaacgtttt 5280
   ccaatgatga gcacttttaa agttctgcta tgtggcgcgg tattatcccg tattgacgcc 5340
   gggcaagagc aactcggtcg ccgcatacac tattctcaga atgacttggt tgagtactca 5400
   ccagtcacag aaaagcatct tacggatggc atgacagtaa gagaattatg cagtgctgcc 5460
   ataaccatga gtgataacac tgcggccaac ttacttctga caacgatcgg aggaccgaag 5520
   gagctaaccg cttttttgca caacatgggg gatcatgtaa ctcgccttga tcgttgggaa 5580
   ccggagctga atgaagccat accaaacgac gagcgtgaca ccacgatgcc tgtagcaatg 5640
   gcaacaacgt tgcgcaaact attaactggc gaactactta ctctagcttc ccggcaacaa 5700
   ttaatagact ggatggaggc ggataaagtt gcaggaccac ttctgcgctc ggcccttccg 5760
   gctggctggt ttattgctga taaatctgga gccggtgagc gtgggtctcg cggtatcatt 5820
   gcagcactgg ggccagatgg taagccctcc cgtatcgtag ttatctacac gacggggagt 5880
   caggcaacta tggatgaacg aaatagacag atcgctgaga taggtgcctc actgattaag 5940
   cattggtaac tgtcagacca agtttactca tatatacttt agattgattt aaaacttcat 6000
   ttttaattta aaaggatcta ggtgaagatc ctttttgata atctcatgac caaaatccct 6060
   taacgtgagt tttcgttcca ctgagcgtca gaccccgtag aaaagatcaa aggatcttct 6120
   tgagatcctt tttttctgcg cgtaatctgc tgcttgcaaa caaaaaaacc accgctacca 6180
   gcggtggttt gtttgccgga tcaagagcta ccaactcttt ttccgaaggt aactggcttc 6240
   agcagagcgc agataccaaa tactgttctt ctagtgtagc cgtagttagg ccaccacttc 6300
   aagaactctg tagcaccgcc tacatacctc gctctgctaa tcctgttacc agtggctgct 6360
   gccagtggcg ataagtcgtg tcttaccggg ttggactcaa gacgatagtt accggataag 6420
   gcgcagcggt cgggctgaac ggggggttcg tgcacacagc ccagcttgga gcgaacgacc 6480
   tacaccgaac tgagatacct acagcgtgag ctatgagaaa gcgccacgct tcccgaaggg 6540
   agaaaggcgg acaggtatcc ggtaagcggc agggtcggaa caggagagcg cacgagggag 6600
   cttccagggg gaaacgcctg gtatctttat agtcctgtcg ggtttcgcca cctctgactt 6660
   gagcgtcgat ttttgtgatg ctcgtcaggg gggcggagcc tatggaaaaa cgccagcaac 6720
   gcggcctttt tacggttcct ggccttttgc tggccttttg ctcacatgtt ctttcctgcg 6780
   ttatcccctg attctgtgga taaccgtatt accgcctttg agtgagctga taccgctcgc 6840
   cgcagccgaa cgaccgagcg cagcgagtca gtgagcgagg aagcggaaga gcgcccaata 6900
   cgcaaaccgc ctctccccgc gcgttggccg attcattaat gcagctggca cgacaggttt 6960
   cccgactgga aagcgggcag tgagcgcaac gcaattaatg tgagttagct cactcattag 7020
   gcaccccagg ctttacactt tatgcttccg gctcgtatgt tgtgtggaat tgtgagcgga 7080
   taacaatttc acacaggaaa cagctatgac catgattacg ccagatttaa ttaaggc 7137
<210> 52
   <211> 130
   <212> DNA
   <213> Artificial Sequence
<220>
   <223> Description of Artificial Sequence: Synthetic polynucleotide
<220>
   <223> 5'ITR
<400> 52
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 53
   <211> 382
   <212> DNA
   <213> Artificial Sequence
<220>
   <223> Description of Artificial Sequence: Synthetic polynucleotide
<220>
   <223> CMV IE enhancer
<400> 53
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 54
   <211> 382
   <212> DNA
   <213> Artificial Sequence
<220>
   <223> Description of Artificial Sequence: Synthetic polynucleotide
<220>
   <223> CB promoter
<400> 54
   ctagtcgaca ttgattattg actagttatt aatagtaatc aattacgggg tcattagttc        60
   atagcccata tatggagttc cgcgttacat aacttacggt aaatggcccg cctggctgac       120
   cgcccaacga cccccgccca ttgacgtcaa taatgacgta tgttcccata gtaacgccaa       180
   tagggacttt ccattgacgt caatgggtgg agtatttacg gtaaactgcc cacttggcag       240
   tacatcaagt gtatcatatg ccaagtacgc cccctattga cgtcaatgac ggtaaatggc       300
   ccgcctggca ttatgcccag tacatgacct tatgggactt tcctacttgg cagtacatct       360
   acgtattagt catcgctatt ac                                                382
<210> 55
   <211> 1479
   <212> DNA
   <213> Artificial Sequence
<220>
   <223> Description of Artificial Sequence: Synthetic polynucleotide
<220>
   <223> hGlut1 cDNA
<400> 55
   atggagccca gcagcaagaa gctgacgggt cgcctcatgc tggccgtggg aggagcagtg        60
   cttggctccc tgcagtttgg ctacaacact ggagtcatca atgcccccca gaaggtgatc       120
   gaggagttct acaaccagac atgggtccac cgctatgggg agagcatcct gcccaccacg       180
   ctcaccacgc tctggtccct ctcagtggcc atcttttctg ttgggggcat gattggctcc       240
   ttctctgtgg gccttttcgt taaccgcttt ggccggcgga attcaatgct gatgatgaac       300
   ctgctggcct tcgtgtccgc cgtgctcatg ggcttctcga aactgggcaa gtcctttgag       360
   atgctgatcc tgggccgctt catcatcggt gtgtactgtg gcctgaccac aggcttcgtg       420
   cccatgtatg tgggtgaagt gtcacccaca gcccttcgtg gggccctggg caccctgcac       480
   cagctgggca tcgtcgtcgg catcctcatc gcccaggtgt tcggcctgga ctccatcatg       540
   ggcaacaagg acctgtggcc cctgctgctg agcatcatct tcatcccggc cctgctgcag       600
   tgcatcgtgc tgcccttctg ccccgagagt ccccgcttcc tgctcatcaa ccgcaacgag       660
   gagaaccggg ccaagagtgt gctaaagaag ctgcgcggga cagctgacgt gacccatgac       720
   ctgcaggaga tgaaggaaga gagtcggcag atgatgcggg agaagaaggt caccatcctg       780
   gagctgttcc gctcccccgc ctaccgccag cccatcctca tcgctgtggt gctgcagctg       840
   tcccagcagc tgtctggcat caacgctgtc ttctattact ccacgagcat cttcgagaag       900
   gcgggggtgc agcagcctgt gtatgccacc attggctccg gtatcgtcaa cacggccttc       960
   actgtcgtgt cgctgtttgt ggtggagcga gcaggccggc ggaccctgca cctcataggc      1020
   ctcgctggca tggcgggttg tgccatactc atgaccatcg cgctagcact gctggagcag      1080
   ctaccccgga tgtcctatct gagcatcgtg gccatctttg gctttgtggc cttctttgaa      1140
   gtgggtcctg gccccatccc atggttcatc gtggctgaac tcttcagcca gggtccacgt      1200
   ccagctgcca ttgccgttgc aggcttctcc aactggacct caaatttcat tgtgggcatg      1260
   tgcttccagt atgtggagca actgtgtggt ccctacgtct tcatcatctt cactgtgctc      1320
   ctggttctgt tcttcatctt cacctacttc aaagttcctg agactaaagg ccggaccttc      1380
   gatgagatcg cttccggctt ccggcagggg ggagccagcc aaagtgacaa gacacccgag      1440
   gagctgttcc atcccctggg ggctgattcc caagtgtga                              1479
<210> 56
   <211> 1035
   <212> DNA
   <213> Artificial Sequence
<220>
   <223> Description of Artificial Sequence: Synthetic polynucleotide
<220>
   <223> 3' UTR and 3xmiR-122
<400> 56
   ggagccccac acccagcccg gcctgctccc tgcagcccaa ggatctctct ggagcacagg      60
   cagctagatg agacctcttc cgaaccgaca gatctcgggc aagccgggcc tgggcgcctt     120
   tcctcagcca gcagtgaagt ccaggaggat attcaggact ttgatggctc cagaattttt     180
   aatgaaagca agactgctgc tcagatctat tcagataagc agcaggtttt ataatttttt     240
   tattactgat tttgttattt ttttttttta tcagccactc tcctatctcc acactgtagt     300
   cttcaccttg attggcccag tgcctgaggg tggggaccac gccctgtcca gacacttgcc     360
   ttctttgcca agctaatctg tagggctgga cctatggcca aggacacact aataccgaac     420
   tctgagctag gaggctttac cgctggaggc ggtagctgcc acccacttcc gcaggcctgg     480
   acctcggcac cataggggtc cggactccat tttaggattc gcccattcct gtctcttcct      540
   acccaaccac tcaattaatc tttccttgcc tgagaccagt tggaagcact ggagtgcagg     600
   gaggagaggg aagggccagg ctgggctgcc aggttctagt ctcctgtgca ctgagggcca     660
   cacaaacacc atgagaagga ccgaaacaaa caccattgtc acactccaac aaacaccatt     720
   gtcacactcc aacaaacacc attgtcacac tccattcgga cctcggaggc tgagaactta     780
   actgctgaag acacggacac tcctgccctg ctgtgtatag atggaagata tttatatatt     840
   ttttggttgt caatattaaa tacagacact aagttatagt atatctggac aaacccactt     900
   gtaaatacac caacaaactc ctgtaacttt acctaagcag atataaatgg ctggttttta     960
   gaaaaaaaaa aaaaaaaaaa aaaaaaaaaa aaaaaaaaaa aaaaaaaaaa aaaaaaaaaa    1020
   aaaaaaaaaa aaaaa                                                     1035
<210> 57
   <211> 127
   <212> DNA
   <213> Artificial Sequence
<220>
   <223> Description of Artificial Sequence: Synthetic polynucleotide
<220>
   <223> Poly A signal
<400> 57
   gatctttttc cctctgccaa aaattatggg gacatcatga agccccttga gcatctgact      60
   tctggctaat aaaggaaatt tattttcatt gcaatagtgt gttggaattt tttgtgtctc     120
   tcactcg                                                               127
<210> 58
   <211> 130
   <212> DNA
   <213> Artificial Sequence
<220>
   <223> Description of Artificial Sequence: Synthetic polynucleotide
<220>
   <223> 3' ITR
<400> 58
   aggaacccct agtgatggag ttggccactc cctctctgcg cgctcgctcg ctcactgagg        60
   ccgggcgacc aaaggtcgcc cgacgcccgg gctttgcccg ggcggcctca gtgagcgagc       120
   gagcgcgcag                                                              130
<210> 59
   <211> 7137
   <212> DNA
   <213> Artificial Sequence
<220>
   <223> Description of Artificial Sequence: Synthetic polynucleotide
<220>
   <223> pAAV CB6 PI mGlut1-in3xmiR-122 BS
<400> 59
   cttaattagg ctgcgcgctc gctcgctcac tgaggccgcc cgggcaaagc ccgggcgtcg        60
   ggcgaccttt ggtcgcccgg cctcagtgag cgagcgagcg cgcagagagg gagtggccaa       120
   ctccatcact aggggttcct tgtagttaat gattaacccg ccatgctact tatctaccag 180
   ggtaatgggg atcctctaga actatagcta gtcgacattg attattgact agttattaat 240
   agtaatcaat tacggggtca ttagttcata gcccatatat ggagttccgc gttacataac 300
   ttacggtaaa tggcccgcct ggctgaccgc ccaacgaccc ccgcccattg acgtcaataa 360
   tgacgtatgt tcccatagta acgccaatag ggactttcca ttgacgtcaa tgggtggagt 420
   atttacggta aactgcccac ttggcagtac atcaagtgta tcatatgcca agtacgcccc 480
   ctattgacgt caatgacggt aaatggcccg cctggcatta tgcccagtac atgaccttat 540
   gggactttcc tacttggcag tacatctacg tattagtcat cgctattacc atgtcgaggc 600
   cacgttctgc ttcactctcc ccatctcccc cccctcccca cccccaattt tgtatttatt 660
   tattttttaa ttattttgtg cagcgatggg ggcggggggg gggggcgcgc gccaggcggg 720
   gcggggcggg gcgaggggcg gggcggggcg aggcggagag gtgcggcggc agccaatcag 780
   agcggcgcgc tccgaaagtt tccttttatg gcgaggcggc ggcggcggcg gccctataaa 840
   aagcgaagcg cgcggcgggc gggagcaagc tttattgcgg tagtttatca cagttaaatt 900
   gctaacgcag tcagtgcttc tgacacaaca gtctcgaact taagctgcag aagttggtcg 960
   tgaggcactg ggcaggtaag tatcaaggtt acaagacagg tttaaggaga ccaatagaaa 1020
   ctgggcttgt cgagacagag aagactcttg cgtttctgat aggcacctat tggtcttact 1080
   gacatccact ttgcctttct ctccacaggt gtccactccc agttcaatta cagctcttaa 1140
   ggctagagta cttaatacga ctcactatag gctagcgcgc cgaattcggc acgaggaaaa 1200
   aggcagctcc gcgcgctctc ccccaagagc agaggcttgc ttgtagagtg acgatctgag 1260
   ctacggggtc ttaagtgcgt cagggcgtgg aggtctggcg ggagacgcat agttacagcg 1320
   cgtccgttct ccgtctcgca gccggcacag ctagagcttc gagcgcagcg cggccatgga 1380
   tcccagcagc aagaaggtga cgggccgcct catgttggct gtgggaggag cagtgctcgg 1440
   atcactgcag ttcggctata acactggtgt catcaacgcc ccccagaagg ttattgagga 1500
   gttctacaat caaacatgga accaccgcta cggagagccc atcccatcca ccacactcac 1560
   cacgctttgg tctctctccg tggccatctt ctctgtcggg ggcatgattg gttccttctc 1620
   tgtcggcctc tttgttaatc gctttggcag gcggaactcc atgctgatga tgaacctgtt 1680
   ggcctttgtg gctgctgtgc ttatgggctt ctccaaactg ggcaagtcct ttgagatgct 1740
   gatcctgggc cgcttcatca tcggtgtgta ctgcggcctg actactggct ttgtgcccat 1800
   gtatgtggga gaggtgtcac ctacagctct acgtggagcc ctaggcacac tgcaccagct 1860
   gggaatcgtc gttggcatcc ttattgccca ggtgtttggc ttagactcca tcatgggcaa 1920
   tgcagacttg tggcctctgc tgctcagtgt catcttcatc ccagccctgc tacagtgtat 1980
   cctgttgccc ttctgccccg agagcccccg cttcctgctc atcaatcgta acgaggagaa 2040
   ccgggccaag agtgtgctga agaagcttcg agggacagcc gatgtgaccc gagacctgca 2100
   ggagatgaaa gaagagggtc ggcagatgat gcgggagaag aaggtcacca tcttggagct 2160
   gttccgctca cccgcctacc gccagcccat cctcatcgct gtggtgctgc agctgtccca 2220
   gcagctgtcg ggtatcaatg ctgtgttcta ctactcaacg agcatcttcg agaaggcagg 2280
   tgtgcagcag cctgtgtacg ccaccatcgg ctccggtatc gtcaacacgg ccttcactgt 2340
   ggtgtcgctg tttgttgtag agcgagctgg acgacggacc ctgcacctca ttggcctggc 2400
   tggcatggca ggctgtgctg tgctcatgac catcgccctg gccttgctgg aacggctgcc 2460
   ttggatgtcc tatctgagca tcgtggccat ctttggcttt gtggccttct ttgaagtagg 2520
   ccctggtcct attccatggt tcattgtggc cgagctgttc agccaggggc cccgtcctgc 2580
   tgctattgct gtggctggct tctccaactg gacctcaaac ttcattgtgg gcatgtgctt 2640
   ccagtatgtg gagcaactgt gcggccccta cgtcttcatc atcttcacgg tgctcctcgt 2700
   gctcttcttc atcttcacct acttcaaagt ccctgagacc aaaggccgaa ccttcgatga 2760
   gatcgcttcc ggcttccggc aggggggtgc cagccaaagt gacaagacac ccgaggagct 2820
   cttccaccct ctgggggcgg actcccaagt gtgaggagcc ccacacccag cccggcctgc 2880
   tccctgcagc ccaaggatct ctctggagca caggcagcta gatgagacct cttccgaacc 2940
   gacagatctc gggcaagccg ggcctgggcg cctttcctca gccagcagtg aagtccagga 3000
   ggatattcag gactttgatg gctccagaat ttttaatgaa agcaagactg ctgctcagat 3060
   ctattcagat aagcagcagg ttttataatt tttttattac tgattttgtt attttttttt 3120
   tttatcagcc actctcctat ctccacactg tagtcttcac cttgattggc ccagtgcctg 3180
   agggtgggga ccacgccctg tccagacact tgccttcttt gccaagctaa tctgtagggc 3240
   tggacctatg gccaaggaca cactaatacc gaactctgag ctaggaggct ttaccgctgg 3300
   aggcggtagc tgccacccac ttccgcaggc ctggacctcg gcaccatagg ggtccggact 3360
   ccattttagg attcgcccat tcctgtctct tcctacccaa ccactcaatt aatctttcct 3420
   tgcctgagac cagttggaag cactggagtg cagggaggag agggaagggc caggctgggc 3480
   tgccaggttc tagtctcctg tgcactgagg gccacacaaa caccatgaga aggaccgaaa 3540
   caaacaccat tgtcacactc caacaaacac cattgtcaca ctccaacaaa caccattgtc 3600
   acactccatt cggacctcgg aggctgagaa cttaactgct gaagacacgg acactcctgc 3660
   cctgctgtgt atagatggaa gatatttata tattttttgg ttgtcaatat taaatacaga 3720
   cactaagtta tagtatatct ggacaaaccc acttgtaaat acaccaacaa actcctgtaa 3780
   ctttacctaa gcagatataa atggctggtt tttagaaaaa aaaaaaaaaa aaaaaaaaaa 3840
   aaaaaaaaaa aaaaaaaaaa aaaaaaaaaa aaaaaaaaaa aaaaaaaaaa actgtctcgc 3900
   tgcggccgct ctagagtatc cctcgactct agagtcgacc cgggcggcct cgaggacggg 3960
   gtgaactacg cctgaggatc cgatcttttt ccctctgcca aaaattatgg ggacatcatg 4020
   aagccccttg agcatctgac ttctggctaa taaaggaaat ttattttcat tgcaatagtg 4080
   tgttggaatt ttttgtgtct ctcactcgga agcaattcgt tgatctgaat ttcgaccacc 4140
   cataataccc attaccctgg tagataagta gcatggcggg ttaatcatta actacaagga 4200
   acccctagtg atggagttgg ccactccctc tctgcgcgct cgctcgctca ctgaggccgg 4260
   gcgaccaaag gtcgcccgac gcccgggctt tgcccgggcg gcctcagtga gcgagcgagc 4320
   gcgcagcctt aattaaccta attcactggc cgtcgtttta caacgtcgtg actgggaaaa 4380
   ccctggcgtt acccaactta atcgccttgc agcacatccc cctttcgcca gctggcgtaa 4440
   tagcgaagag gcccgcaccg atcgcccttc ccaacagttg cgcagcctga atggcgaatg 4500
   ggacgcgccc tgtagcggcg cattaagcgc ggcgggtgtg gtggttacgc gcagcgtgac 4560
   cgctacactt gccagcgccc tagcgcccgc tcctttcgct ttcttccctt cctttctcgc 4620
   cacgttcgcc ggctttcccc gtcaagctct aaatcggggg ctccctttag ggttccgatt 4680
   tagtgcttta cggcacctcg accccaaaaa acttgattag ggtgatggtt cacgtagtgg 4740
   gccatcgccc tgatagacgg tttttcgccc tttgacgttg gagtccacgt tctttaatag 4800
   tggactcttg ttccaaactg gaacaacact caaccctatc tcggtctatt cttttgattt 4860
   ataagggatt ttgccgattt cggcctattg gttaaaaaat gagctgattt aacaaaaatt 4920
   taacgcgaat tttaacaaaa tattaacgct tacaatttag gtggcacttt tcggggaaat 4980
   gtgcgcggaa cccctatttg tttatttttc taaatacatt caaatatgta tccgctcatg 5040
   agacaataac cctgataaat gcttcaataa tattgaaaaa ggaagagtat gagtattcaa 5100
   catttccgtg tcgcccttat tccctttttt gcggcatttt gccttcctgt ttttgctcac 5160
   ccagaaacgc tggtgaaagt aaaagatgct gaagatcagt tgggtgcacg agtgggttac 5220
   atcgaactgg atctcaacag cggtaagatc cttgagagtt ttcgccccga agaacgtttt 5280
   ccaatgatga gcacttttaa agttctgcta tgtggcgcgg tattatcccg tattgacgcc 5340
   gggcaagagc aactcggtcg ccgcatacac tattctcaga atgacttggt tgagtactca 5400
   ccagtcacag aaaagcatct tacggatggc atgacagtaa gagaattatg cagtgctgcc 5460
   ataaccatga gtgataacac tgcggccaac ttacttctga caacgatcgg aggaccgaag 5520
   gagctaaccg cttttttgca caacatgggg gatcatgtaa ctcgccttga tcgttgggaa 5580
   ccggagctga atgaagccat accaaacgac gagcgtgaca ccacgatgcc tgtagcaatg 5640
   gcaacaacgt tgcgcaaact attaactggc gaactactta ctctagcttc ccggcaacaa 5700
   ttaatagact ggatggaggc ggataaagtt gcaggaccac ttctgcgctc ggcccttccg 5760
   gctggctggt ttattgctga taaatctgga gccggtgagc gtgggtctcg cggtatcatt 5820
   gcagcactgg ggccagatgg taagccctcc cgtatcgtag ttatctacac gacggggagt 5880
   caggcaacta tggatgaacg aaatagacag atcgctgaga taggtgcctc actgattaag 5940
   cattggtaac tgtcagacca agtttactca tatatacttt agattgattt aaaacttcat 6000
   ttttaattta aaaggatcta ggtgaagatc ctttttgata atctcatgac caaaatccct 6060
   taacgtgagt tttcgttcca ctgagcgtca gaccccgtag aaaagatcaa aggatcttct 6120
   tgagatcctt tttttctgcg cgtaatctgc tgcttgcaaa caaaaaaacc accgctacca 6180
   gcggtggttt gtttgccgga tcaagagcta ccaactcttt ttccgaaggt aactggcttc 6240
   agcagagcgc agataccaaa tactgttctt ctagtgtagc cgtagttagg ccaccacttc 6300
   aagaactctg tagcaccgcc tacatacctc gctctgctaa tcctgttacc agtggctgct 6360
   gccagtggcg ataagtcgtg tcttaccggg ttggactcaa gacgatagtt accggataag 6420
   gcgcagcggt cgggctgaac ggggggttcg tgcacacagc ccagcttgga gcgaacgacc 6480
   tacaccgaac tgagatacct acagcgtgag ctatgagaaa gcgccacgct tcccgaaggg 6540
   agaaaggcgg acaggtatcc ggtaagcggc agggtcggaa caggagagcg cacgagggag 6600
   cttccagggg gaaacgcctg gtatctttat agtcctgtcg ggtttcgcca cctctgactt 6660
   gagcgtcgat ttttgtgatg ctcgtcaggg gggcggagcc tatggaaaaa cgccagcaac 6720
   gcggcctttt tacggttcct ggccttttgc tggccttttg ctcacatgtt ctttcctgcg 6780
   ttatcccctg attctgtgga taaccgtatt accgcctttg agtgagctga taccgctcgc 6840
   cgcagccgaa cgaccgagcg cagcgagtca gtgagcgagg aagcggaaga gcgcccaata 6900
   cgcaaaccgc ctctccccgc gcgttggccg attcattaat gcagctggca cgacaggttt 6960
   cccgactgga aagcgggcag tgagcgcaac gcaattaatg tgagttagct cactcattag 7020
   gcaccccagg ctttacactt tatgcttccg gctcgtatgt tgtgtggaat tgtgagcgga 7080
   taacaatttc acacaggaaa cagctatgac catgattacg ccagatttaa ttaaggc 7137
<210> 60
   <211> 130
   <212> DNA
   <213> Artificial Sequence
<220>
   <223> Description of Artificial Sequence: Synthetic polynucleotide
<220>
   <223> 5'ITR
<400> 60
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 61
   <211> 382
   <212> DNA
   <213> Artificial Sequence
<220>
   <223> Description of Artificial Sequence: Synthetic polynucleotide
<220>
   <223> CMV IE enhancer
<400> 61
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 62
   <211> 382
   <212> DNA
   <213> Artificial Sequence
<220>
   <223> Description of Artificial Sequence: Synthetic polynucleotide
<220>
   <223> CB promoter
<400> 62
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 63
   <211> 1479
   <212> DNA
   <213> Artificial Sequence
<220>
   <223> Description of Artificial Sequence: Synthetic polynucleotide
<220>
   <223> mGlut1 cDNA
<400> 63
   atggatccca gcagcaagaa ggtgacgggc cgcctcatgt tggctgtggg aggagcagtg 60
   ctcggatcac tgcagttcgg ctataacact ggtgtcatca acgcccccca gaaggttatt 120
   gaggagttct acaatcaaac atggaaccac cgctacggag agcccatccc atccaccaca 180
   ctcaccacgc tttggtctct ctccgtggcc atcttctctg tcgggggcat gattggttcc 240
   ttctctgtcg gcctctttgt taatcgcttt ggcaggcgga actccatgct gatgatgaac 300
   ctgttggcct ttgtggctgc tgtgcttatg ggcttctcca aactgggcaa gtcctttgag 360
   atgctgatcc tgggccgctt catcatcggt gtgtactgcg gcctgactac tggctttgtg 420
   cccatgtatg tgggagaggt gtcacctaca gctctacgtg gagccctagg cacactgcac 480
   cagctgggaa tcgtcgttgg catccttatt gcccaggtgt ttggcttaga ctccatcatg 540
   ggcaatgcag acttgtggcc tctgctgctc agtgtcatct tcatcccagc cctgctacag 600
   tgtatcctgt tgcccttctg ccccgagagc ccccgcttcc tgctcatcaa tcgtaacgag 660
   gagaaccggg ccaagagtgt gctgaagaag cttcgaggga cagccgatgt gacccgagac 720
   ctgcaggaga tgaaagaaga gggtcggcag atgatgcggg agaagaaggt caccatcttg 780
   gagctgttcc gctcacccgc ctaccgccag cccatcctca tcgctgtggt gctgcagctg 840
   tcccagcagc tgtcgggtat caatgctgtg ttctactact caacgagcat cttcgagaag 900
   gcaggtgtgc agcagcctgt gtacgccacc atcggctccg gtatcgtcaa cacggccttc 960
   actgtggtgt cgctgtttgt tgtagagcga gctggacgac ggaccctgca cctcattggc 1020
   ctggctggca tggcaggctg tgctgtgctc atgaccatcg ccctggcctt gctggaacgg 1080
   ctgccttgga tgtcctatct gagcatcgtg gccatctttg gctttgtggc cttctttgaa 1140
   gtaggccctg gtcctattcc atggttcatt gtggccgagc tgttcagcca ggggccccgt 1200
   cctgctgcta ttgctgtggc tggcttctcc aactggacct caaacttcat tgtgggcatg 1260
   tgcttccagt atgtggagca actgtgcggc ccctacgtct tcatcatctt cacggtgctc 1320
   ctcgtgctct tcttcatctt cacctacttc aaagtccctg agaccaaagg ccgaaccttc 1380
   gatgagatcg cttccggctt ccggcagggg ggtgccagcc aaagtgacaa gacacccgag 1440
   gagctcttcc accctctggg ggcggactcc caagtgtga 1479
<210> 64
   <211> 1035
   <212> DNA
   <213> Artificial Sequence
<220>
   <223> Description of Artificial Sequence: Synthetic polynucleotide
<220>
   <223> 3'UTR and 3x-miR122BS
<400> 64
   ggagccccac acccagcccg gcctgctccc tgcagcccaa ggatctctct ggagcacagg      60
   cagctagatg agacctcttc cgaaccgaca gatctcgggc aagccgggcc tgggcgcctt     120
   tcctcagcca gcagtgaagt ccaggaggat attcaggact ttgatggctc cagaattttt     180
   aatgaaagca agactgctgc tcagatctat tcagataagc agcaggtttt ataatttttt     240
   tattactgat tttgttattt ttttttttta tcagccactc tcctatctcc acactgtagt     300
   cttcaccttg attggcccag tgcctgaggg tggggaccac gccctgtcca gacacttgcc     360
   ttctttgcca agctaatctg tagggctgga cctatggcca aggacacact aataccgaac     420
   tctgagctag gaggctttac cgctggaggc ggtagctgcc acccacttcc gcaggcctgg     480
   acctcggcac cataggggtc cggactccat tttaggattc gcccattcct gtctcttcct      540
   acccaaccac tcaattaatc tttccttgcc tgagaccagt tggaagcact ggagtgcagg     600
   gaggagaggg aagggccagg ctgggctgcc aggttctagt ctcctgtgca ctgagggcca     660
   cacaaacacc atgagaagga ccgaaacaaa caccattgtc acactccaac aaacaccatt     720
   gtcacactcc aacaaacacc attgtcacac tccattcgga cctcggaggc tgagaactta     780
   actgctgaag acacggacac tcctgccctg ctgtgtatag atggaagata tttatatatt     840
   ttttggttgt caatattaaa tacagacact aagttatagt atatctggac aaacccactt     900
   gtaaatacac caacaaactc ctgtaacttt acctaagcag atataaatgg ctggttttta     960
   gaaaaaaaaa aaaaaaaaaa aaaaaaaaaa aaaaaaaaaa aaaaaaaaaa aaaaaaaaaa    1020
   aaaaaaaaaa aaaaa                                                     1035
<210> 65
   <211> 127
   <212> **DNA**
   <213> Artificial Sequence
<220>
   <223> Description of Artificial Sequence: Synthetic polynucleotide
<220>
   <223> Poly A signal
<400> 65
   gatctttttc cctctgccaa aaattatggg gacatcatga agccccttga gcatctgact      60
   tctggctaat aaaggaaatt tattttcatt gcaatagtgt gttggaattt tttgtgtctc     120
   tcactcg                                                               127
<210> 66
   <211> 130
   <212> DNA
   <213> Artificial Sequence
<220>
   <223> Description of Artificial Sequence: Synthetic polynucleotide
<220>
   <223> 3'ITR
<400> 66
   aggaacccct agtgatggag ttggccactc cctctctgcg cgctcgctcg ctcactgagg 60
   ccgggcgacc aaaggtcgcc cgacgcccgg gctttgcccg ggcggcctca gtgagcgagc 120
   gagcgcgcag 130
<210> 67
   <211> 7138
   <212> DNA
   <213> Artificial Sequence
<220>
   <223> Description of Artificial Sequence: Synthetic polynucleotide
<220>
   <223> pAAV CB6 PI mGlut1-out3xmiR-122 BS
<400> 67
   cttaattagg ctgcgcgctc gctcgctcac tgaggccgcc cgggcaaagc ccgggcgtcg 60
   ggcgaccttt ggtcgcccgg cctcagtgag cgagcgagcg cgcagagagg gagtggccaa 120
   ctccatcact aggggttcct tgtagttaat gattaacccg ccatgctact tatctaccag 180
   ggtaatgggg atcctctaga actatagcta gtcgacattg attattgact agttattaat 240
   agtaatcaat tacggggtca ttagttcata gcccatatat ggagttccgc gttacataac 300
   ttacggtaaa tggcccgcct ggctgaccgc ccaacgaccc ccgcccattg acgtcaataa 360
   tgacgtatgt tcccatagta acgccaatag ggactttcca ttgacgtcaa tgggtggagt 420
   atttacggta aactgcccac ttggcagtac atcaagtgta tcatatgcca agtacgcccc 480
   ctattgacgt caatgacggt aaatggcccg cctggcatta tgcccagtac atgaccttat 540
   gggactttcc tacttggcag tacatctacg tattagtcat cgctattacc atgtcgaggc 600
   cacgttctgc ttcactctcc ccatctcccc cccctcccca cccccaattt tgtatttatt 660
   tattttttaa ttattttgtg cagcgatggg ggcggggggg gggggcgcgc gccaggcggg 720
   gcggggcggg gcgaggggcg gggcggggcg aggcggagag gtgcggcggc agccaatcag 780
   agcggcgcgc tccgaaagtt tccttttatg gcgaggcggc ggcggcggcg gccctataaa 840
   aagcgaagcg cgcggcgggc gggagcaagc tttattgcgg tagtttatca cagttaaatt 900
   gctaacgcag tcagtgcttc tgacacaaca gtctcgaact taagctgcag aagttggtcg 960
   tgaggcactg ggcaggtaag tatcaaggtt acaagacagg tttaaggaga ccaatagaaa 1020
   ctgggcttgt cgagacagag aagactcttg cgtttctgat aggcacctat tggtcttact 1080
   gacatccact ttgcctttct ctccacaggt gtccactccc agttcaatta cagctcttaa 1140
   ggctagagta cttaatacga ctcactatag gctagcgcgc cgaattcggc acgaggaaaa 1200
   aggcagctcc gcgcgctctc ccccaagagc agaggcttgc ttgtagagtg acgatctgag 1260
   ctacggggtc ttaagtgcgt cagggcgtgg aggtctggcg ggagacgcat agttacagcg 1320
   cgtccgttct ccgtctcgca gccggcacag ctagagcttc gagcgcagcg cggccatgga 1380
   tcccagcagc aagaaggtga cgggccgcct catgttggct gtgggaggag cagtgctcgg 1440
   atcactgcag ttcggctata acactggtgt catcaacgcc ccccagaagg ttattgagga 1500
   gttctacaat caaacatgga accaccgcta cggagagccc atcccatcca ccacactcac 1560
   cacgctttgg tctctctccg tggccatctt ctctgtcggg ggcatgattg gttccttctc 1620
   tgtcggcctc tttgttaatc gctttggcag gcggaactcc atgctgatga tgaacctgtt 1680
   ggcctttgtg gctgctgtgc ttatgggctt ctccaaactg ggcaagtcct ttgagatgct 1740
   gatcctgggc cgcttcatca tcggtgtgta ctgcggcctg actactggct ttgtgcccat 1800
   gtatgtggga gaggtgtcac ctacagctct acgtggagcc ctaggcacac tgcaccagct 1860
   gggaatcgtc gttggcatcc ttattgccca ggtgtttggc ttagactcca tcatgggcaa 1920
   tgcagacttg tggcctctgc tgctcagtgt catcttcatc ccagccctgc tacagtgtat 1980
   cctgttgccc ttctgccccg agagcccccg cttcctgctc atcaatcgta acgaggagaa 2040
   ccgggccaag agtgtgctga agaagcttcg agggacagcc gatgtgaccc gagacctgca 2100
   ggagatgaaa gaagagggtc ggcagatgat gcgggagaag aaggtcacca tcttggagct 2160
   gttccgctca cccgcctacc gccagcccat cctcatcgct gtggtgctgc agctgtccca 2220
   gcagctgtcg ggtatcaatg ctgtgttcta ctactcaacg agcatcttcg agaaggcagg 2280
   tgtgcagcag cctgtgtacg ccaccatcgg ctccggtatc gtcaacacgg ccttcactgt 2340
   ggtgtcgctg tttgttgtag agcgagctgg acgacggacc ctgcacctca ttggcctggc 2400
   tggcatggca ggctgtgctg tgctcatgac catcgccctg gccttgctgg aacggctgcc 2460
   ttggatgtcc tatctgagca tcgtggccat ctttggcttt gtggccttct ttgaagtagg 2520
   ccctggtcct attccatggt tcattgtggc cgagctgttc agccaggggc cccgtcctgc 2580
   tgctattgct gtggctggct tctccaactg gacctcaaac ttcattgtgg gcatgtgctt 2640
   ccagtatgtg gagcaactgt gcggccccta cgtcttcatc atcttcacgg tgctcctcgt 2700
   gctcttcttc atcttcacct acttcaaagt ccctgagacc aaaggccgaa ccttcgatga 2760
   gatcgcttcc ggcttccggc aggggggtgc cagccaaagt gacaagacac ccgaggagct 2820
   cttccaccct ctgggggcgg actcccaagt gtgaggagcc ccacacccag cccggcctgc 2880
   tccctgcagc ccaaggatct ctctggagca caggcagcta gatgagacct cttccgaacc 2940
   gacagatctc gggcaagccg ggcctgggcg cctttcctca gccagcagtg aagtccagga 3000
   ggatattcag gactttgatg gctccagaat ttttaatgaa agcaagactg ctgctcagat 3060
   ctattcagat aagcagcagg ttttataatt tttttattac tgattttgtt attttttttt 3120
   tttatcagcc actctcctat ctccacactg tagtcttcac cttgattggc ccagtgcctg 3180
   agggtgggga ccacgccctg tccagacact tgccttcttt gccaagctaa tctgtagggc 3240
   tggacctatg gccaaggaca cactaatacc gaactctgag ctaggaggct ttaccgctgg 3300
   aggcggtagc tgccacccac ttccgcaggc ctggacctcg gcaccatagg ggtccggact 3360
   ccattttagg attcgcccat tcctgtctct tcctacccaa ccactcaatt aatctttcct 3420
   tgcctgagac cagttggaag cactggagtg cagggaggag agggaagggc caggctgggc 3480
   tgccaggttc tagtctcctg tgcactgagg gccacacaaa caccatgaga aggacctcgg 3540
   aggctgagaa cttaactgct gaagacacgg acactcctgc cctgctgtgt atagatggaa 3600
   gatatttata tattttttgg ttgtcaatat taaatacaga cactaagtta tagtatatct 3660
   ggacaaaccc acttgtaaat acaccaacaa actcctgtaa ctttacctaa gcagatataa 3720
   atggctggtt tttagaaaaa aaaaaaaaaa aaaaaaaaaa aaaaaaaaaa aaaaaaaaaa 3780
   aaaaaaaaaa aaaaaaaaaa aaaaaaaaaa actgtctcgc tgcggcccga aacaaacacc 3840
   attgtcacac tccaacaaac accattgtca cactccaaca aacaccattg tcacactcca 3900
   ttcgggccgc tctagagtat ccctcgactc tagagtcgac ccgggcggcc tcgaggacgg 3960
   ggtgaactac gcctgaggat ccgatctttt tccctctgcc aaaaattatg gggacatcat 4020
   gaagcccctt gagcatctga cttctggcta ataaaggaaa tttattttca ttgcaatagt 4080
   gtgttggaat tttttgtgtc tctcactcgg aagcaattcg ttgatctgaa tttcgaccac 4140
   ccataatacc cattaccctg gtagataagt agcatggcgg gttaatcatt aactacaagg 4200
   aacccctagt gatggagttg gccactccct ctctgcgcgc tcgctcgctc actgaggccg 4260
   ggcgaccaaa ggtcgcccga cgcccgggct ttgcccgggc ggcctcagtg agcgagcgag 4320
   cgcgcagcct taattaacct aattcactgg ccgtcgtttt acaacgtcgt gactgggaaa 4380
   accctggcgt tacccaactt aatcgccttg cagcacatcc ccctttcgcc agctggcgta 4440
   atagcgaaga ggcccgcacc gatcgccctt cccaacagtt gcgcagcctg aatggcgaat 4500
   gggacgcgcc ctgtagcggc gcattaagcg cggcgggtgt ggtggttacg cgcagcgtga 4560
   ccgctacact tgccagcgcc ctagcgcccg ctcctttcgc tttcttccct tcctttctcg 4620
   ccacgttcgc cggctttccc cgtcaagctc taaatcgggg gctcccttta gggttccgat 4680
   ttagtgcttt acggcacctc gaccccaaaa aacttgatta gggtgatggt tcacgtagtg 4740
   ggccatcgcc ctgatagacg gtttttcgcc ctttgacgtt ggagtccacg ttctttaata 4800
   gtggactctt gttccaaact ggaacaacac tcaaccctat ctcggtctat tcttttgatt 4860
   tataagggat tttgccgatt tcggcctatt ggttaaaaaa tgagctgatt taacaaaaat 4920
   ttaacgcgaa ttttaacaaa atattaacgc ttacaattta ggtggcactt ttcggggaaa 4980
   tgtgcgcgga acccctattt gtttattttt ctaaatacat tcaaatatgt atccgctcat 5040
   gagacaataa ccctgataaa tgcttcaata atattgaaaa aggaagagta tgagtattca 5100
   acatttccgt gtcgccctta ttcccttttt tgcggcattt tgccttcctg tttttgctca 5160
   cccagaaacg ctggtgaaag taaaagatgc tgaagatcag ttgggtgcac gagtgggtta 5220
   catcgaactg gatctcaaca gcggtaagat ccttgagagt tttcgccccg aagaacgttt 5280
   tccaatgatg agcactttta aagttctgct atgtggcgcg gtattatccc gtattgacgc 5340
   cgggcaagag caactcggtc gccgcataca ctattctcag aatgacttgg ttgagtactc 5400
   accagtcaca gaaaagcatc ttacggatgg catgacagta agagaattat gcagtgctgc 5460
   cataaccatg agtgataaca ctgcggccaa cttacttctg acaacgatcg gaggaccgaa 5520
   ggagctaacc gcttttttgc acaacatggg ggatcatgta actcgccttg atcgttggga 5580
   accggagctg aatgaagcca taccaaacga cgagcgtgac accacgatgc ctgtagcaat 5640
   ggcaacaacg ttgcgcaaac tattaactgg cgaactactt actctagctt cccggcaaca 5700
   attaatagac tggatggagg cggataaagt tgcaggacca cttctgcgct cggcccttcc 5760
   ggctggctgg tttattgctg ataaatctgg agccggtgag cgtgggtctc gcggtatcat 5820
   tgcagcactg gggccagatg gtaagccctc ccgtatcgta gttatctaca cgacggggag 5880
   tcaggcaact atggatgaac gaaatagaca gatcgctgag ataggtgcct cactgattaa 5940
   gcattggtaa ctgtcagacc aagtttactc atatatactt tagattgatt taaaacttca 6000
   tttttaattt aaaaggatct aggtgaagat cctttttgat aatctcatga ccaaaatccc 6060
   ttaacgtgag ttttcgttcc actgagcgtc agaccccgta gaaaagatca aaggatcttc 6120
   ttgagatcct ttttttctgc gcgtaatctg ctgcttgcaa acaaaaaaac caccgctacc 6180
   agcggtggtt tgtttgccgg atcaagagct accaactctt tttccgaagg taactggctt 6240
   cagcagagcg cagataccaa atactgttct tctagtgtag ccgtagttag gccaccactt 6300
   caagaactct gtagcaccgc ctacatacct cgctctgcta atcctgttac cagtggctgc 6360
   tgccagtggc gataagtcgt gtcttaccgg gttggactca agacgatagt taccggataa 6420
   ggcgcagcgg tcgggctgaa cggggggttc gtgcacacag cccagcttgg agcgaacgac 6480
   ctacaccgaa ctgagatacc tacagcgtga gctatgagaa agcgccacgc ttcccgaagg 6540
   gagaaaggcg gacaggtatc cggtaagcgg cagggtcgga acaggagagc gcacgaggga 6600
   gcttccaggg ggaaacgcct ggtatcttta tagtcctgtc gggtttcgcc acctctgact 6660
   tgagcgtcga tttttgtgat gctcgtcagg ggggcggagc ctatggaaaa acgccagcaa 6720
   cgcggccttt ttacggttcc tggccttttg ctggcctttt gctcacatgt tctttcctgc 6780
   gttatcccct gattctgtgg ataaccgtat taccgccttt gagtgagctg ataccgctcg 6840
   ccgcagccga acgaccgagc gcagcgagtc agtgagcgag gaagcggaag agcgcccaat 6900
   acgcaaaccg cctctccccg cgcgttggcc gattcattaa tgcagctggc acgacaggtt 6960
   tcccgactgg aaagcgggca gtgagcgcaa cgcaattaat gtgagttagc tcactcatta 7020
   ggcaccccag gctttacact ttatgcttcc ggctcgtatg ttgtgtggaa ttgtgagcgg 7080
   ataacaattt cacacaggaa acagctatga ccatgattac gccagattta attaaggc 7138
<210> 68
   <211> 130
   <212> DNA
   <213> Artificial Sequence
<220>
   <223> Description of Artificial Sequence: Synthetic polynucleotide
<220>
   <223> 5'ITR
<400> 68
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 69
   <211> 382
   <212> DNA
   <213> Artificial Sequence
<220>
   <223> Description of Artificial Sequence: Synthetic polynucleotide
<220>
   <223> CMV IE enhancer
<400> 69
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 70
   <211> 382
   <212> DNA
   <213> Artificial Sequence
<220>
   <223> Description of Artificial Sequence: Synthetic polynucleotide
<220>
   <223> CB promoter
<400> 70
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 71
   <211> 1479
   <212> DNA
   <213> Artificial Sequence
<220>
   <223> Description of Artificial Sequence: Synthetic polynucleotide
<220>
   <223> mGlut1 cDNA
<400> 71
   atggatccca gcagcaagaa ggtgacgggc cgcctcatgt tggctgtggg aggagcagtg 60
   ctcggatcac tgcagttcgg ctataacact ggtgtcatca acgcccccca gaaggttatt 120
   gaggagttct acaatcaaac atggaaccac cgctacggag agcccatccc atccaccaca 180
   ctcaccacgc tttggtctct ctccgtggcc atcttctctg tcgggggcat gattggttcc 240
   ttctctgtcg gcctctttgt taatcgcttt ggcaggcgga actccatgct gatgatgaac 300
   ctgttggcct ttgtggctgc tgtgcttatg ggcttctcca aactgggcaa gtcctttgag 360
   atgctgatcc tgggccgctt catcatcggt gtgtactgcg gcctgactac tggctttgtg 420
   cccatgtatg tgggagaggt gtcacctaca gctctacgtg gagccctagg cacactgcac 480
   cagctgggaa tcgtcgttgg catccttatt gcccaggtgt ttggcttaga ctccatcatg 540
   ggcaatgcag acttgtggcc tctgctgctc agtgtcatct tcatcccagc cctgctacag 600
   tgtatcctgt tgcccttctg ccccgagagc ccccgcttcc tgctcatcaa tcgtaacgag 660
   gagaaccggg ccaagagtgt gctgaagaag cttcgaggga cagccgatgt gacccgagac 720
   ctgcaggaga tgaaagaaga gggtcggcag atgatgcggg agaagaaggt caccatcttg 780
   gagctgttcc gctcacccgc ctaccgccag cccatcctca tcgctgtggt gctgcagctg 840
   tcccagcagc tgtcgggtat caatgctgtg ttctactact caacgagcat cttcgagaag 900
   gcaggtgtgc agcagcctgt gtacgccacc atcggctccg gtatcgtcaa cacggccttc 960
   actgtggtgt cgctgtttgt tgtagagcga gctggacgac ggaccctgca cctcattggc 1020
   ctggctggca tggcaggctg tgctgtgctc atgaccatcg ccctggcctt gctggaacgg 1080
   ctgccttgga tgtcctatct gagcatcgtg gccatctttg gctttgtggc cttctttgaa 1140
   gtaggccctg gtcctattcc atggttcatt gtggccgagc tgttcagcca ggggccccgt 1200
   cctgctgcta ttgctgtggc tggcttctcc aactggacct caaacttcat tgtgggcatg 1260
   tgcttccagt atgtggagca actgtgcggc ccctacgtct tcatcatctt cacggtgctc 1320
   ctcgtgctct tcttcatctt cacctacttc aaagtccctg agaccaaagg ccgaaccttc 1380
   gatgagatcg cttccggctt ccggcagggg ggtgccagcc aaagtgacaa gacacccgag 1440
   gagctcttcc accctctggg ggcggactcc caagtgtga 1479
<210> 72
   <211> 955
   <212> DNA
   <213> Artificial Sequence
<220>
   <223> Description of Artificial Sequence: Synthetic polynucleotide
<220>
   <223> 3'UTR
<400> 72
   ggagccccac acccagcccg gcctgctccc tgcagcccaa ggatctctct ggagcacagg 60
   cagctagatg agacctcttc cgaaccgaca gatctcgggc aagccgggcc tgggcgcctt 120
   tcctcagcca gcagtgaagt ccaggaggat attcaggact ttgatggctc cagaattttt 180
   aatgaaagca agactgctgc tcagatctat tcagataagc agcaggtttt ataatttttt 240
   tattactgat tttgttattt ttttttttta tcagccactc tcctatctcc acactgtagt 300
   cttcaccttg attggcccag tgcctgaggg tggggaccac gccctgtcca gacacttgcc 360
   ttctttgcca agctaatctg tagggctgga cctatggcca aggacacact aataccgaac 420
   tctgagctag gaggctttac cgctggaggc ggtagctgcc acccacttcc gcaggcctgg 480
   acctcggcac cataggggtc cggactccat tttaggattc gcccattcct gtctcttcct 540
   acccaaccac tcaattaatc tttccttgcc tgagaccagt tggaagcact ggagtgcagg 600
   gaggagaggg aagggccagg ctgggctgcc aggttctagt ctcctgtgca ctgagggcca 660
   cacaaacacc atgagaagga cctcggaggc tgagaactta actgctgaag acacggacac 720
   tcctgccctg ctgtgtatag atggaagata tttatatatt ttttggttgt caatattaaa 780
   tacagacact aagttatagt atatctggac aaacccactt gtaaatacac caacaaactc 840
   ctgtaacttt acctaagcag atataaatgg ctggttttta gaaaaaaaaa aaaaaaaaaa 900
   aaaaaaaaaa aaaaaaaaaa aaaaaaaaaa aaaaaaaaaa aaaaaaaaaa aaaaa 955
<210> 73
   <211> 77
   <212> DNA
   <213> Artificial Sequence
<220>
   <223> Description of Artificial Sequence: Synthetic oligonucleotide
<220>
   <223> 3xmiR-122BS
<400> 73
   cgaaacaaac accattgtca cactccaaca aacaccattg tcacactcca acaaacacca 60
   ttgtcacact ccattcg 77
<210> 74
   <211> 127
   <212> DNA
   <213> Artificial Sequence
<220>
   <223> Description of Artificial Sequence: Synthetic polynucleotide
<220>
   <223> Poly A signal
<400> 74
   gatctttttc cctctgccaa aaattatggg gacatcatga agccccttga gcatctgact 60
   tctggctaat aaaggaaatt tattttcatt gcaatagtgt gttggaattt tttgtgtctc 120
   tcactcg 127
<210> 75
   <211> 130
   <212> DNA
   <213> Artificial Sequence
<220>
   <223> Description of Artificial Sequence: Synthetic polynucleotide
<220>
   <223> 3'ITR
<400> 75
   aggaacccct agtgatggag ttggccactc cctctctgcg cgctcgctcg ctcactgagg 60
   ccgggcgacc aaaggtcgcc cgacgcccgg gctttgcccg ggcggcctca gtgagcgagc 120
   gagcgcgcag 130
<210> 76
   <211> 21
   <212> DNA
   <213> Artificial Sequence
<220>
   <223> Description of Artificial Sequence: Synthetic primer
<220>
   <223> Glut1QPCR F1
<400> 76
   cttgcttgta gagtgacgat c 21
<210> 77
   <211> 20
   <212> DNA
   <213> Artificial Sequence
<220>
   <223> Description of Artificial Sequence: Synthetic primer
<220>
   <223> Glut1QPCR R1
<400> 77
   cagtgatccg agcactgctc 20
<210> 78
   <211> 492
   <212> PRT
   <213> Mus musculus
<220>
   <223> mGlut1 amino acid sequence; solute carrier family 2, facilitated glucose transporter member 1
<400> 78
   Met Asp Pro Ser Ser Lys Lys Val Thr Gly Arg Leu Met Leu Ala Val
   1 5 10 15
   Gly Gly Ala Val Leu Gly Ser Leu Gln Phe Gly Tyr Asn Thr Gly Val
               20                  25                  30
   Ile Asn Ala Pro Gln Lys Val Ile Glu Glu Phe Tyr Asn Gln Thr Trp
           35                  40                  45
   Asn His Arg Tyr Gly Glu Pro Ile Pro Ser Thr Thr Leu Thr Thr Leu
       50                  55                  60
   Trp Ser Leu Ser Val Ala Ile Phe Ser Val Gly Gly Met Ile Gly Ser
   65                  70                  75                  80
   Phe Ser Val Gly Leu Phe Val Asn Arg Phe Gly Arg Arg Asn Ser Met
                   85                  90                  95
   Leu Met Met Asn Leu Leu Ala Phe Val Ala Ala Val Leu Met Gly Phe
               100                 105                 110
   Ser Lys Leu Gly Lys Ser Phe Glu Met Leu Ile Leu Gly Arg Phe Ile
           115                 120                 125
   Ile Gly Val Tyr Cys Gly Leu Thr Thr Gly Phe Val Pro Met Tyr Val
       130                 135                 140
   Gly Glu Val Ser Pro Thr Ala Leu Arg Gly Ala Leu Gly Thr Leu His
   145                 150                 155                 160
   Gln Leu Gly Ile Val Val Gly Ile Leu Ile Ala Gln Val Phe Gly Leu
                   165                 170                 175
   Asp Ser Ile Met Gly Asn Ala Asp Leu Trp Pro Leu Leu Leu Ser Val
               180                 185                 190
   Ile Phe Ile Pro Ala Leu Leu Gln Cys Ile Leu Leu Pro Phe Cys Pro
           195                 200                 205
   Glu Ser Pro Arg Phe Leu Leu Ile Asn Arg Asn Glu Glu Asn Arg Ala
       210                 215                 220
   Lys Ser Val Leu Lys Lys Leu Arg Gly Thr Ala Asp Val Thr Arg Asp
   225                 230                 235                 240
   Leu Gln Glu Met Lys Glu Glu Gly Arg Gln Met Met Arg Glu Lys Lys
                   245                 250                 255
   Val Thr Ile Leu Glu Leu Phe Arg Ser Pro Ala Tyr Arg Gln Pro Ile
               260                 265                 270
   Leu Ile Ala Val Val Leu Gln Leu Ser Gln Gln Leu Ser Gly Ile Asn
   275 280 285
   Ala Val Phe Tyr Tyr Ser Thr Ser Ile Phe Glu Lys Ala Gly Val Gln
   290 295 300
   Gln Pro Val Tyr Ala Thr Ile Gly Ser Gly Ile Val Asn Thr Ala Phe
   305 310 315 320
   Thr Val Val Ser Leu Phe Val Val Glu Arg Ala Gly Arg Arg Thr Leu
   325 330 335
   His Leu Ile Gly Leu Ala Gly Met Ala Gly Cys Ala Val Leu Met Thr
   340 345 350
   Ile Ala Leu Ala Leu Leu Glu Arg Leu Pro Trp Met Ser Tyr Leu Ser
   355 360 365
   Ile Val Ala Ile Phe Gly Phe Val Ala Phe Phe Glu Val Gly Pro Gly
   370 375 380
   Pro Ile Pro Trp Phe Ile Val Ala Glu Leu Phe Ser Gln Gly Pro Arg
   385 390 395 400
   Pro Ala Ala Ile Ala Val Ala Gly Phe Ser Asn Trp Thr Ser Asn Phe
   405 410 415
   Ile Val Gly Met Cys Phe Gln Tyr Val Glu Gln Leu Cys Gly Pro Tyr
   420 425 430
   Val Phe Ile Ile Phe Thr Val Leu Leu Val Leu Phe Phe Ile Phe Thr
   435 440 445
   Tyr Phe Lys Val Pro Glu Thr Lys Gly Arg Thr Phe Asp Glu Ile Ala
   450 455 460
   Ser Gly Phe Arg Gln Gly Gly Ala Ser Gln Ser Asp Lys Thr Pro Glu
   465 470 475 480
   Glu Leu Phe His Pro Leu Gly Ala Asp Ser Gln Val
   485 490
<210> 79
   <211> 492
   <212> PRT
   <213> Homo sapiens
<220>
<210> 80
   <211> 6834
   <212> DNA
   <213> Artificial Sequence
<220>
   <223> Description of Artificial Sequence: Synthetic polynucleotide
<220>
   <223> pAAV CB6 PI hGlut1- EGFP
<400> 80
   cttaattagg ctgcgcgctc gctcgctcac tgaggccgcc cgggcaaagc ccgggcgtcg        60
   ggcgaccttt ggtcgcccgg cctcagtgag cgagcgagcg cgcagagagg gagtggccaa       120
   ctccatcact aggggttcct tgtagttaat gattaacccg ccatgctact tatctaccag       180
   ggtaatgggg atcctctaga actatagcta gtcgacattg attattgact agttattaat       240
   agtaatcaat tacggggtca ttagttcata gcccatatat ggagttccgc gttacataac       300
   ttacggtaaa tggcccgcct ggctgaccgc ccaacgaccc ccgcccattg acgtcaataa       360
   tgacgtatgt tcccatagta acgccaatag ggactttcca ttgacgtcaa tgggtggagt       420
   atttacggta aactgcccac ttggcagtac atcaagtgta tcatatgcca agtacgcccc       480
   ctattgacgt caatgacggt aaatggcccg cctggcatta tgcccagtac atgaccttat       540
   gggactttcc tacttggcag tacatctacg tattagtcat cgctattacc atgtcgaggc       600
   cacgttctgc ttcactctcc ccatctcccc cccctcccca cccccaattt tgtatttatt       660
   tattttttaa ttattttgtg cagcgatggg ggcggggggg gggggcgcgc gccaggcggg       720
   gcggggcggg gcgaggggcg gggcggggcg aggcggagag gtgcggcggc agccaatcag       780
   agcggcgcgc tccgaaagtt tccttttatg gcgaggcggc ggcggcggcg gccctataaa       840
   aagcgaagcg cgcggcgggc gggagcaagc tttattgcgg tagtttatca cagttaaatt       900
   gctaacgcag tcagtgcttc tgacacaaca gtctcgaact taagctgcag aagttggtcg       960
   tgaggcactg ggcaggtaag tatcaaggtt acaagacagg tttaaggaga ccaatagaaa      1020
   ctgggcttgt cgagacagag aagactcttg cgtttctgat aggcacctat tggtcttact      1080
   gacatccact ttgcctttct ctccacaggt gtccactccc agttcaatta cagctcttaa      1140
   ggctagagta cttaatacga ctcactatag gctagcgcgc cgaattcggc acgaggaaaa      1200
   aggcagctcc gcgcgctctc ccccaagagc agaggcttgc ttgtagagtg acgatctgag      1260
   ctacggggtc ttaagtgcgt cagggcgtgg aggtctggcg ggagacgcat agttacagcg      1320
   cgtccgttct ccgtctcgca gccggcacag ctagagcttc gagcgcagcg cggccatgga      1380
   tcccagcagc aagaaggtga cgggccgcct catgttggct gtgggaggag cagtgctcgg      1440
   atcactgcag ttcggctata acactggtgt catcaacgcc ccccagaagg ttattgagga 1500
   gttctacaat caaacatgga accaccgcta cggagagccc atcccatcca ccacactcac 1560
   cacgctttgg tctctctccg tggccatctt ctctgtcggg ggcatgattg gttccttctc 1620
   tgtcggcctc tttgttaatc gctttggcag gcggaactcc atgctgatga tgaacctgtt 1680
   ggcctttgtg gctgctgtgc ttatgggctt ctccaaactg ggcaagtcct ttgagatgct 1740
   gatcctgggc cgcttcatca tcggtgtgta ctgcggcctg actactggct ttgtgcccat 1800
   gtatgtggga gaggtgtcac ctacagctct acgtggagcc ctaggcacac tgcaccagct 1860
   gggaatcgtc gttggcatcc ttattgccca ggtgtttggc ttagactcca tcatgggcaa 1920
   tgcagacttg tggcctctgc tgctcagtgt catcttcatc ccagccctgc tacagtgtat 1980
   cctgttgccc ttctgccccg agagcccccg cttcctgctc atcaatcgta acgaggagaa 2040
   ccgggccaag agtgtgctga agaagcttcg agggacagcc gatgtgaccc gagacctgca 2100
   ggagatgaaa gaagagggtc ggcagatgat gcgggagaag aaggtcacca tcttggagct 2160
   gttccgctca cccgcctacc gccagcccat cctcatcgct gtggtgctgc agctgtccca 2220
   gcagctgtcg ggtatcaatg ctgtgttcta ctactcaacg agcatcttcg agaaggcagg 2280
   tgtgcagcag cctgtgtacg ccaccatcgg ctccggtatc gtcaacacgg ccttcactgt 2340
   ggtgtcgctg tttgttgtag agcgagctgg acgacggacc ctgcacctca ttggcctggc 2400
   tggcatggca ggctgtgctg tgctcatgac catcgccctg gccttgctgg aacggctgcc 2460
   ttggatgtcc tatctgagca tcgtggccat ctttggcttt gtggccttct ttgaagtagg 2520
   ccctggtcct attccatggt tcattgtggc cgagctgttc agccaggggc cccgtcctgc 2580
   tgctattgct gtggctggct tctccaactg gacctcaaac ttcattgtgg gcatgtgctt 2640
   ccagtatgtg gagcaactgt gcggccccta cgtcttcatc atcttcacgg tgctcctcgt 2700
   gctcttcttc atcttcacct acttcaaagt ccctgagacc aaaggccgaa ccttcgatga 2760
   gatcgcttcc ggcttccggc aggggggtgc cagccaaagt gacaagacac ccgaggagct 2820
   cttccaccct ctgggggcgg actcccaagt gaccggtgcc atggtgagca agggcgagga 2880
   gctgttcacc ggggtggtgc ccatcctggt cgagctggac ggcgacgtaa acggccacaa 2940
   gttcagcgtg tccggcgagg gcgagggcga tgccacctac ggcaagctga ccctgaagtt 3000
   catctgcacc accggcaagc tgcccgtgcc ctggcccacc ctcgtgacca ccctgaccta 3060
   cggcgtgcag tgcttcagcc gctaccccga ccacatgaag cagcacgact tcttcaagtc 3120
   cgccatgccc gaaggctacg tccaggagcg caccatcttc ttcaaggacg acggcaacta 3180
   caagacccgc gccgaggtga agttcgaggg cgacaccctg gtgaaccgca tcgagctgaa 3240
   gggcatcgac ttcaaggagg acggcaacat cctggggcac aagctggagt acaactacaa 3300
   cagccacaac gtctatatca tggccgacaa gcagaagaac ggcatcaagg tgaacttcaa 3360
   gatccgccac aacatcgagg acggcagcgt gcagctcgcc gaccactacc agcagaacac 3420
   ccccatcggc gacggccccg tgctgctgcc cgacaaccac tacctgagca cccagtccgc 3480
   cctgagcaaa gaccccaacg agaagcgcga tcacatggtc ctgctggagt tcgtgaccgc 3540
   cgccgggatc actctcggca tggacgagct gtacaagtaa agcggccatc aagcttatcg 3600
   ggccgctcta gagtatccct cgactctaga gtcgacccgg gcggcctcga ggacggggtg 3660
   aactacgcct gaggatccga tctttttccc tctgccaaaa attatgggga catcatgaag 3720
   ccccttgagc atctgacttc tggctaataa aggaaattta ttttcattgc aatagtgtgt 3780
   tggaattttt tgtgtctctc actcggaagc aattcgttga tctgaatttc gaccacccat 3840
   aatacccatt accctggtag ataagtagca tggcgggtta atcattaact acaaggaacc 3900
   cctagtgatg gagttggcca ctccctctct gcgcgctcgc tcgctcactg aggccgggcg 3960
   accaaaggtc gcccgacgcc cgggctttgc ccgggcggcc tcagtgagcg agcgagcgcg 4020
   cagccttaat taacctaatt cactggccgt cgttttacaa cgtcgtgact gggaaaaccc 4080
   tggcgttacc caacttaatc gccttgcagc acatccccct ttcgccagct ggcgtaatag 4140
   cgaagaggcc cgcaccgatc gcccttccca acagttgcgc agcctgaatg gcgaatggga 4200
   cgcgccctgt agcggcgcat taagcgcggc gggtgtggtg gttacgcgca gcgtgaccgc 4260
   tacacttgcc agcgccctag cgcccgctcc tttcgctttc ttcccttcct ttctcgccac 4320
   gttcgccggc tttccccgtc aagctctaaa tcgggggctc cctttagggt tccgatttag 4380
   tgctttacgg cacctcgacc ccaaaaaact tgattagggt gatggttcac gtagtgggcc 4440
   atcgccctga tagacggttt ttcgcccttt gacgttggag tccacgttct ttaatagtgg 4500
   actcttgttc caaactggaa caacactcaa ccctatctcg gtctattctt ttgatttata 4560
   agggattttg ccgatttcgg cctattggtt aaaaaatgag ctgatttaac aaaaatttaa 4620
   cgcgaatttt aacaaaatat taacgcttac aatttaggtg gcacttttcg gggaaatgtg 4680
   cgcggaaccc ctatttgttt atttttctaa atacattcaa atatgtatcc gctcatgaga 4740
   caataaccct gataaatgct tcaataatat tgaaaaagga agagtatgag tattcaacat 4800
   ttccgtgtcg cccttattcc cttttttgcg gcattttgcc ttcctgtttt tgctcaccca 4860
   gaaacgctgg tgaaagtaaa agatgctgaa gatcagttgg gtgcacgagt gggttacatc 4920
   gaactggatc tcaacagcgg taagatcctt gagagttttc gccccgaaga acgttttcca 4980
   atgatgagca cttttaaagt tctgctatgt ggcgcggtat tatcccgtat tgacgccggg 5040
   caagagcaac tcggtcgccg catacactat tctcagaatg acttggttga gtactcacca 5100
   gtcacagaaa agcatcttac ggatggcatg acagtaagag aattatgcag tgctgccata 5160
   accatgagtg ataacactgc ggccaactta cttctgacaa cgatcggagg accgaaggag 5220
   ctaaccgctt ttttgcacaa catgggggat catgtaactc gccttgatcg ttgggaaccg 5280
   gagctgaatg aagccatacc aaacgacgag cgtgacacca cgatgcctgt agcaatggca 5340
   acaacgttgc gcaaactatt aactggcgaa ctacttactc tagcttcccg gcaacaatta 5400
   atagactgga tggaggcgga taaagttgca ggaccacttc tgcgctcggc ccttccggct 5460
   ggctggttta ttgctgataa atctggagcc ggtgagcgtg ggtctcgcgg tatcattgca 5520
   gcactggggc cagatggtaa gccctcccgt atcgtagtta tctacacgac ggggagtcag 5580
   gcaactatgg atgaacgaaa tagacagatc gctgagatag gtgcctcact gattaagcat 5640
   tggtaactgt cagaccaagt ttactcatat atactttaga ttgatttaaa acttcatttt 5700
   taatttaaaa ggatctaggt gaagatcctt tttgataatc tcatgaccaa aatcccttaa 5760
   cgtgagtttt cgttccactg agcgtcagac cccgtagaaa agatcaaagg atcttcttga 5820
   gatccttttt ttctgcgcgt aatctgctgc ttgcaaacaa aaaaaccacc gctaccagcg 5880
   gtggtttgtt tgccggatca agagctacca actctttttc cgaaggtaac tggcttcagc 5940
   agagcgcaga taccaaatac tgttcttcta gtgtagccgt agttaggcca ccacttcaag 6000
   aactctgtag caccgcctac atacctcgct ctgctaatcc tgttaccagt ggctgctgcc 6060
   agtggcgata agtcgtgtct taccgggttg gactcaagac gatagttacc ggataaggcg 6120
   cagcggtcgg gctgaacggg gggttcgtgc acacagccca gcttggagcg aacgacctac 6180
   accgaactga gatacctaca gcgtgagcta tgagaaagcg ccacgcttcc cgaagggaga 6240
   aaggcggaca ggtatccggt aagcggcagg gtcggaacag gagagcgcac gagggagctt 6300
   ccagggggaa acgcctggta tctttatagt cctgtcgggt ttcgccacct ctgacttgag 6360
   cgtcgatttt tgtgatgctc gtcagggggg cggagcctat ggaaaaacgc cagcaacgcg 6420
   gcctttttac ggttcctggc cttttgctgg ccttttgctc acatgttctt tcctgcgtta 6480
   tcccctgatt ctgtggataa ccgtattacc gcctttgagt gagctgatac cgctcgccgc 6540
   agccgaacga ccgagcgcag cgagtcagtg agcgaggaag cggaagagcg cccaatacgc 6600
   aaaccgcctc tccccgcgcg ttggccgatt cattaatgca gctggcacga caggtttccc 6660
   gactggaaag cgggcagtga gcgcaacgca attaatgtga gttagctcac tcattaggca 6720
   ccccaggctt tacactttat gcttccggct cgtatgttgt gtggaattgt gagcggataa 6780
   caatttcaca caggaaacag ctatgaccat gattacgcca gatttaatta aggc 6834
<210> 81
   <211> 130
   <212> DNA
   <213> Artificial Sequence
<220>
   <223> Description of Artificial Sequence: Synthetic
<220>
   <223> 5'ITR
<400> 81
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 82
   <211> 382
   <212> DNA
   <213> Artificial Sequence
<220>
   <223> Description of Artificial Sequence: Synthetic polynucleotide
<220>
   <223> CMV IE enhancer
<400> 82
   ctagtcgaca ttgattattg actagttatt aatagtaatc aattacgggg tcattagttc 60
   atagcccata tatggagttc cgcgttacat aacttacggt aaatggcccg cctggctgac 120
   cgcccaacga cccccgccca ttgacgtcaa taatgacgta tgttcccata gtaacgccaa 180
   tagggacttt ccattgacgt caatgggtgg agtatttacg gtaaactgcc cacttggcag 240
   tacatcaagt gtatcatatg ccaagtacgc cccctattga cgtcaatgac ggtaaatggc 300
   ccgcctggca ttatgcccag tacatgacct tatgggactt tcctacttgg cagtacatct 360
   acgtattagt catcgctatt ac 382
<210> 83
   <211> 382
   <212> DNA
   <213> Artificial Sequence
<220>
   <223> Description of Artificial Sequence: Synthetic polynucleotide
<220>
   <223> CB promoter
<400> 83
   ctagtcgaca ttgattattg actagttatt aatagtaatc aattacgggg tcattagttc        60
   atagcccata tatggagttc cgcgttacat aacttacggt aaatggcccg cctggctgac       120
   cgcccaacga cccccgccca ttgacgtcaa taatgacgta tgttcccata gtaacgccaa       180
   tagggacttt ccattgacgt caatgggtgg agtatttacg gtaaactgcc cacttggcag       240
   tacatcaagt gtatcatatg ccaagtacgc cccctattga cgtcaatgac ggtaaatggc       300
   ccgcctggca ttatgcccag tacatgacct tatgggactt tcctacttgg cagtacatct       360
   acgtattagt catcgctatt ac                                                382
<210> 84
   <211> 717
   <212> DNA
   <213> Artificial Sequence
<220>
   <223> Description of Artificial Sequence: Synthetic polynucleotide
<220>
   <223> eGFP
<400> 84
   atggtgagca agggcgagga gctgttcacc ggggtggtgc ccatcctggt cgagctggac        60
   ggcgacgtaa acggccacaa gttcagcgtg tccggcgagg gcgagggcga tgccacctac       120
   ggcaagctga ccctgaagtt catctgcacc accggcaagc tgcccgtgcc ctggcccacc       180
   ctcgtgacca ccctgaccta cggcgtgcag tgcttcagcc gctaccccga ccacatgaag       240
   cagcacgact tcttcaagtc cgccatgccc gaaggctacg tccaggagcg caccatcttc       300
   ttcaaggacg acggcaacta caagacccgc gccgaggtga agttcgaggg cgacaccctg       360
   gtgaaccgca tcgagctgaa gggcatcgac ttcaaggagg acggcaacat cctggggcac       420
   aagctggagt acaactacaa cagccacaac gtctatatca tggccgacaa gcagaagaac       480
   ggcatcaagg tgaacttcaa gatccgccac aacatcgagg acggcagcgt gcagctcgcc       540
   gaccactacc agcagaacac ccccatcggc gacggccccg tgctgctgcc cgacaaccac       600
   tacctgagca cccagtccgc cctgagcaaa gaccccaacg agaagcgcga tcacatggtc       660
   ctgctggagt tcgtgaccgc cgccgggatc actctcggca tggacgagct gtacaag          717
<210> 85
   <211> 1959
   <212> DNA
   <213> Artificial Sequence
<220>
   <223> Description of Artificial Sequence: Synthetic polynucleotide
<220>
   <223> hGlut1 cDNA and 3'UTR
<400> 85
   atggagccca gcagcaagaa gctgacgggt cgcctcatgc tggccgtggg aggagcagtg      60
   cttggctccc tgcagtttgg ctacaacact ggagtcatca atgcccccca gaaggtgatc     120
   gaggagttct acaaccagac atgggtccac cgctatgggg agagcatcct gcccaccacg     180
   ctcaccacgc tctggtccct ctcagtggcc atcttttctg ttgggggcat gattggctcc     240
   ttctctgtgg gccttttcgt taaccgcttt ggccggcgga attcaatgct gatgatgaac 300
   ctgctggcct tcgtgtccgc cgtgctcatg ggcttctcga aactgggcaa gtcctttgag 360
   atgctgatcc tgggccgctt catcatcggt gtgtactgtg gcctgaccac aggcttcgtg 420
   cccatgtatg tgggtgaagt gtcacccaca gcccttcgtg gggccctggg caccctgcac 480
   cagctgggca tcgtcgtcgg catcctcatc gcccaggtgt tcggcctgga ctccatcatg 540
   ggcaacaagg acctgtggcc cctgctgctg agcatcatct tcatcccggc cctgctgcag 600
   tgcatcgtgc tgcccttctg ccccgagagt ccccgcttcc tgctcatcaa ccgcaacgag 660
   gagaaccggg ccaagagtgt gctaaagaag ctgcgcggga cagctgacgt gacccatgac 720
   ctgcaggaga tgaaggaaga gagtcggcag atgatgcggg agaagaaggt caccatcctg 780
   gagctgttcc gctcccccgc ctaccgccag cccatcctca tcgctgtggt gctgcagctg 840
   tcccagcagc tgtctggcat caacgctgtc ttctattact ccacgagcat cttcgagaag 900
   gcgggggtgc agcagcctgt gtatgccacc attggctccg gtatcgtcaa cacggccttc 960
   actgtcgtgt cgctgtttgt ggtggagcga gcaggccggc ggaccctgca cctcataggc 1020
   ctcgctggca tggcgggttg tgccatactc atgaccatcg cgctagcact gctggagcag 1080
   ctaccccgga tgtcctatct gagcatcgtg gccatctttg gctttgtggc cttctttgaa 1140
   gtgggtcctg gccccatccc atggttcatc gtggctgaac tcttcagcca gggtccacgt 1200
   ccagctgcca ttgccgttgc aggcttctcc aactggacct caaatttcat tgtgggcatg 1260
   tgcttccagt atgtggagca actgtgtggt ccctacgtct tcatcatctt cactgtgctc 1320
   ctggttctgt tcttcatctt cacctacttc aaagttcctg agactaaagg ccggaccttc 1380
   gatgagatcg cttccggctt ccggcagggg ggagccagcc aaagtgacaa gacacccgag 1440
   gagctgttcc atcccctggg ggctgattcc caagtgtgag tcgccccaga tcaccagccc 1500
   ggcctgctcc cagcagccct aaggatctct caggagcaca ggcagctgga tgagacttcc 1560
   aaacctgaca gatgtcagcc gagccgggcc tggggctcct ttctccagcc agcaatgatg 1620
   tccagaagaa tattcaggac ttaacggctc caggatttta acaaaagcaa gactgttgct 1680
   caaatctatt cagacaagca acaggtttta taattttttt attactgatt ttgttatttt 1740
   tatatcagcc tgagtctcct gtgcccacat cccaggcttc accctgaatg gttccatgcc 1800
   tgagggtgga gactaagccc tgtcgagaca cttgccttct tcacccagct aatctgtagg 1860
   gctggaccta tgtcctaagg acacactaat cgaactatga actacaaagc ttctatccca 1920
   ggaggtggct atggccaccc gttctgctgg cctggatct 1959
<210> 86
   <211> 127
   <212> DNA
   <213> Artificial Sequence
<220>
   <223> Description of Artificial Sequence: Synthetic polynucleotide
<220>
   <223> Poly A signal
<400> 86
   gatctttttc cctctgccaa aaattatggg gacatcatga agccccttga gcatctgact       60
   tctggctaat aaaggaaatt tattttcatt gcaatagtgt gttggaattt tttgtgtctc      120
   tcactcg                                                                127
<210> 87
   <211> 130
   <212> DNA
   <213> Artificial Sequence
<220>
   <223> Description of Artificial Sequence: Synthetic polynucleotide
<220>
   <223> 3' ITR
<400> 87
   aggaacccct agtgatggag ttggccactc cctctctgcg cgctcgctcg ctcactgagg       60
   ccgggcgacc aaaggtcgcc cgacgcccgg gctttgcccg ggcggcctca gtgagcgagc      120
   gagcgcgcag                                                             130
<210> 88
   <211> 6885
   <212> DNA
   <213> Artificial Sequence
<220>
   <223> Description of Artificial Sequence: Synthetic polynucleotide
<220>
   <223> pAAV CB6 PI mGlut1-2A- EGFP
<400> 88
   cttaattagg ctgcgcgctc gctcgctcac tgaggccgcc cgggcaaagc ccgggcgtcg      60
   ggcgaccttt ggtcgcccgg cctcagtgag cgagcgagcg cgcagagagg gagtggccaa     120
   ctccatcact aggggttcct tgtagttaat gattaacccg ccatgctact tatctaccag     180
   ggtaatgggg atcctctaga actatagcta gtcgacattg attattgact agttattaat     240
   agtaatcaat tacggggtca ttagttcata gcccatatat ggagttccgc gttacataac     300
   ttacggtaaa tggcccgcct ggctgaccgc ccaacgaccc ccgcccattg acgtcaataa     360
   tgacgtatgt tcccatagta acgccaatag ggactttcca ttgacgtcaa tgggtggagt     420
   atttacggta aactgcccac ttggcagtac atcaagtgta tcatatgcca agtacgcccc 480
   ctattgacgt caatgacggt aaatggcccg cctggcatta tgcccagtac atgaccttat 540
   gggactttcc tacttggcag tacatctacg tattagtcat cgctattacc atgtcgaggc 600
   cacgttctgc ttcactctcc ccatctcccc cccctcccca cccccaattt tgtatttatt 660
   tattttttaa ttattttgtg cagcgatggg ggcggggggg gggggcgcgc gccaggcggg 720
   gcggggcggg gcgaggggcg gggcggggcg aggcggagag gtgcggcggc agccaatcag 780
   agcggcgcgc tccgaaagtt tccttttatg gcgaggcggc ggcggcggcg gccctataaa 840
   aagcgaagcg cgcggcgggc gggagcaagc tttattgcgg tagtttatca cagttaaatt 900
   gctaacgcag tcagtgcttc tgacacaaca gtctcgaact taagctgcag aagttggtcg 960
   tgaggcactg ggcaggtaag tatcaaggtt acaagacagg tttaaggaga ccaatagaaa 1020
   ctgggcttgt cgagacagag aagactcttg cgtttctgat aggcacctat tggtcttact 1080
   gacatccact ttgcctttct ctccacaggt gtccactccc agttcaatta cagctcttaa 1140
   ggctagagta cttaatacga ctcactatag gctagcgcgc cgaattcggc acgaggaaaa 1200
   aggcagctcc gcgcgctctc ccccaagagc agaggcttgc ttgtagagtg acgatctgag 1260
   ctacggggtc ttaagtgcgt cagggcgtgg aggtctggcg ggagacgcat agttacagcg 1320
   cgtccgttct ccgtctcgca gccggcacag ctagagcttc gagcgcagcg cggccatgga 1380
   tcccagcagc aagaaggtga cgggccgcct catgttggct gtgggaggag cagtgctcgg 1440
   atcactgcag ttcggctata acactggtgt catcaacgcc ccccagaagg ttattgagga 1500
   gttctacaat caaacatgga accaccgcta cggagagccc atcccatcca ccacactcac 1560
   cacgctttgg tctctctccg tggccatctt ctctgtcggg ggcatgattg gttccttctc 1620
   tgtcggcctc tttgttaatc gctttggcag gcggaactcc atgctgatga tgaacctgtt 1680
   ggcctttgtg gctgctgtgc ttatgggctt ctccaaactg ggcaagtcct ttgagatgct 1740
   gatcctgggc cgcttcatca tcggtgtgta ctgcggcctg actactggct ttgtgcccat 1800
   gtatgtggga gaggtgtcac ctacagctct acgtggagcc ctaggcacac tgcaccagct 1860
   gggaatcgtc gttggcatcc ttattgccca ggtgtttggc ttagactcca tcatgggcaa 1920
   tgcagacttg tggcctctgc tgctcagtgt catcttcatc ccagccctgc tacagtgtat 1980
   cctgttgccc ttctgccccg agagcccccg cttcctgctc atcaatcgta acgaggagaa 2040
   ccgggccaag agtgtgctga agaagcttcg agggacagcc gatgtgaccc gagacctgca 2100
   ggagatgaaa gaagagggtc ggcagatgat gcgggagaag aaggtcacca tcttggagct 2160
   gttccgctca cccgcctacc gccagcccat cctcatcgct gtggtgctgc agctgtccca 2220
   gcagctgtcg ggtatcaatg ctgtgttcta ctactcaacg agcatcttcg agaaggcagg 2280
   tgtgcagcag cctgtgtacg ccaccatcgg ctccggtatc gtcaacacgg ccttcactgt 2340
   ggtgtcgctg tttgttgtag agcgagctgg acgacggacc ctgcacctca ttggcctggc 2400
   tggcatggca ggctgtgctg tgctcatgac catcgccctg gccttgctgg aacggctgcc 2460
   ttggatgtcc tatctgagca tcgtggccat ctttggcttt gtggccttct ttgaagtagg 2520
   ccctggtcct attccatggt tcattgtggc cgagctgttc agccaggggc cccgtcctgc 2580
   tgctattgct gtggctggct tctccaactg gacctcaaac ttcattgtgg gcatgtgctt 2640
   ccagtatgtg gagcaactgt gcggccccta cgtcttcatc atcttcacgg tgctcctcgt 2700
   gctcttcttc atcttcacct acttcaaagt ccctgagacc aaaggccgaa ccttcgatga 2760
   gatcgcttcc ggcttccggc aggggggtgc cagccaaagt gacaagacac ccgaggagct 2820
   cttccaccct ctgggggcgg actcccaagt gaccggtaat tttgaccttc ttaagcttgc 2880
   gggagacgtc gagtccaacc ctgggcccgc catggtgagc aagggcgagg agctgttcac 2940
   cggggtggtg cccatcctgg tcgagctgga cggcgacgta aacggccaca agttcagcgt 3000
   gtccggcgag ggcgagggcg atgccaccta cggcaagctg accctgaagt tcatctgcac 3060
   caccggcaag ctgcccgtgc cctggcccac cctcgtgacc accctgacct acggcgtgca 3120
   gtgcttcagc cgctaccccg accacatgaa gcagcacgac ttcttcaagt ccgccatgcc 3180
   cgaaggctac gtccaggagc gcaccatctt cttcaaggac gacggcaact acaagacccg 3240
   cgccgaggtg aagttcgagg gcgacaccct ggtgaaccgc atcgagctga agggcatcga 3300
   cttcaaggag gacggcaaca tcctggggca caagctggag tacaactaca acagccacaa 3360
   cgtctatatc atggccgaca agcagaagaa cggcatcaag gtgaacttca agatccgcca 3420
   caacatcgag gacggcagcg tgcagctcgc cgaccactac cagcagaaca cccccatcgg 3480
   cgacggcccc gtgctgctgc ccgacaacca ctacctgagc acccagtccg ccctgagcaa 3540
   agaccccaac gagaagcgcg atcacatggt cctgctggag ttcgtgaccg ccgccgggat 3600
   cactctcggc atggacgagc tgtacaagta aagcggccat caagcttatc gggccgctct 3660
   agagtatccc tcgactctag agtcgacccg ggcggcctcg aggacggggt gaactacgcc 3720
   tgaggatccg atctttttcc ctctgccaaa aattatgggg acatcatgaa gccccttgag 3780
   catctgactt ctggctaata aaggaaattt attttcattg caatagtgtg ttggaatttt 3840
   ttgtgtctct cactcggaag caattcgttg atctgaattt cgaccaccca taatacccat 3900
   taccctggta gataagtagc atggcgggtt aatcattaac tacaaggaac ccctagtgat 3960
   ggagttggcc actccctctc tgcgcgctcg ctcgctcact gaggccgggc gaccaaaggt 4020
   cgcccgacgc ccgggctttg cccgggcggc ctcagtgagc gagcgagcgc gcagccttaa 4080
   ttaacctaat tcactggccg tcgttttaca acgtcgtgac tgggaaaacc ctggcgttac 4140
   ccaacttaat cgccttgcag cacatccccc tttcgccagc tggcgtaata gcgaagaggc 4200
   ccgcaccgat cgcccttccc aacagttgcg cagcctgaat ggcgaatggg acgcgccctg 4260
   tagcggcgca ttaagcgcgg cgggtgtggt ggttacgcgc agcgtgaccg ctacacttgc 4320
   cagcgcccta gcgcccgctc ctttcgcttt cttcccttcc tttctcgcca cgttcgccgg 4380
   ctttccccgt caagctctaa atcgggggct ccctttaggg ttccgattta gtgctttacg 4440
   gcacctcgac cccaaaaaac ttgattaggg tgatggttca cgtagtgggc catcgccctg 4500
   atagacggtt tttcgccctt tgacgttgga gtccacgttc tttaatagtg gactcttgtt 4560
   ccaaactgga acaacactca accctatctc ggtctattct tttgatttat aagggatttt 4620
   gccgatttcg gcctattggt taaaaaatga gctgatttaa caaaaattta acgcgaattt 4680
   taacaaaata ttaacgctta caatttaggt ggcacttttc ggggaaatgt gcgcggaacc 4740
   cctatttgtt tatttttcta aatacattca aatatgtatc cgctcatgag acaataaccc 4800
   tgataaatgc ttcaataata ttgaaaaagg aagagtatga gtattcaaca tttccgtgtc 4860
   gcccttattc ccttttttgc ggcattttgc cttcctgttt ttgctcaccc agaaacgctg 4920
   gtgaaagtaa aagatgctga agatcagttg ggtgcacgag tgggttacat cgaactggat 4980
   ctcaacagcg gtaagatcct tgagagtttt cgccccgaag aacgttttcc aatgatgagc 5040
   acttttaaag ttctgctatg tggcgcggta ttatcccgta ttgacgccgg gcaagagcaa 5100
   ctcggtcgcc gcatacacta ttctcagaat gacttggttg agtactcacc agtcacagaa 5160
   aagcatctta cggatggcat gacagtaaga gaattatgca gtgctgccat aaccatgagt 5220
   gataacactg cggccaactt acttctgaca acgatcggag gaccgaagga gctaaccgct 5280
   tttttgcaca acatggggga tcatgtaact cgccttgatc gttgggaacc ggagctgaat 5340
   gaagccatac caaacgacga gcgtgacacc acgatgcctg tagcaatggc aacaacgttg 5400
   cgcaaactat taactggcga actacttact ctagcttccc ggcaacaatt aatagactgg 5460
   atggaggcgg ataaagttgc aggaccactt ctgcgctcgg cccttccggc tggctggttt 5520
   attgctgata aatctggagc cggtgagcgt gggtctcgcg gtatcattgc agcactgggg 5580
   ccagatggta agccctcccg tatcgtagtt atctacacga cggggagtca ggcaactatg 5640
   gatgaacgaa atagacagat cgctgagata ggtgcctcac tgattaagca ttggtaactg 5700
   tcagaccaag tttactcata tatactttag attgatttaa aacttcattt ttaatttaaa 5760
   aggatctagg tgaagatcct ttttgataat ctcatgacca aaatccctta acgtgagttt 5820
   tcgttccact gagcgtcaga ccccgtagaa aagatcaaag gatcttcttg agatcctttt 5880
   tttctgcgcg taatctgctg cttgcaaaca aaaaaaccac cgctaccagc ggtggtttgt 5940
   ttgccggatc aagagctacc aactcttttt ccgaaggtaa ctggcttcag cagagcgcag 6000
   ataccaaata ctgttcttct agtgtagccg tagttaggcc accacttcaa gaactctgta 6060
   gcaccgccta catacctcgc tctgctaatc ctgttaccag tggctgctgc cagtggcgat 6120
   aagtcgtgtc ttaccgggtt ggactcaaga cgatagttac cggataaggc gcagcggtcg 6180
   ggctgaacgg ggggttcgtg cacacagccc agcttggagc gaacgaccta caccgaactg 6240
   agatacctac agcgtgagct atgagaaagc gccacgcttc ccgaagggag aaaggcggac 6300
   aggtatccgg taagcggcag ggtcggaaca ggagagcgca cgagggagct tccaggggga 6360
   aacgcctggt atctttatag tcctgtcggg tttcgccacc tctgacttga gcgtcgattt 6420
   ttgtgatgct cgtcaggggg gcggagccta tggaaaaacg ccagcaacgc ggccttttta 6480
   cggttcctgg ccttttgctg gccttttgct cacatgttct ttcctgcgtt atcccctgat 6540
   tctgtggata accgtattac cgcctttgag tgagctgata ccgctcgccg cagccgaacg 6600
   accgagcgca gcgagtcagt gagcgaggaa gcggaagagc gcccaatacg caaaccgcct 6660
   ctccccgcgc gttggccgat tcattaatgc agctggcacg acaggtttcc cgactggaaa 6720
   gcgggcagtg agcgcaacgc aattaatgtg agttagctca ctcattaggc accccaggct 6780
   ttacacttta tgcttccggc tcgtatgttg tgtggaattg tgagcggata acaatttcac 6840
   acaggaaaca gctatgacca tgattacgcc agatttaatt aaggc 6885
<210> 89
   <211> 130
   <212> DNA
   <213> Artificial Sequence
<220>
   <223> Description of Artificial Sequence: Synthetic polynucleotide
<220>
   <223> 5'ITR
<400> 89
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct 130
<210> 90
   <211> 382
   <212> DNA
   <213> Artificial Sequence
<220>
   <223> Description of Artificial Sequence: Synthetic polynucleotide
<220>
   <223> CMV IE enhancer
<400> 90
   ctagtcgaca ttgattattg actagttatt aatagtaatc aattacgggg tcattagttc        60
   atagcccata tatggagttc cgcgttacat aacttacggt aaatggcccg cctggctgac       120
   cgcccaacga cccccgccca ttgacgtcaa taatgacgta tgttcccata gtaacgccaa       180
   tagggacttt ccattgacgt caatgggtgg agtatttacg gtaaactgcc cacttggcag       240
   tacatcaagt gtatcatatg ccaagtacgc cccctattga cgtcaatgac ggtaaatggc       300
   ccgcctggca ttatgcccag tacatgacct tatgggactt tcctacttgg cagtacatct       360
   acgtattagt catcgctatt ac                                                382
<210> 91
   <211> 382
   <212> DNA
   <213> Artificial Sequence
<220>
   <223> Description of Artificial Sequence: Synthetic polynucleotide
<220>
   <223> CB promoter
<400> 91
   ctagtcgaca ttgattattg actagttatt aatagtaatc aattacgggg tcattagttc        60
   atagcccata tatggagttc cgcgttacat aacttacggt aaatggcccg cctggctgac       120
   cgcccaacga cccccgccca ttgacgtcaa taatgacgta tgttcccata gtaacgccaa       180
   tagggacttt ccattgacgt caatgggtgg agtatttacg gtaaactgcc cacttggcag       240
   tacatcaagt gtatcatatg ccaagtacgc cccctattga cgtcaatgac ggtaaatggc       300
   ccgcctggca ttatgcccag tacatgacct tatgggactt tcctacttgg cagtacatct       360
   acgtattagt catcgctatt ac                                                382
<210> 92
   <211> 717
   <212> DNA
   <213> Artificial Sequence
<220>
   <223> Description of Artificial Sequence: Synthetic polynucleotide
<220>
   <223> eGFP
<400> 92
   atggtgagca agggcgagga gctgttcacc ggggtggtgc ccatcctggt cgagctggac 60
   ggcgacgtaa acggccacaa gttcagcgtg tccggcgagg gcgagggcga tgccacctac 120
   ggcaagctga ccctgaagtt catctgcacc accggcaagc tgcccgtgcc ctggcccacc 180
   ctcgtgacca ccctgaccta cggcgtgcag tgcttcagcc gctaccccga ccacatgaag 240
   cagcacgact tcttcaagtc cgccatgccc gaaggctacg tccaggagcg caccatcttc 300
   ttcaaggacg acggcaacta caagacccgc gccgaggtga agttcgaggg cgacaccctg 360
   gtgaaccgca tcgagctgaa gggcatcgac ttcaaggagg acggcaacat cctggggcac 420
   aagctggagt acaactacaa cagccacaac gtctatatca tggccgacaa gcagaagaac 480
   ggcatcaagg tgaacttcaa gatccgccac aacatcgagg acggcagcgt gcagctcgcc 540
   gaccactacc agcagaacac ccccatcggc gacggccccg tgctgctgcc cgacaaccac 600
   tacctgagca cccagtccgc cctgagcaaa gaccccaacg agaagcgcga tcacatggtc 660
   ctgctggagt tcgtgaccgc cgccgggatc actctcggca tggacgagct gtacaag 717
<210> 93
   <211> 1476
   <212> DNA
   <213> Artificial Sequence
<220>
   <223> Description of Artificial Sequence: Synthetic polynucleotide
<220>
   <223> mGlut1 cDNA and 3'UTR
<400> 93
   atggatccca gcagcaagaa ggtgacgggc cgcctcatgt tggctgtggg aggagcagtg      60
   ctcggatcac tgcagttcgg ctataacact ggtgtcatca acgcccccca gaaggttatt     120
   gaggagttct acaatcaaac atggaaccac cgctacggag agcccatccc atccaccaca     180
   ctcaccacgc tttggtctct ctccgtggcc atcttctctg tcgggggcat gattggttcc     240
   ttctctgtcg gcctctttgt taatcgcttt ggcaggcgga actccatgct gatgatgaac     300
   ctgttggcct ttgtggctgc tgtgcttatg ggcttctcca aactgggcaa gtcctttgag     360
   atgctgatcc tgggccgctt catcatcggt gtgtactgcg gcctgactac tggctttgtg     420
   cccatgtatg tgggagaggt gtcacctaca gctctacgtg gagccctagg cacactgcac     480
   cagctgggaa tcgtcgttgg catccttatt gcccaggtgt ttggcttaga ctccatcatg     540
   ggcaatgcag acttgtggcc tctgctgctc agtgtcatct tcatcccagc cctgctacag     600
   tgtatcctgt tgcccttctg ccccgagagc ccccgcttcc tgctcatcaa tcgtaacgag    660
   gagaaccggg ccaagagtgt gctgaagaag cttcgaggga cagccgatgt gacccgagac     720
   ctgcaggaga tgaaagaaga gggtcggcag atgatgcggg agaagaaggt caccatcttg     780
   gagctgttcc gctcacccgc ctaccgccag cccatcctca tcgctgtggt gctgcagctg     840
   tcccagcagc tgtcgggtat caatgctgtg ttctactact caacgagcat cttcgagaag     900
   gcaggtgtgc agcagcctgt gtacgccacc atcggctccg gtatcgtcaa cacggccttc     960
   actgtggtgt cgctgtttgt tgtagagcga gctggacgac ggaccctgca cctcattggc    1020
   ctggctggca tggcaggctg tgctgtgctc atgaccatcg ccctggcctt gctggaacgg    1080
   ctgccttgga tgtcctatct gagcatcgtg gccatctttg gctttgtggc cttctttgaa    1140
   gtaggccctg gtcctattcc atggttcatt gtggccgagc tgttcagcca ggggccccgt    1200
   cctgctgcta ttgctgtggc tggcttctcc aactggacct caaacttcat tgtgggcatg    1260
   tgcttccagt atgtggagca actgtgcggc ccctacgtct tcatcatctt cacggtgctc    1320
   ctcgtgctct tcttcatctt cacctacttc aaagtccctg agaccaaagg ccgaaccttc    1380
   gatgagatcg cttccggctt ccggcagggg ggtgccagcc aaagtgacaa gacacccgag    1440
   gagctcttcc accctctggg ggcggactcc caagtg                              1476
<210> 94
   <211> 127
   <212> DNA
   <213> Artificial Sequence
<220>
   <223> Description of Artificial Sequence: Synthetic polynucleotide
<220>
   <223> Poly A signal
<400> 94
   gatctttttc cctctgccaa aaattatggg gacatcatga agccccttga gcatctgact 60
   tctggctaat aaaggaaatt tattttcatt gcaatagtgt gttggaattt tttgtgtctc 120
   tcactcg 127
<210> 95
   <211> 130
   <212> DNA
   <213> Artificial Sequence
<220>
   <223> Description of Artificial Sequence: Synthetic polynucleotide
<220>
   <223> 3' ITR
<400> 95
   aggaacccct agtgatggag ttggccactc cctctctgcg cgctcgctcg ctcactgagg 60
   ccgggcgacc aaaggtcgcc cgacgcccgg gctttgcccg ggcggcctca gtgagcgagc 120
   gagcgcgcag 130
<210> 96
   <211> 2208
   <212> DNA
   <213> Artificial Sequence
<220>
   <223> Description of Artificial Sequence: Synthetic polynucleotide
<220>
   <223> AAV9 DNA
<400> 96
   atggctgccg atggttatct tccagattgg ctcgaggaca accttagtga aggaattcgc 60
   gagtggtggg ctttgaaacc tggagcccct caacccaagg caaatcaaca acatcaagac 120
   aacgctcgag gtcttgtgct tccgggttac aaataccttg gacccggcaa cggactcgac 180
   aagggggagc cggtcaacgc agcagacgcg gcggccctcg agcacgacaa ggcctacgac 240
   cagcagctca aggccggaga caacccgtac ctcaagtaca accacgccga cgccgagttc 300
   caggagcggc tcaaagaaga tacgtctttt gggggcaacc tcgggcgagc agtcttccag 360
   gccaaaaaga ggcttcttga acctcttggt ctggttgagg aagcggctaa gacggctcct 420
   ggaaagaaga ggcctgtaga gcagtctcct caggaaccgg actcctccgc gggtattggc 480
   aaatcgggtg cacagcccgc taaaaagaga ctcaatttcg gtcagactgg cgacacagag 540
   tcagtcccag accctcaacc aatcggagaa cctcccgcag ccccctcagg tgtgggatct 600
   cttacaatgg cttcaggtgg tggcgcacca gtggcagaca ataacgaagg tgccgatgga 660
   gtgggtagtt cctcgggaaa ttggcattgc gattcccaat ggctggggga cagagtcatc 720
   accaccagca cccgaacctg ggccctgccc acctacaaca atcacctcta caagcaaatc 780
   tccaacagca catctggagg atcttcaaat gacaacgcct acttcggcta cagcaccccc 840
   tgggggtatt ttgacttcaa cagattccac tgccacttct caccacgtga ctggcagcga 900
   ctcatcaaca acaactgggg attccggcct aagcgactca acttcaagct cttcaacatt 960
   caggtcaaag aggttacgga caacaatgga gtcaagacca tcgccaataa ccttaccagc 1020
   acggtccagg tcttcacgga ctcagactat cagctcccgt acgtgctcgg gtcggctcac 1080
   gagggctgcc tcccgccgtt cccagcggac gttttcatga ttcctcagta cgggtatctg 1140
   acgcttaatg atggaagcca ggccgtgggt cgttcgtcct tttactgcct ggaatatttc 1200
   ccgtcgcaaa tgctaagaac gggtaacaac ttccagttca gctacgagtt tgagaacgta 1260
   cctttccata gcagctacgc tcacagccaa agcctggacc gactaatgaa tccactcatc 1320
   gaccaatact tgtactatct ctcaaagact attaacggtt ctggacagaa tcaacaaacg 1380
   ctaaaattca gtgtggccgg acccagcaac atggctgtcc agggaagaaa ctacatacct 1440
   ggacccagct accgacaaca acgtgtctca accactgtga ctcaaaacaa caacagcgaa 1500
   tttgcttggc ctggagcttc ttcttgggct ctcaatggac gtaatagctt gatgaatcct 1560
   ggacctgcta tggccagcca caaagaagga gaggaccgtt tctttccttt gtctggatct 1620
   ttaatttttg gcaaacaagg aactggaaga gacaacgtgg atgcggacaa agtcatgata 1680
   accaacgaag aagaaattaa aactactaac ccggtagcaa cggagtccta tggacaagtg 1740
   gccacaaacc accagagtgc ccaagcacag gcgcagaccg gctgggttca aaaccaagga 1800
   atacttccgg gtatggtttg gcaggacaga gatgtgtacc tgcaaggacc catttgggcc 1860
   aaaattcctc acacggacgg caactttcac ccttctccgc tgatgggagg gtttggaatg 1920
   aagcacccgc ctcctcagat cctcatcaaa aacacacctg tacctgcgga tcctccaacg 1980
   gccttcaaca aggacaagct gaactctttc atcacccagt attctactgg ccaagtcagc 2040
   gtggagatcg agtgggagct gcagaaggaa aacagcaagc gctggaaccc ggagatccag 2100
   tacacttcca actattacaa gtctaataat gttgaatttg ctgttaatac tgaaggtgta 2160
   tatagtgaac cccgccccat tggcaccaga tacctgactc gtaatctg 2208
<210> 97
   <211> 736
   <212> PRT
   <213> Artificial Sequence
<220>
   <223> Description of Artificial Sequence: Synthetic polypeptide
<220>
   <223> AAV9 protein
<400> 97
   Met Ala Ala Asp Gly Tyr Leu Pro Asp Trp Leu Glu Asp Asn Leu Ser
   1               5                   10                  15
   Glu Gly Ile Arg Glu Trp Trp Ala Leu Lys Pro Gly Ala Pro Gln Pro
               20                  25                  30
   Lys Ala Asn Gln Gln His Gln Asp Asn Ala Arg Gly Leu Val Leu Pro
           35                  40                  45
   Gly Tyr Lys Tyr Leu Gly Pro Gly Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Lys Tyr Asn His Ala
                   85                  90                  95
   Asp Ala Glu Phe Gln Glu Arg Leu Lys Glu Asp Thr Ser Phe Gly Gly
               100                 105                 110
   Asn Leu Gly Arg Ala Val Phe Gln Ala Lys Lys Arg Leu Leu Glu Pro
           115                 120                 125
   Leu Gly Leu Val Glu Glu Ala Ala Lys Thr Ala Pro Gly Lys Lys Arg
       130                 135                 140
   Pro Val Glu Gln Ser Pro Gln Glu Pro Asp Ser Ser Ala Gly Ile Gly
   145                 150                 155                 160
   Lys Ser Gly Ala Gln Pro Ala Lys Lys Arg Leu Asn Phe Gly Gln Thr
                   165                 170                 175
   Gly Asp Thr Glu Ser Val Pro Asp Pro Gln Pro Ile Gly Glu Pro Pro
               180                 185                 190
   Ala Ala Pro Ser Gly Val Gly Ser Leu Thr Met Ala Ser Gly Gly Gly
           195                 200                 205
   Ala Pro Val Ala Asp Asn Asn Glu Gly Ala Asp Gly Val Gly Ser Ser
       210                 215                 220
   Ser Gly Asn Trp His Cys Asp Ser Gln Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Asn Ser Thr Ser Gly Gly Ser Ser Asn Asp Asn
               260                 265                 270
   Ala Tyr Phe Gly Tyr Ser Thr Pro Trp Gly Tyr Phe Asp Phe Asn Arg
           275                 280                 285
   Phe His Cys His Phe Ser Pro Arg Asp Trp Gln Arg Leu Ile Asn Asn
       290                 295                 300
   Asn Trp Gly Phe Arg Pro Lys Arg Leu Asn Phe Lys Leu Phe Asn Ile
   305                 310                 315                 320
   Gln Val Lys Glu Val Thr Asp Asn Asn Gly Val Lys Thr Ile Ala Asn
                   325                 330                 335
   Asn Leu Thr Ser Thr Val Gln Val Phe Thr Asp Ser Asp Tyr Gln Leu
               340                 345                 350
   Pro Tyr Val Leu Gly Ser Ala His Glu Gly Cys Leu Pro Pro Phe Pro
           355                 360                 365
   Ala Asp Val Phe Met Ile Pro Gln Tyr Gly Tyr Leu Thr Leu Asn Asp
       370                 375                 380
   Gly Ser Gln Ala Val Gly Arg Ser Ser Phe Tyr Cys Leu Glu Tyr Phe
   385 390 395 400
   Pro Ser Gln Met Leu Arg Thr Gly Asn Asn Phe Gln Phe Ser Tyr Glu
   405 410 415
   Phe Glu Asn Val Pro Phe His Ser Ser Tyr Ala His Ser Gln Ser Leu
   420 425 430
   Asp Arg Leu Met Asn Pro Leu Ile Asp Gln Tyr Leu Tyr Tyr Leu Ser
   435 440 445
   Lys Thr Ile Asn Gly Ser Gly Gln Asn Gln Gln Thr Leu Lys Phe Ser
   450 455 460
   Val Ala Gly Pro Ser Asn Met Ala Val Gln Gly Arg Asn Tyr Ile Pro
   465 470 475 480
   Gly Pro Ser Tyr Arg Gln Gln Arg Val Ser Thr Thr Val Thr Gln Asn
   485 490 495
   Asn Asn Ser Glu Phe Ala Trp Pro Gly Ala Ser Ser Trp Ala Leu Asn
   500 505 510
   Gly Arg Asn Ser Leu Met Asn Pro Gly Pro Ala Met Ala Ser His Lys
   515 520 525
   Glu Gly Glu Asp Arg Phe Phe Pro Leu Ser Gly Ser Leu Ile Phe Gly
   530 535 540
   Lys Gln Gly Thr Gly Arg Asp Asn Val Asp Ala Asp Lys Val Met Ile
   545 550 555 560
   Thr Asn Glu Glu Glu Ile Lys Thr Thr Asn Pro Val Ala Thr Glu Ser
   565 570 575
   Tyr Gly Gln Val Ala Thr Asn His Gln Ser Ala Gln Ala Gln Ala Gln
   580 585 590
   Thr Gly Trp Val Gln Asn Gln Gly Ile Leu Pro Gly Met Val Trp Gln
   595 600 605
   Asp Arg Asp Val Tyr Leu Gln Gly Pro Ile Trp Ala Lys Ile Pro His
   610 615 620
   Thr Asp Gly Asn Phe His Pro Ser Pro Leu Met Gly Gly Phe Gly Met
   625                 630                 635                 640
   Lys His Pro Pro Pro Gln Ile Leu Ile Lys Asn Thr Pro Val Pro Ala
                   645                 650                 655
   Asp Pro Pro Thr Ala Phe Asn Lys Asp Lys Leu Asn Ser Phe Ile Thr
               660                 665                 670
   Gln Tyr Ser Thr Gly Gln Val Ser Val Glu Ile Glu Trp Glu Leu Gln
           675                 680                 685
   Lys Glu Asn Ser Lys Arg Trp Asn Pro Glu Ile Gln Tyr Thr Ser Asn
       690                 695                 700
   Tyr Tyr Lys Ser Asn Asn Val Glu Phe Ala Val Asn Thr Glu Gly Val
   705                 710                 715                 720
   Tyr Ser Glu Pro Arg Pro Ile Gly Thr Arg Tyr Leu Thr Arg Asn Leu
                   725                 730                 735

## Description

### CROSS-REFERENCE TO RELATED APPLICATIONS

This present application claims priority to U.S. Provisional Patent Application Ser. No. 62/130,899 filed March 10, 2015.

### Sequence Listing

The instant application contains a Sequence Listing which has been filed electronically in ASCII format. Said ASCII copy, created on March 4, 2016, is named 01001-003887-WO0_SL.txt and is 189,953 bytes in size.

### FIELD OF THE INVENTION

The present invention relates to recombinant Glut1 adeno-associated viral vector (AAV) constructs and related uses for restoring Glut1 expression in Glut1 deficient mammals.

### BACKGROUND

Glucose is the primary source of energy for the mammalian brain. Glucose transporter 1 (Glut1), also known as solute carrier family 2 is the predominant glucose transporter expressed in the blood-brain barrier (BBB), is responsible for glucose entry into the brain, and is the first identified member of the facilitated glucose transporter family (SLC2A). The human gene *SLC2A1* encoding the Glut1 protein has been localized to the short arm of chromosome 1 (1p34.2) and is 35 kb in length, containing 10 exons encoding a protein of 492 amino acids (SEQ ID NO:79). The protein is highly conserved among different species including human, rat, mouse, and pig. The mouse *Slc2a1* gene encoding the mGlut1 protein is localized to chromosome 4 and has a very similar gene structure to human *SLC2A1* (Mouse Genome Informatics) (the 492 amino acid mGlut1 sequence is SEQ ID NO:78). Mouse *Slc2a1* cDNA (NM 011400) is >97% identical to that of human *SLC2A1c*DNA. (See: Mueckler M et al, 1985; Veggiotti, P et al, 2013; Seidner et al, 1998).

Glut1 deficiency syndrome (Glut1 DS, OMIM 606777) is a rare but debilitating childhood neurological disorder caused by haplo-insufficiency of the *SLC2A1* gene. Glut1 deficiency syndrome is an autosomal-dominant disorder. The most prominent patient phenotype includes infantile seizures, acquired microcephaly, developmental delay and hypoglycoracchia (De Vivo D.C. *et al.* 1991).

Current treatments for the disease include the use of ketogenic diets, as ketone bodies form an alternative source of energy for neurons in the brain. However, the diet involves ingesting large quantities of oils and is reported to have only modest effects on neurobehavioral symptoms. There is an ongoing need for better treatments, especially for gene therapy to restore Glut1 expression in patients.

US 2012/0232130 A1 is directed to methods for inhibiting starvation of a cell by enhancing intracellular generation and/or uptake of glucose, pyruvate, lactate and/or NADPH. A suggested strategy to supply cone cells with more glucose is to provide them with a gene encoding a glucose transporter, such as glut1. The gene encoding glut1 is delivered using an AAV2/5 or AAV2/8 vector.

### SUMMARY OF INVENTION

In certain embodiments, the present invention relates to a recombinant adeno-associated vector (rAAV) comprising a nucleic acid sequence comprising a transgene encoding Glut1. In certain embodiments, the Glut1 comprises SEQ ID NO:78 or 79. The rAAV further comprises a chicken Beta-actin promoter wherein the rAAV is capable of crossing the blood-brain barrier (BBB). In certain embodiments, the transgene is capable of being expressed in endothelial cells lining the brain microvasculature. In certain embodiments, the chicken Beta-actin promoter is selected from the group consisting of SEQ ID NO:31, 38, 45, 54, 62, and 70. The rAAV is AAV8 or AAV9. In certain embodiments, the rAAV further comprises miRNA elements selected from the group consisting of SEQ ID NO:48, 56, 59, 64, and 73. In certain embodiments, the rAAV further comprises inverted terminal repeats (ITRs) flanking the miRNA elements.

In certain embodiments, the present invention relates to a composition comprising any of the recombinant AAV's described herein. In certain embodiments, the composition further comprises a pharmaceutical carrier.

In certain embodiments, the present invention relates to a kit comprising a container housing comprising the composition described herein. In certain embodiments, the container is a syringe.

In certain embodiments, the present invention relates to any of the recombinant AAV vectors described herein for use in treating Glut1 deficiency syndrome in a subject in need thereof, wherein the use comprises administering to the subject an effective amount of any of the recombinant AAV vectors described herein which is capable of crossing the BBB and also is capable of being expressed in endothelial cells lining the brain microvasculature.

In certain embodiments, the present invention relates to any of the recombinant AAV vectors described herein for use in alleviating in a subject at least one of the symptoms associated with Glut1 deficiency syndrome selected from the group consisting of hypoglycorrhachia, acquired microcephaly, ataxic and dystonic motor dysfunction, wherein the use comprises administering to the subject an effective amount of any of the recombinant AAV vectors described herein which is capable of crossing the BBB and also which is capable of being expressed in endothelial cells lining the brain microvasculature.

In certain aspects, embodiments of the invention relate to a use in treating Glut1 DS in a subject characterized by the defect or haploinsufficiency of an *SLC2A1* gene. The use may include administering to the subject an effective amount of a recombinant adeno-associated virus carrying a nucleic acid sequence (*i.e.* a transgene) encoding the normal/wild-type Glut1 protein, under the control of a promoter sequence which expresses the Glut1 product in the desired cells. In certain embodiments, the promoter sequence provides for expression of the Glut1 product in BBB cells. In certain embodiments, the expression is in endothelial cells lining the brain microvasculature. In certain embodiments, expression of the transgene gene provides to the cells the product necessary to restore or maintain desired Glut1 levels in the subject. In still another embodiment, the invention provides a composition for treatment of Glut1 DS. Such compositions may be formulated with a carrier and additional components suitable for injection.

Other aspects and advantages of the present invention are described further in the following detailed description of the preferred embodiments thereof.

### BRIEF DESCRIPTION OF THE DRAWINGS

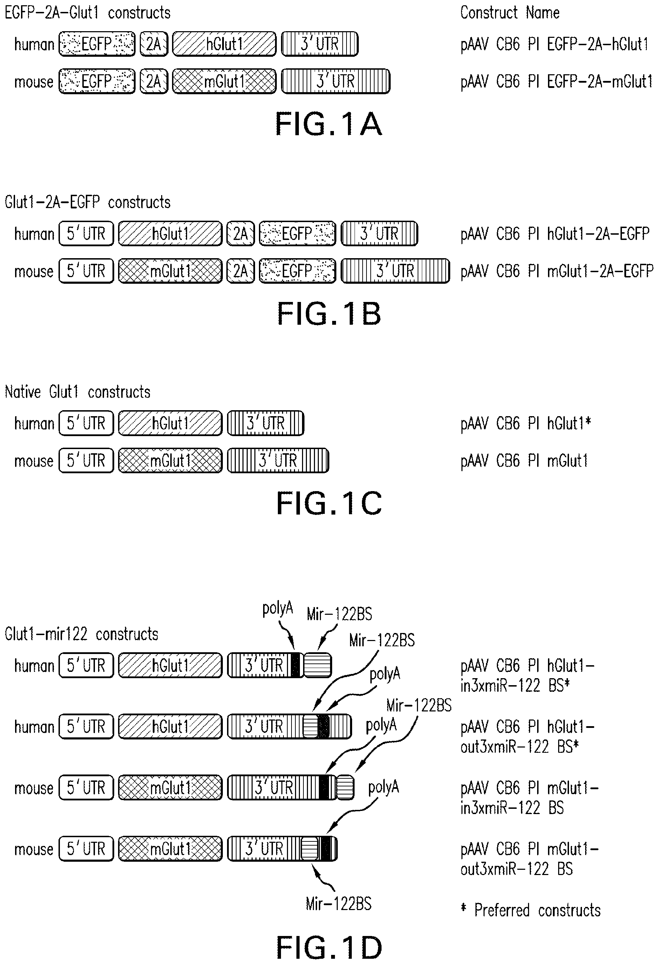

Figures 1A-D are plasmid maps of (ten) Glut1 encoding constructs. Fig.1 A shows two EGFP-2A-Glut1 constructs carrying the Glut1 and EGFP reporter genes, along with the 2A encoding sequence. Fig. 1B shows two Glut1-2A-EGFP constructs. Fig. 1C shows two native Glutl constructs. Fig. 1D shows four constructs carrying a miRNA-122 binding site (Mir-122BS) which serves to selectively turn off expression of Glut1 in liver tissue.

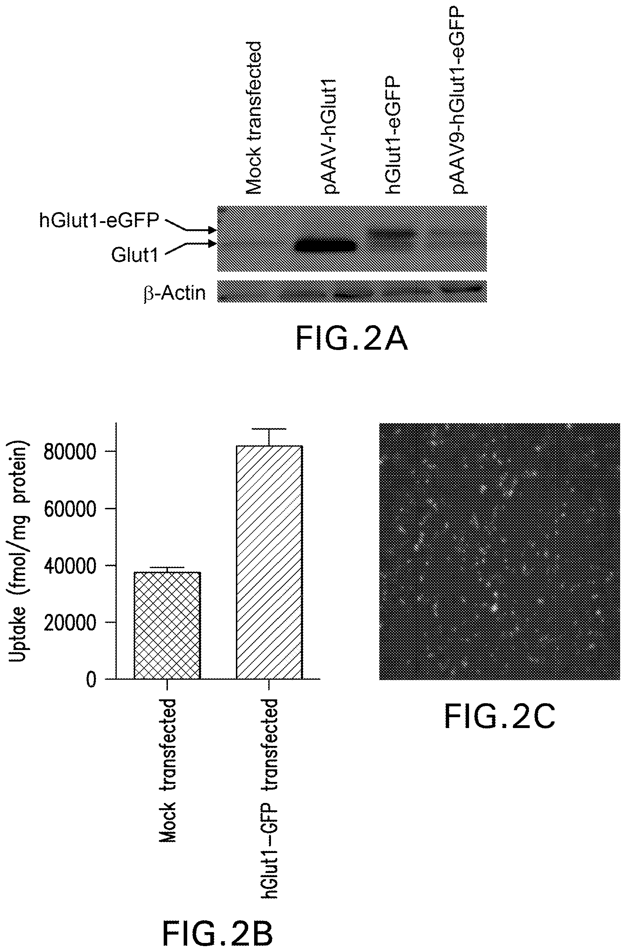

Figures 2A-C are blots and graphs showing expression of the AAV9-hGlutl-eGFP (SEQ ID NO: 80) construct and Glut1 function in an *in vitro* CHO cell assay. Fig. 2A shows a Western blot demonstrating expression of AAV9-hGlutl-eGFP construct. Fig. 2B is a graph showing enhanced uptake of glucose into CHO cells transfected with the AAV9-hGlut1-eGFP construct; demonstrating its ability to perform in a functional assay. Fig. 2C shows GFP fluorescence of the construct following transfection into CHO cells.

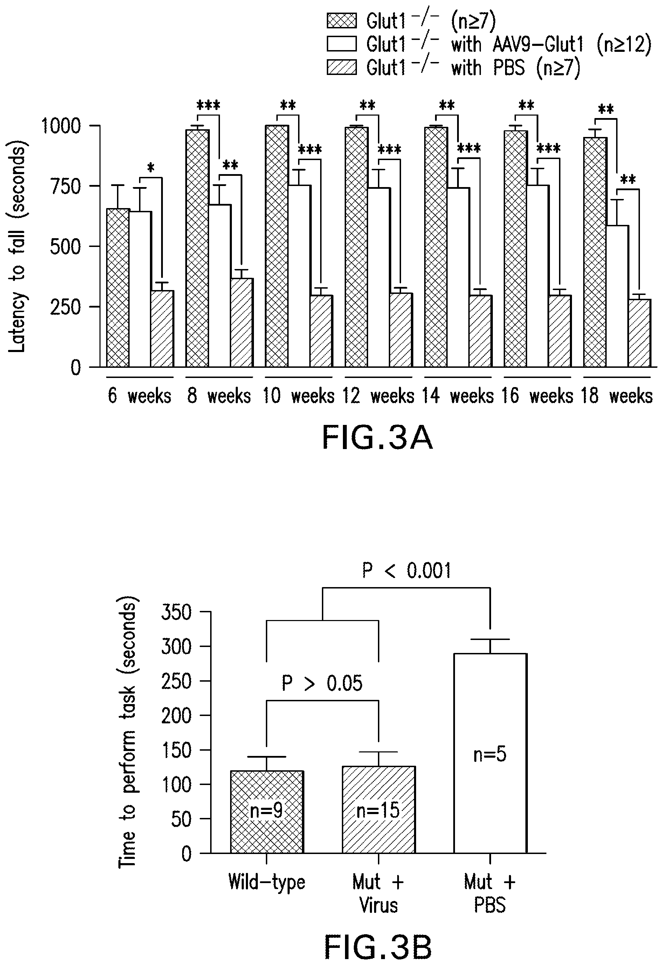

Figures 3A-B are graphs showing enhanced motor performance following reintroduction of the murine *slc2a1* gene into Glut1 DS mice. Fig. 3A is a graph showing improved rotarod performance in AAV9-mGlutl (SEQ ID NO:35) treated mutant mice. Fig. 3B is a graph showing improved vertical pole climbing following restoration of mGlut1 in treated mutant mice.

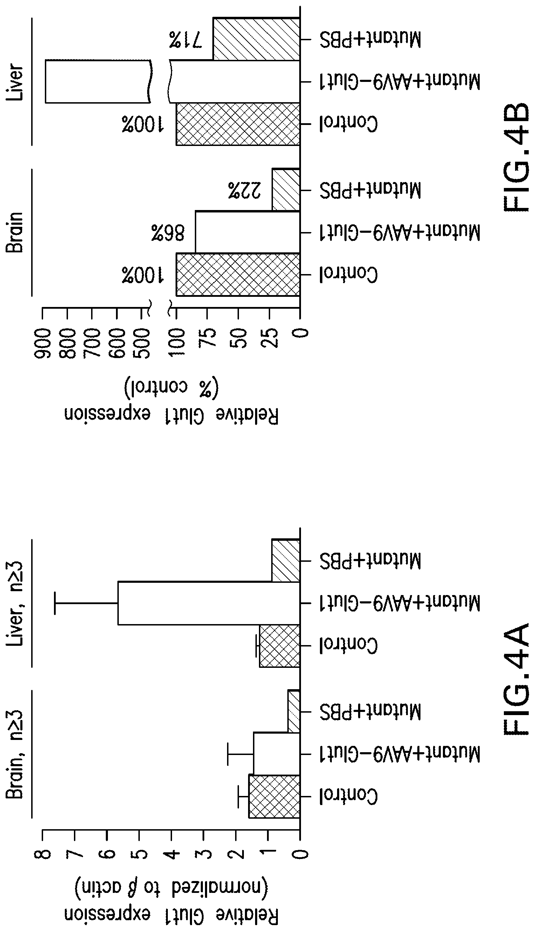

Figures 4A-B are graphs showing enhanced tissue specific expression of Glut1 in AAV9-mGlut1 (SEQ ID NO:35) treated mice. Fig. 4A is a graph showing relative *slc2a1.* gene expression in treated mutants and the relevant controls. Fig. 4B is a graph showing Glut1 expression as a percent of expression in the wild-type Glutl+/+ mice.

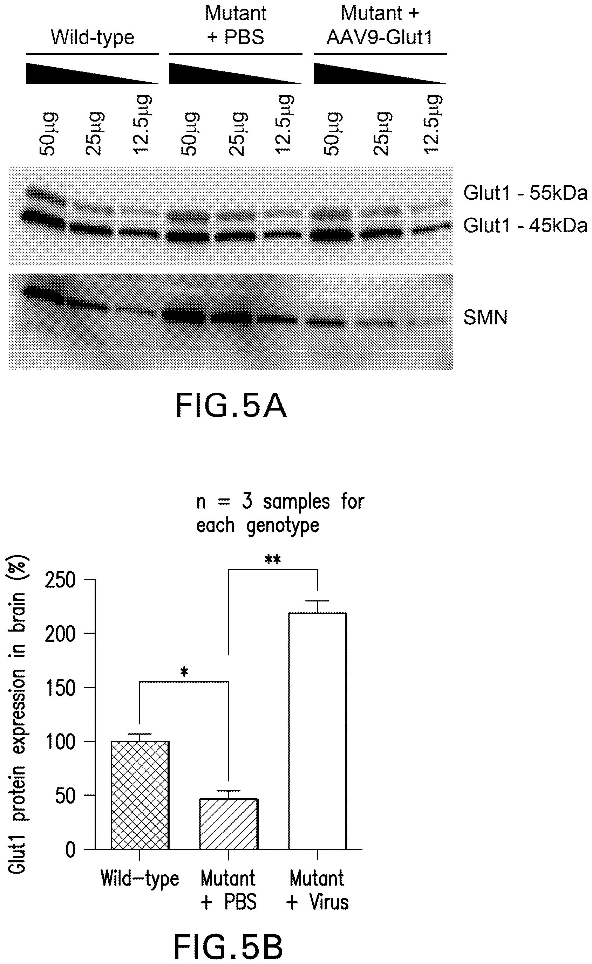

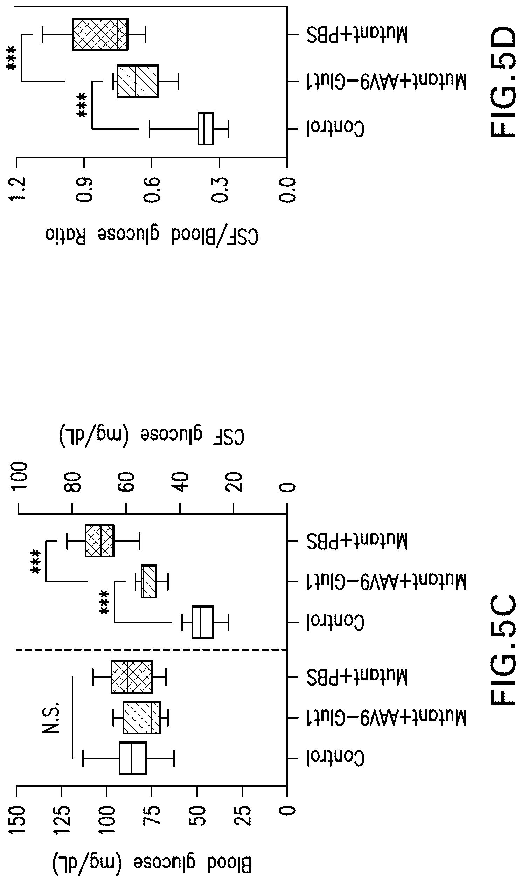

Figures 5A-D are blots and graphs showing increased Glut1 protein and CSF glucose levels in AAV9-mGlut1-treated mutant mice; demonstrating that restoring Glut1 mitigates hypoglycorrachia in Glut1 DS model mice. Fig. 5A is a Western blot of Glutl protein in brain tissue of treated Glut1 DS mutant mice and relevant controls. Fig. 5B is a graph showing the quantification of protein levels in treated Glut1 DS mutant mice and controls. Fig.5C is a graph showing the blood and CSF glucose concentrations in AAV9-mGlut1 (SEQ ID NO:35) treated mice and controls. Fig. 5D is a box and whisker plot showing the ratio of CSF:blood glucose concentrations in the various mice. Note: N.S. - not significant. *, *P <* 0.05, **, *P* < 0.01, ***, *P* < 0.001, one-way ANOVA, N ≥8.

### DETAILED DESCRIPTION

Mutations in the *SLC2A1* (also referred to as *Glut1*) gene result in Glut1 deficiency syndrome (Glut1 DS), a rare but devastating neurodevelopmental disorder (De Vivo D.C. *et al.* 1991). The wild-type Glutl protein is widely expressed. However, its predominant cellular site of action appears to be the endothelial cells of the brain micro-vessels where it functions in the facilitated transport of glucose across the blood-brain barrier. Reduced levels or loss of the protein results in a complex phenotype whose signature features include hypoglycorrhachia, developmental delay and acquired microcephaly. Patients also exhibit a motor phenotype that is both ataxic as well as dystonic. Mice haploinsufficient for the *slc2a1* gene exhibit many of the features of the human disease. A homozygous knockout of the murine *slc2a1* gene is embryonic lethal. Haploinsufficient animal models exhibit many aspects of the human disease. The present invention relates to using AAV9-Glutl constructs to restore Glut1 protein expression in the brain. It is expected that such methods and AAV9-Glut1 constructs can be effective treatments for the human disease. For ease of reference, the vector constructs described herein are referred to as various AAV9-Glut1 constructs, which indicate AAV9 constructs comprising nucleic acid sequences that encode mouse or human Glutl protein, among other elements. As used herein, *SLC2A1* refers to the human gene, while *slc2a1* refers to the mouse gene encoding the respective Glut1 protein.

### DEFINITIONS

So that the invention may be more readily understood, certain technical and scientific terms are specifically defined below. Unless specifically defined elsewhere in this document, all other technical and scientific terms used herein have the meaning commonly understood by one of ordinary skill in the art to which this invention belongs.

As used herein, including the appended claims, the singular forms of words such as "a," "an," and "the," include their corresponding plural references unless the context clearly dictates otherwise.

"Activation," "stimulation," and "treatment," as it applies to cells or to receptors, may have the same meaning, e.g., activation, stimulation, or treatment of a cell or receptor with a ligand, unless indicated otherwise by the context or explicitly. "Ligand" encompasses natural and synthetic ligands, e.g., cytokines, cytokine variants, analogues, muteins, and binding compounds derived from antibodies. "Ligand" also encompasses small molecules, e.g., peptide mimetics of cytokines and peptide mimetics of antibodies. "Activation" can refer to cell activation as regulated by internal mechanisms as well as by external or environmental factors. "Response," e.g., of a cell, tissue, organ, or organism, encompasses a change in biochemical or physiological behavior, e.g., concentration, density, adhesion, or migration within a biological compartment, rate of gene expression, or state of differentiation, where the change is correlated with activation, stimulation, or treatment, or with internal mechanisms such as genetic programming.

"Activity" of a molecule may describe or refer to the binding of the molecule to a ligand or to a receptor, to catalytic activity; to the ability to stimulate gene expression or cell signaling, differentiation, or maturation; to antigenic activity, to the modulation of activities of other molecules, and the like. "Activity" of a molecule may also refer to activity in modulating or maintaining cell-to-cell interactions, e.g., adhesion, or activity in maintaining a structure of a cell, e.g., cell membranes or cytoskeleton. "Activity" can also mean specific activity, e.g., [catalytic activity]/[mg protein], or [immunological activity]/[mg protein], concentration in a biological compartment, or the like. "Activity" may refer to modulation of components of the innate or the adaptive immune systems.

"Administration" and "treatment," as it applies to an animal, human, experimental subject, cell, tissue, organ, or biological fluid, refers to contact of an exogenous pharmaceutical, therapeutic, diagnostic agent, or composition to the animal, human, subject, cell, tissue, organ, or biological fluid. "Administration" and "treatment" can refer, e.g., to therapeutic, pharmacokinetic, diagnostic, research, and experimental methods. Treatment of a cell encompasses contact of a reagent to the cell, as well as contact of a reagent to a fluid, where the fluid is in contact with the cell. "Administration" and "treatment" also means *in vitro* and *ex vivo* treatments, e.g., of a cell, by a reagent, diagnostic, binding compound, or by another cell. The term "subject" includes any organism, preferably an animal, more preferably a mammal (*e.g*., rat, mouse, dog, cat, rabbit) and most preferably a human, including a human patient.

"Treat" or "treating" means to administer a therapeutic agent, such as a composition containing any of the rAAV constructs of the present invention, internally or externally to a subject or patient having one or more disease symptoms, or being suspected of having a disease or being at elevated at risk of acquiring a disease, for which the agent has therapeutic activity. Typically, the agent is administered in an amount effective to alleviate one or more disease symptoms in the treated subject or population, whether by inducing the regression of or inhibiting the progression of such symptom(s) by any clinically measurable degree. The amount of a therapeutic agent that is effective to alleviate any particular disease symptom (also referred to as the "therapeutically effective amount") may vary according to factors such as the disease state, age, and weight of the patient, and the ability of the drug to elicit a desired response in the subject Whether a disease symptom has been alleviated can be assessed by any clinical measurement typically used by physicians or other skilled healthcare providers to assess the severity or progression status of that symptom. While an embodiment of the present invention (e.g., a treatment method or article of manufacture) may not be effective in alleviating the target disease symptom(s) in every subject, it should alleviate the target disease symptom(s) in a statistically significant number of subjects as determined by any statistical test known in the art such as the Student's t-test, the chi²-test, the U-test according to Mann and Whitney, the Kruskal-Wallis test (H-test), Jonckheere-Terpstra-test and the Wilcoxon-test.

"Treatment," as it applies to a human, veterinary, or research subject, refers to therapeutic treatment, prophylactic or preventative measures, to research and diagnostic applications. "Treatment" as it applies to a human, veterinary, or research subject, or cell, tissue, or organ, encompasses transfection of any of the rAAV constructs or related methods of the present invention as applied to a human or animal subject, a cell, tissue, physiological compartment, or physiological fluid.

"Isolated nucleic acid molecule" means a DNA or RNA of genomic, mRNA, cDNA, or synthetic origin or some combination thereof which is not associated with all or a portion of a polynucleotide in which the isolated polynucleotide is found in nature, or is linked to a polynucleotide to which it is not linked in nature. For purposes of this disclosure, it should be understood that "a nucleic acid molecule comprising" a particular nucleotide sequence does not encompass intact chromosomes. Isolated nucleic acid molecules "comprising" specified nucleic acid sequences may include, in addition to the specified sequences, coding sequences for up to ten or even up to twenty or more other proteins or portions or fragments thereof, or may include operably linked regulatory sequences that control expression of the coding region of the recited nucleic acid sequences, and/or may include vector sequences.

The phrase "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to use promoters, polyadenylation signals, and enhancers.

A nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that not all progeny will have precisely identical DNA content, due to deliberate or inadvertent mutations. Mutant progeny that have the same function or biological activity as screened for in the originally transformed cell are included. Where distinct designations are intended, it will be clear from the context.

### Recombinant AAVs

In some aspects, the invention provides isolated AAVs. As used herein with respect to AAVs, the term "isolated" refers to an AAV that has been isolated from its natural environment (e.g., from a host cell, tissue, or subject) or artificially produced. Isolated AAVs may be produced using recombinant methods. Such AAVs are referred to herein as "recombinant AAVs". Recombinant AAVs (rAAVs) preferably have tissue-specific targeting capabilities, such that a transgene of the rAAV will be delivered specifically to one or more predetermined tissue(s). The AAV capsid is an important element in determining these tissue-specific targeting capabilities. Thus, a rAAV having a capsid appropriate for the tissue being targeted can be selected. In some embodiments, the rAAV comprises sequences (such as SEQ ID NO:96) encoding the AAV9 capsid having an amino acid sequence as set forth as SEQ ID NO:97, or a protein having substantial homology thereto.

For targeting the desired tissue in the context of treating Glut1 DS, a preferred rAAV is a combination of AAV9 capsid and AAV2 backbone, resulting in the various rAAV's described herein (See Table 1 and the sequence listing).

Methods for obtaining recombinant AAVs having a desired capsid protein have been described (See, for example, US 2003/0138772). A number of different AAV capsid proteins have been described, for example, those disclosed in G. Gao, et al., J. Virol, 78(12):6381-6388 (June 2004); G. Gao, et al, Proc Natl Acad Sci USA, 100(10):6081-6086 (May 13, 2003); US 2003-0138772, US 2007/0036760, US 2009/0197338. For the desired packaging of the presently described constructs and methods, the AAV9 vector and capsid is preferred. However, it is noted that other suitable AAVs such as rAAVrh.8 and rAAVrh.10, or other similar vectors may be adapted for use in the present invention as defined by the claims. Typically the methods involve culturing a host cell which contains a nucleic acid sequence encoding an AAV capsid protein or fragment thereof; a functional rep gene; a recombinant AAV vector composed of AAV inverted terminal repeats (ITRs) and a transgene; and sufficient helper functions to permit packaging of the recombinant AAV vector into the AAV capsid proteins.

The components to be cultured in the host cell to package a rAAV vector in an AAV capsid may be provided to the host cell in trans. Alternatively, any one or more of the required components (e.g., recombinant AAV vector, rep sequences, cap sequences, and/or helper functions) may be provided by a stable host cell which has been engineered to contain one or more of the required components using methods known to those of skill in the art. Most suitably, such a stable host cell will contain the required component(s) under the control of an inducible promoter. However, the required component(s) may be under the control of a constitutive promoter. In still another alternative, a selected stable host cell may contain selected component(s) under the control of a constitutive promoter and other selected component(s) under the control of one or more inducible promoters. For example, a stable host cell may be generated which is derived from 293 cells (which contain E1 helper functions under the control of a constitutive promoter), but which contain the rep and/or cap proteins under the control of inducible promoters.

The recombinant AAV vector, rep sequences, cap sequences, and helper functions for producing the rAAV may be delivered to the packaging host cell using any appropriate genetic element (vector). The selected genetic element may be delivered by any suitable method, including those described herein. See, e.g., K. Fisher et al, J. Virol., 70:520-532 (1993) and U.S. Pat. No. 5,478,745.

In some embodiments, recombinant AAVs may be produced using the triple transfection method (e.g., as described in detail in U.S. Pat. No. 6,001,650). Typically, the recombinant AAVs are produced by transfecting a host cell with a recombinant AAV vector (comprising a transgene) to be packaged into AAV particles, an AAV helper function vector, and an accessory function vector. An AAV helper function vector encodes the "AAV helper function" sequences (i.e., rep and cap), which function in trans for productive AAV replication and encapsidation. Preferably, the AAV helper function vector supports efficient AAV vector production without generating any detectable wild-type AAV virions (i.e., AAV virions containing functional rep and cap genes). Non-limiting examples of vectors suitable for use with the present invention include pHLP19, described in U.S. Pat. No. 6,001,650 and pRep6cap6 vector, described in U.S. Pat. No. 6,156,303. The accessory function vector encodes nucleotide sequences for non-AAV derived viral and/or cellular functions upon which AAV is dependent for replication (i.e., "accessory functions"). The accessory functions include those functions required for AAV replication, including, without limitation, those moieties involved in activation of AAV gene transcription, stage specific AAV mRNA splicing, AAV DNA replication, synthesis of cap expression products, and AAV capsid assembly. Viral-based accessory functions can be derived from any of the known helper viruses such as adenovirus, herpesvirus (other than herpes simplex virus type-1), and vaccinia virus.

With respect to transfected host cells, the term "transfection" is used to refer to the uptake of foreign DNA by a cell, and a cell has been "transfected" when exogenous DNA has been introduced inside the cell membrane. A number of transfection techniques are generally known in the art. See, e.g., Graham et al. (1973) Virology, 52:456, Sambrook et al. (1989) Molecular Cloning, a laboratory manual, Cold Spring Harbor Laboratories, New York, Davis et al. (1986) Basic Methods in Molecular Biology, Elsevier, and Chu et al. (1981) Gene 13:197. Such techniques can be used to introduce one or more exogenous nucleic acids, such as a nucleotide integration vector and other nucleic acid molecules, into suitable host cells.

A "host cell" refers to any cell that harbors, or is capable of harboring, a substance of interest. Often a host cell is a mammalian cell. A host cell may be used as a recipient of an AAV helper construct, an AAV minigene plasmid, an accessory function vector, or other transfer DNA associated with the production of recombinant AAVs. The term includes the progeny of the original cell which has been transfected. Thus, a "host cell" as used herein may refer to a cell which has been transfected with an exogenous DNA sequence. It is understood that the progeny of a single parental cell may not necessarily be completely identical in morphology or in genomic or total DNA complement as the original parent, due to natural, accidental, or deliberate mutation.

With respect to cells, the term "isolated" refers to a cell that has been isolated from its natural environment (e.g., from a tissue or subject). The term "cell line" refers to a population of cells capable of continuous or prolonged growth and division in vitro. Often, cell lines are clonal populations derived from a single progenitor cell. It is further known in the art that spontaneous or induced changes can occur in karyotype during storage or transfer of such clonal populations. Therefore, cells derived from the cell line referred to may not be precisely identical to the ancestral cells or cultures, and the cell line referred to includes such variants. As used herein, the terms "recombinant cell" refers to a cell into which an exogenous DNA segment, such as DNA segment that leads to the transcription of a biologically-active polypeptide or production of a biologically active nucleic acid such as an RNA, has been introduced.

The term "vector" includes any genetic element, such as a plasmid, phage, transposon, cosmid, chromosome, artificial chromosome, virus, virion, etc., which is capable of replication when associated with the proper control elements and which can transfer gene sequences between cells. Thus, the term includes cloning and expression vehicles, as well as viral vectors. In some embodiments, useful vectors are contemplated to be those vectors in which the nucleic acid segment to be transcribed is positioned under the transcriptional control of a promoter. A "promoter" refers to a DNA sequence recognized by the synthetic machinery of the cell, or introduced synthetic machinery, required to initiate the specific transcription of a gene. The phrases "operatively positioned," "operatively linked," "under control," or "under transcriptional control" means that the promoter is in the correct location and orientation in relation to the nucleic acid to control RNA polymerase initiation and expression of the gene. The term "expression vector or construct" means any type of genetic construct containing a nucleic acid in which part or all of the nucleic acid encoding sequence is capable of being transcribed. In some embodiments, expression includes transcription of the nucleic acid, for example, to generate a biologically-active polypeptide product or inhibitory RNA (e.g., shRNA, miRNA) from a transcribed gene.

### Recombinant AAV Vectors

"Recombinant AAV (rAAV) vectors" described herein are typically composed of, at a minimum, a transgene (e.g. encoding Glutl) and its regulatory sequences, and 5' and 3' AAV inverted terminal repeats (ITRs). It is this recombinant AAV vector which is packaged into a capsid protein and delivered to a selected target cell. In some embodiments, the transgene is a nucleic acid sequence, heterologous to the vector sequences, which encodes a polypeptide, protein, functional RNA molecule (e.g., miRNA, miRNA inhibitor) or other gene product of interest (*e.g*. Glut1). The nucleic acid coding sequence is operatively linked to regulatory components in a manner which permits transgene transcription, translation, and/or expression in a cell of a target tissue.

The AAV sequences of the vector may comprise the cis-acting 5' and 3' inverted terminal repeat sequences (See, e.g., B. J. Carter, in "Handbook of Parvoviruses", ed., P. Tijsser, CRC Press, pp. 155 168 (1990)). The ITR sequences are typically about 145 bp in length. Preferably, substantially the entire sequences encoding the ITRs are used in the molecule, although some degree of minor modification of these sequences is permissible. (See, e.g., texts such as Sambrook et al, "Molecular Cloning. A Laboratory Manual", 2d ed., Cold Spring harbor Laboratory, New York (1989); and K. Fisher et al., J. Virol., 70:520 532 (1996)). An example of such a molecule is a "cis-acting" plasmid containing the transgene, in which the selected transgene sequence and associated regulatory elements are flanked by the 5' and 3' AAV ITR sequences. The AAV ITR sequences may be obtained from any known AAV, including presently identified mammalian AAV types.

In addition to the elements identified above for recombinant AAV vectors, the vector may also include conventional control elements which are operably linked to the transgene in a manner which permits its transcription, translation and/or expression in a cell transfected with the plasmid vector or infected with the virus produced by the invention. As used herein, "operably linked" sequences include both expression control sequences that are contiguous with the gene of interest and expression control sequences that act in trans or at a distance to control the gene of interest. Expression control sequences include appropriate transcription initiation, termination, promoter and enhancer sequences; efficient RNA processing signals such as splicing and polyadenylation (polyA) signals; sequences that stabilize cytoplasmic mRNA; sequences that enhance translation efficiency (i.e., Kozak consensus sequence); sequences that enhance protein stability; and when desired, sequences that enhance secretion of the encoded product. A great number of expression control sequences, including promoters which are native, constitutive, inducible and/or tissue-specific, are known in the art and may be utilized.

As used herein, a nucleic acid sequence (e.g., coding sequence) and regulatory sequences are said to be operably linked when they are covalently linked in such a way as to place the expression or transcription of the nucleic acid sequence under the influence or control of the regulatory sequences. If it is desired that the nucleic acid sequences be translated into a functional protein, two DNA sequences are said to be operably linked if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably linked to a nucleic acid sequence if the promoter region were capable of effecting transcription of that DNA sequence such that the resulting transcript might be translated into the desired protein or polypeptide. Similarly two or more coding regions are operably linked when they are linked in such a way that their transcription from a common promoter results in the expression of two or more proteins having been translated in frame. In some embodiments, operably linked coding sequences yield a fusion protein. In some embodiments, operably linked coding sequences yield a functional RNA (e.g., shRNA, miRNA).

For nucleic acids encoding proteins, a polyadenylation sequence generally is inserted following the transgene sequences and before the 3' AAV ITR sequence. An rAAV construct useful in the present invention may also contain an intron, desirably located between the promoter/enhancer sequence and the transgene. One possible intron sequence is derived from SV-40, and is referred to as the SV-40 T intron sequence. Another vector element that may be used is an internal ribosome entry site (IRES). An IRES sequence is used to produce more than one polypeptide from a single gene transcript. An IRES sequence would be used to produce a protein that contain more than one polypeptide chains. Selection of these and other common vector elements are conventional and many such sequences are available [see, e.g., Sambrook et al, and references cited therein at, for example, pages 3.18 3.26 and 16.17 16.27 and Ausubel et al., Current Protocols in Molecular Biology, John Wiley & Sons, New York, 1989]. In some circumstances, a Foot and Mouth Disease Virus 2A sequence may be included in a polyprotein; this is a small peptide (approximately 18 amino acids in length) that has been shown to mediate the cleavage of polyproteins (Ryan, M D et al., EMBO, 1994; 4: 928-933; Mattion, N M et al., J Virology, November 1996; p. 8124-8127; Furler, S et al., Gene Therapy, 2001; 8: 864-873; and Halpin, C et al., The Plant Journal, 1999; 4: 453-459). The cleavage activity of the 2A sequence has previously been demonstrated in artificial systems including plasmids and gene therapy vectors (AAV and retroviruses) (Ryan, M D et al., EMBO, 1994; 4: 928-933; Mattion, N M et al., J Virology, November 1996; p. 8124-8127; Furler, S et al., Gene Therapy, 2001; 8: 864-873; and Halpin, C et al., The Plant Journal, 1999; 4: 453-459; de Felipe, P et al,, Gene Therapy, 1999; 6: 198-208; de Felipe, P et al., Human Gene Therapy, 2000; 11: 1921-1931.; and Klump, H et al., Gene Therapy, 2001; 8: 811-817).

The precise nature of the regulatory sequences needed for gene expression in host cells may vary between species, tissues or cell types, but shall in general include, as necessary, 5' non-transcribed and 5' non-translated sequences involved with the initiation of transcription and translation respectively, such as a TATA box, capping sequence, CAAT sequence, enhancer elements, and the like. Especially, such 5' non-transcribed regulatory sequences will include a promoter region that includes a promoter sequence for transcriptional control of the operably joined gene. Regulatory sequences may also include enhancer sequences or upstream activator sequences as desired. The vectors may optionally include 5' leader or signal sequences.

Examples of constitutive promoters include, without limitation, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the 13-actin promoter, the phosphoglycerol kinase (PGK) promoter, and the EFla promoter [Invitrogen].

Inducible promoters allow regulation of gene expression and can be regulated by exogenously supplied compounds, environmental factors such as temperature, or the presence of a specific physiological state, e.g., acute phase, a particular differentiation state of the cell, or in replicating cells only. Inducible promoters and inducible systems are available from a variety of commercial sources, including, without limitation, Invitrogen, Clontech and Ariad. Examples of inducible promoters regulated by exogenously supplied promoters include the zinc-inducible sheep metallothionine (MT) promoter, the dexamethasone (Dex)-inducible mouse mammary tumor virus (MMTV) promoter, the T7 polymerase promoter system (WO 98/10088); the ecdysone insect promoter (No et al, Proc. Natl. Acad. Sci. USA, 93:3346-3351 (1996)), the tetracycline-repressible system (Gossen et al, Proc. Natl. Acad. Sci. USA, 89:5547-5551 (1992)), the tetracycline-inducible system (Gossen et al, Science, 268:1766-1769 (1995), see also Harvey et al, Curr. Opin. Chem. Biol., 2:512-518 (1998)), the RU486-inducible system (Wang et al, Nat. Biotech., 15:239-243 (1997) and Wang et al, Gene Ther., 4:432-441 (1997)) and the rapamycin-inducible system (Magari et al, J. Clin. Invest., 100:2865-2872 (1997)). Still other types of inducible promoters which may be useful in this context are those which are regulated by a specific physiological state, e.g., temperature, acute phase, a particular differentiation state of the cell, or in replicating cells only.

In another disclosure the native promoter, or fragment thereof, for the transgene will be used. The native promoter may be preferred when it is desired that expression of the transgene should mimic the native expression. The native promoter may be used when expression of the transgene must be regulated temporally or developmentally, or in a tissue-specific manner, or in response to specific transcriptional stimuli. In a further embodiment, other native expression control elements, such as enhancer elements, polyadenylation sites or Kozak consensus sequences may also be used to mimic the native expression.

In some disclosures the regulatory sequences impart tissue-specific gene expression capabilities. In some cases, the tissue-specific regulatory sequences bind tissue-specific transcription factors that induce transcription in a tissue specific manner. Such tissue-specific regulatory sequences (e.g., promoters, enhancers, etc.) are well known in the art. Exemplary tissue-specific regulatory sequences include, but are not limited to the following tissue specific promoters: neuronal such as neuron-specific enolase (NSE) promoter (Andersen et al., Cell. Mol. Neurobiol., 13:503-15 (1993)), neurofilament light-chain gene promoter (Piccioli et al., Proc. Natl. Acad. Sci. IDSA, 88:5611-5 (1991)), and the neuron-specific vgf gene promoter (Piccioli et al., Neuron, 15:373-84 (1995)). In some disclosures the tissue-specific promoter is a promoter of a gene selected from: neuronal nuclei (NeuN), glial fibrillary acidic protein (GFAP), adenomatous polyposis coli (APC), and ionized calcium-binding adapter molecule 1 (Iba-1). In accordance with the invention, the promoter is a chicken Beta-actin promoter.

In some embodiments, one or more bindings sites for one or more of miRNAs are incorporated in a transgene of a rAAV vector, to inhibit the expression of the transgene in one or more tissues of a subject harboring the transgenes, e.g., non-CNS tissues. The skilled artisan will appreciate that binding sites may be selected to control the expression of a transgene in a tissue specific manner. For example, expression of a transgene in the liver may be inhibited by incorporating a binding site for miR-122 such that mRNA expressed from the transgene binds to and is inhibited by miR-122 in the liver. Expression of a transgene in the heart may be inhibited by incorporating a binding site for miR-133a or miR-1, such that mRNA expressed from the transgene binds to and is inhibited by miR-133a or miR-1 in the heart. The miRNA target sites in the mRNA may be in the 5' UTR, the 3' UTR or in the coding region. Typically, the target site is in the 3' UTR of the mRNA. Furthermore, the transgene may be designed such that multiple miRNAs regulate the mRNA by recognizing the same or multiple sites. The presence of multiple miRNA binding sites may result in the cooperative action of multiple RISCs and provide highly efficient inhibition of expression. The target site sequence may comprise a total of 5-100, 10-60. or more nucleotides. The target site sequence may comprise at least 5 nucleotides of the sequence of a target gene binding site.

### Transgene Coding Sequences

The composition of the transgene sequence of a rAAV vector will depend upon the use to which the resulting vector will be put. For example, one type of transgene sequence includes a reporter sequence, which upon expression produces a detectable signal. In accordance with the invention, the transgene encodes a therapeutic Glut1 protein or therapeutic functional RNA. In another example, the transgene encodes a protein or functional RNA that is intended to be used for research purposes, e.g., to create a somatic transgenic animal model harboring the transgene, e.g., to study the function of the transgene product. In another example, the transgene encodes a protein or functional RNA that is intended to be used to create an animal model of disease. Appropriate transgene coding sequences will be apparent to the skilled artisan.

In some aspects, the invention provides rAAV vectors for use in methods of preventing or treating an *SLC2A1* gene defect (e.g., heritable gene defects, somatic gene alterations) in a mammal, such as for example, a gene defect that results in a Glut1 polypeptide deficiency in a subject, and particularly for treating or reducing the severity or extent of deficiency in a subject manifesting a Glut1 deficiency. In some embodiments, methods involve administration of a rAAV vector that encodes one or more therapeutic peptides, polypeptides, shRNAs, microRNAs, antisense nucleotides, etc. in a pharmaceutically-acceptable carrier to the subject in an amount and for a period of time sufficient to treat the Glut1 disorder in the subject having or suspected of having such a disorder.

### Recombinant AAV Administration

rAAVS are administered in sufficient amounts to transfect the cells of a desired tissue and to provide sufficient levels of gene transfer and expression without undue adverse effects. Conventional and pharmaceutically acceptable routes of administration include, but are not limited to, direct delivery to the selected tissue (e.g., intracerebral administration, intrathecal administration), intravenous, oral, inhalation (including intranasal and intratracheal delivery), intraocular, intravenous, intramuscular, subcutaneous, intradermal, intratumoral, and other parental routes of administration. Routes of administration may be combined, if desired.

Delivery of certain rAAVs to a subject may be, for example, by administration into the bloodstream of the subject. Administration into the bloodstream may be by injection into a vein, an artery, or any other vascular conduit. Moreover, in certain instances, it may be desirable to deliver the rAAVs to brain tissue, meninges, neuronal cells, glial cells, astrocytes, oligodendrocytes, cerebrospinal fluid (CSF), interstitial spaces and the like. In some embodiments, recombinant AAVs may be delivered directly to the spinal cord or brain by injection into the ventricular region, as well as to the striatum (e.g., the caudate nucleus or putamen of the striatum), and neuromuscular junction, or cerebellar lobule, with a needle, catheter or related device, using neurosurgical techniques known in the art, such as by stereotactic injection (see, e.g., Stein et al., J Virol 73:3424-3429, 1999; Davidson et al., PNAS 97:3428-3432, 2000; Davidson et al., Nat. Genet. 3:219-223, 1993; and Alisky and Davidson, Hum. Gene Ther. 11:2315-2329, 2000). In certain circumstances it will be desirable to deliver the rAAV-based therapeutic constructs in suitably formulated pharmaceutical compositions disclosed herein either subcutaneously, intrapancreatically, intranasally, parenterally, intravenously, intramuscularly, intracerebrally, intrathecally, intracerebrally, orally, intraperitoneally, or by inhalation. In some embodiments, the administration modalities as described in U.S. Pat. Nos. 5,543,158; 5,641,515 and 5,399,363 may be used to deliver rAAVs.

### Recombinant AAV Compositions

The rAAVs may be delivered to a subject in compositions according to any appropriate methods known in the art. The rAAV, preferably suspended in a physiologically compatible carrier (e.g., in a composition), may be administered to a subject, e.g., a human, mouse, rat, cat, dog, sheep, rabbit, horse, cow, goat, pig, guinea pig, hamster, chicken, turkey, or a non-human primate (e.g., Macaque). In certain embodiments, compositions may comprise a rAAV alone, or in combination with one or more other viruses (e.g., a second rAAV encoding having one or more different transgenes).

Suitable carriers may be readily selected by one of skill in the art in view of the indication for which the rAAV is directed. For example, one suitable carrier includes saline, which may be formulated with a variety of buffering solutions (e.g., phosphate buffered saline). Other exemplary carriers include sterile saline, lactose, sucrose, calcium phosphate, gelatin, dextran, agar, pectin, peanut oil, sesame oil, and water. The selection of the carrier is not a limitation of the present invention.

Optionally, the compositions of the invention may contain, in addition to the rAAV and carrier(s), other conventional pharmaceutical ingredients, such as preservatives, or chemical stabilizers. Suitable exemplary preservatives include chlorobutanol, potassium sorbate, sorbic acid, sulfur dioxide, propyl gallate, the parabens, ethyl vanillin, glycerin, phenol, and parachlorophenol. Suitable chemical stabilizers include gelatin and albumin.

The dose of rAAV virions required to achieve a desired effect or "therapeutic effect," e.g., the units of dose in vector genomes/per kilogram of body weight (vg/kg), will vary based on several factors including, but not limited to: the route of rAAV administration, the level of gene or RNA expression required to achieve a therapeutic effect, the specific disease or disorder being treated, and the stability of the gene or RNA product. One of skill in the art can readily determine a rAAV virion dose range to treat a subject having a particular disease or disorder based on the aforementioned factors, as well as other factors that are well known in the art. An effective amount of the rAAV is generally in the range of from about 10 µl to about 100 ml of solution containing from about 10⁹ to 10¹⁶ genome copies per subject. Other volumes of solution may be used. The volume used will typically depend, among other things, on the size of the subject, the dose of the rAAV, and the route of administration. For example, for intrathecal or intracerebral administration a volume in range of 1 µl to 10 µl or 10 µl to 100 µl may be used. For intravenous administration a volume in range of 10 µl to 100 µl, 100 µl to 1 ml, 1 ml to 10 ml, or more may be used. In some cases, a dosage between about 10¹⁰ to 10¹² rAAV genome copies per subject is appropriate. In certain embodiments, 10¹² rAAV genome copies per subject is effective to target CNS tissues. In some embodiments the rAAV is administered at a dose of 10¹⁰, 10¹¹, 10¹², 10¹³, 10¹⁴, or 10¹⁵ genome copies per subject. In some embodiments the rAAV is administered at a dose of 10¹⁰, 10¹¹, 10¹², 10¹³, or 10¹⁴ genome copies per kg.

In some embodiments, rAAV compositions are formulated to reduce aggregation of AAV particles in the composition, particularly where high rAAV concentrations are present (e.g., about 10¹³ GC/ml or more). Methods for reducing aggregation of rAAVs are well known in the art and, include, for example, addition of surfactants, pII adjustment, salt concentration adjustment, etc. (See, e.g., Wright F R, et al., Molecular Therapy (2005) 12, 171-178.)

Formulation of pharmaceutically-acceptable excipients and carrier solutions is well-known to those of skill in the art, as is the development of suitable dosing and treatment regimens for using the particular compositions described herein in a variety of treatment regimens. Typically, these formulations may contain at least about 0.1% of the active ingredient or more, although the percentage of the active ingredient(s) may, of course, be varied and may conveniently be between about 1 or 2% and about 70% or 80% or more of the weight or volume of the total formulation. Naturally, the amount of active ingredient in each therapeutically-useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

The pharmaceutical forms suitable for injectable use include sterile aqueous solutions or dispersions and sterile powders for the extemporaneous preparation of sterile injectable solutions or dispersions. Dispersions may also be prepared in glycerol, liquid polyethylene glycols, and mixtures thereof and in oils. Under ordinary conditions of storage and use, these preparations contain a preservative to prevent the growth of microorganisms. In many cases the form is sterile and fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For administration of an injectable aqueous solution, for example, the solution may be suitably buffered, if necessary, and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the host. The person responsible for administration will, in any event, determine the appropriate dose for the individual host.

Sterile injectable solutions are prepared by incorporating the active rAAV in the required amount in the appropriate solvent with various of the other ingredients enumerated herein, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The rAAV compositions disclosed herein may also be formulated in a neutral or salt form. Pharmaceutically-acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug-release capsules, and the like.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Supplementary active ingredients can also be incorporated into the compositions. The phrase "pharmaceutically-acceptable" refers to molecular entities and compositions that do not produce an allergic or similar untoward reaction when administered to a host.

Delivery vehicles such as liposomes, nanocapsules, microparticles, microspheres, lipid particles, vesicles, and the like, may be used for the introduction of the compositions of the present invention into suitable host cells. In particular, the rAAV vector delivered transgenes may be formulated for delivery either encapsulated in a lipid particle, a liposome, a vesicle, a nanosphere, or a nanoparticle or the like.

Such formulations may be preferred for the introduction of pharmaceutically acceptable formulations of the nucleic acids or the rAAV constructs disclosed herein. The formation and use of liposomes is generally known to those of skill in the art. Recently, liposomes were developed with improved serum stability and circulation half-times (U.S. Pat. No. 5,741,516). Further, various methods of liposome and liposome like preparations as potential drug carriers have been described (U.S. Pat. Nos. 5,567,434; 5,552,157; 5,565,213; 5,738,868 and 5,795,587).

Liposomes have been used successfully with a number of cell types that are normally resistant to transfection by other procedures. In addition, liposomes are free of the DNA length constraints that are typical of viral-based delivery systems. Liposomes have been used effectively to introduce genes, drugs, radiotherapeutic agents, viruses, transcription factors and allosteric effectors into a variety of cultured cell lines and animals. In addition, several successful clinical trials examining the effectiveness of liposome-mediated drug delivery have been completed.

Liposomes are formed from phospholipids that are dispersed in an aqueous medium and spontaneously form multilamellar concentric bilayer vesicles (also termed multilamellar vesicles (MLVs). MLVs generally have diameters of from 25 nm to 4 µm, Sonication of MLVs results in the formation of small unilamellar vesicles (SUVs) with diameters in the range of 200 to 500Å, containing an aqueous solution in the core.

Alternatively, nanocapsule formulations of the rAAV may be used. Nanocapsules can generally entrap substances in a stable and reproducible way. To avoid side effects due to intracellular polymeric overloading, such ultrafine particles (sized around 0.1µm) should be designed using polymers able to be degraded in vivo. Biodegradable polyalkyl-cyanoacrylate nanoparticles that meet these requirements are contemplated for use.

In addition to the methods of delivery described above, the following techniques are also contemplated as alternative methods of delivering the rAAV compositions to a host. Sonophoresis (i.e., ultrasound) has been used and described in U.S. Pat. No. 5,656,016 as a device for enhancing the rate and efficacy of drug permeation into and through the circulatory system. Other drug delivery alternatives contemplated are intraosseous injection (U.S. Pat. No. 5,779,708), microchip devices (U.S. Pat. No. 5,797,898), ophthalmic formulations (Bourlais et al., 1998), transdermal matrices (U.S. Pat. Nos. 5,770,219 and 5,783,208) and feedback-controlled delivery (U.S. Pat. No. 5,697,899).

### General Methods Relating to Delivery of rAAV Compositions

The present invention provides stable pharmaceutical compositions comprising rAAV virions. The compositions remain stable and active even when subjected to freeze/thaw cycling and when stored in containers made of various materials, including glass.

Recombinant AAV virions containing a heterologous nucleotide sequence of interest can be used for gene delivery, such as in gene therapy applications, for the production of transgenic animals, in nucleic acid vaccination, ribozyme and antisense therapy, as well as for the delivery of genes in vitro, to a variety of cell types.

Generally, rAAV virions are introduced into the cells of a subject using either in vivo or in vitro transduction techniques. If transduced in vitro, the desired recipient cell will be removed from the subject, transduced with rAAV virions and reintroduced into the subject. Alternatively, syngeneic or xenogeneic cells can be used where those cells will not generate an inappropriate immune response in the subject.

Suitable methods for the delivery and introduction of transduced cells into a subject have been described. For example, cells can be transduced in vitro by combining recombinant AAV virions with the cells e.g., in appropriate media, and screening for those cells harboring the DNA of interest using conventional techniques such as Southern blots and/or PCR, or by using selectable markers. Transduced cells can then be formulated into pharmaceutical compositions, described more fully below, and the composition introduced into the subject by various routes, such as by intramuscular, intravenous, intra-arterial, subcutaneous and intraperitoneal injection, or by injection into smooth muscle, using e.g.. a catheter, or directly into an organ.

For *in vivo* delivery, the rAAV virions will be formulated into a pharmaceutical composition and will generally be administered parenterally, e.g., by intramuscular injection directly into skeletal muscle, intra-articularly, intravenously or directly into an organ.

Appropriate doses will depend on the subject being treated (e.g., human or nonhuman primate or other mammal), age and general condition of the subject to be treated, the severity of the condition being treated, the mode of administration of the rAAV virions, among other factors. An appropriate effective amount can be readily determined by one of skill in the art.

Thus, a "therapeutically effective amount" will fall in a relatively broad range that can be determined through clinical trials. For example, for *in vivo* injection, i.e., injection directly to the subject, a therapeutically effective dose will be on the order of from about 10⁵ to 10¹⁶ of the rAAV virions, more preferably 10⁸ to 10¹⁴ rAAV virions. For in vitro transduction, an effective amount of rAAV virions to be delivered to cells will be on the order of 10⁵ to 10¹³, preferably 10⁸ to 10¹³ of the rAAV virions. If the composition comprises transduced cells to be delivered back to the subject, the amount of transduced cells in the pharmaceutical compositions will be from about 10⁴ to 10¹⁰ cells, more preferably 10⁵ to 10⁸ cells. The dose, of course, depends on the efficiency of transduction, promoter strength, the stability of the message and the protein encoded thereby, etc. Effective dosages can be readily established by one of ordinary skill in the art through routine trials establishing dose response curves.

Dosage treatment may be a single dose schedule or a multiple dose schedule to ultimately deliver the amount specified above. Moreover, the subject may be administered as many doses as appropriate. Thus, the subject may be given, e.g., 10⁵ to 10⁵⁶ rAAV virions in a single dose, or two, four, five, six or more doses that collectively result in delivery of, e.g., 10⁵ to 10¹⁶ rAAV virions. One of skill in the art can readily determine an appropriate number of doses to administer.

Pharmaceutical compositions will thus comprise sufficient genetic material to produce a therapeutically effective amount of the protein of interest, i.e., an amount sufficient to reduce or ameliorate symptoms of the disease state in question or an amount sufficient to confer the desired benefit. Thus, rAAV virions will be present in the subject compositions in an amount sufficient to provide a therapeutic effect when given in one or more doses. The rAAV virions can be provided as lyophilized preparations and diluted in the virion-stabilizing compositions for immediate or future use. Alternatively, the rAAV virions may be provided immediately after production and stored for future use.

The pharmaceutical compositions will also contain a pharmaceutically acceptable excipient. Such excipients include any pharmaceutical agent that does not itself induce the production of antibodies harmful to the individual receiving the composition, and which may be administered without undue toxicity. Pharmaceutically acceptable excipients include, but are not limited to, liquids such as water, saline, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A thorough discussion of pharmaceutically acceptable excipients is available in REMINGTON'S PHARMACEUTICAL SCIENCES (Mack Pub. Co., N.J. 1991).

As used herein, "polymerase chain reaction" or "PCR" refers to a procedure or technique in which specific nucleic acid sequences, RNA and/or DNA, are amplified as described in, *e.g.,* U.S. Pat. No. 4,683,195. Generally, sequence information from the ends of the region of interest or beyond is used to design oligonucleotide primers. These primers will be identical or similar in sequence to opposite strands of the template to be amplified. The 5' terminal nucleotides of the two primers can coincide with the ends of the amplified material. PCR can be used to amplify specific RNA sequences, specific DNA sequences from total genomic DNA, and cDNA transcribed from total cellular RNA, bacteriophage or plasmid sequences, etc. *See generally* Mullis et al. (1987) Cold Spring Harbor Symp. Quant. Biol 51:263; Erlich, ed., (1989) PCR TECHNOLOGY (Stockton Press, N.Y.) As used herein, PCR is considered to be one, but not the only, example of a nucleic acid polymerase reaction method for amplifying a nucleic acid test sample comprising the use of a known nucleic acid as a primer and a nucleic acid polymerase to amplify or generate a specific piece of nucleic acid.

### Nucleic Acids

The invention also comprises certain constructs and nucleic acids encoding the Glut1 protein described herein. Certain constructs and sequences, including selected sequences listed in Table 1 including SEQ ID NOs:28-75, and 80-97, and in certain aspects one or more of SEQ ID NOs: 2-5, 7-9, 11-14, 16-18, 20-23, 25-27, and 80-95 may be useful in embodiments of the present invention. Unexpectedly, as described herein, it has been found that including the nucleic acid sequences encoding the 2A peptide do not express desired levels of Glut1. Thus, preferred rAAV constructs will lack nucleic acids SEQ ID NOs: 6, 15, and/or 24, which all correspond to the 2A encoding sequences.

Preferably, the nucleic acids hybridize under low, moderate or high stringency conditions, and encode a Glutl protein that maintains biological function. A first nucleic acid molecule is "hybridizable" to a second nucleic acid molecule when a single stranded form of the first nucleic acid molecule can anneal to the second nucleic acid molecule under the appropriate conditions of temperature and solution ionic strength (see Sambrook, *et al.,* supra). The conditions of temperature and ionic strength determine the "stringency" of the hybridization. Typical low stringency hybridization conditions include 55°C, 5X SSC, 0.1% SDS and no formamide; or 30% formamide, 5X SSC, 0.5% SDS at 42°C. Typical moderate stringency hybridization conditions are 40% formamide, with 5X or 6X SSC and 0.1% SDS at 42°C. High stringency hybridization conditions are 50% formamide, 5X or 6X SSC at 42°C or, optionally, at a higher temperature (e.g., 57°C, 59°C, 60°C, 62°C, 63°C, 65°C or 68°C). In general, SSC is 0.15M NaCl and 0.015M Na-citrate. Hybridization requires that the two nucleic acids contain complementary sequences, although, depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the higher the stringency under which the nucleic acids may hybridize. For hybrids of greater than 100 nucleotides in length, equations for calculating the melting temperature have been derived (see Sambrook, *et al.,* supra, 9.50-9.51). For hybridization with shorter nucleic acids, *e.g.,* oligonucleotides, the position of mismatches becomes more important, and the length of the oligonucleotide determines its specificity (see Sambrook, *et al.,* supra, 11.7-11.8).

Glutl polypeptides comprising amino acid sequences that are at least about 70% identical, preferably at least about 80% identical, more preferably at least about 90% identical and most preferably at least about 95% identical (e.g., 95%, 96%, 97%, 98%, 99%, 100%) to the mGlut1 or hGlut1 amino acid sequences provided herein (e.g. SEQ ID NO:78 and SEQ ID NO:79) are contemplated with respect to restoring Glut1 function, when the comparison is performed by a BLAST algorithm wherein the parameters of the algorithm are selected to give the largest match between the respective sequences over the entire length of the respective reference sequences. Polypeptides comprising amino acid sequences that are at least about 70% similar, preferably at least about 80% similar, more preferably at least about 90% similar and most preferably at least about 95% similar (e.g., 95%, 96%, 97%, 98%, 99%, 100%) to any of the reference Glut1 amino acid sequences when the comparison is performed with a BLAST algorithm wherein the parameters of the algorithm are selected to give the largest match between the respective sequences over the entire length of the respective reference sequences, are also included in constructs and methods of the present invention.

Sequence identity refers to the degree to which the amino acids of two polypeptides are the same at equivalent positions when the two sequences are optimally aligned. Sequence similarity includes identical residues and nonidentical, biochemically related amino acids. Biochemically related amino acids that share similar properties and may be interchangeable are discussed above.

"Homology" refers to sequence similarity between two polynucleotide sequences or between two polypeptide sequences when they are optimally aligned. When a position in both of the two compared sequences is occupied by the same base or amino acid monomer subunit, e.g., if a position in each of two DNA molecules is occupied by adenine, then the molecules are homologous at that position. The percent of homology is the number of homologous positions shared by the two sequences divided by the total number of positions compared × 100. For example, if 6 of 10 of the positions in two sequences are matched or homologous when the sequences are optimally aligned then the two sequences are 60% homologous. Generally, the comparison is made when two sequences are aligned to give maximum percent homology.

The following references relate to BLAST algorithms often used for sequence analysis: BLAST ALGORITHMS: Altschul, S.F., et al., (1990) J. Mol. Biol. 215:403-410; Gish, W., et al., (1993) Nature Genet. 3:266-272; Madden, T.L., et al., (1996) Meth. Enzymol. 266:131-141; Altschul, S.F., et al., (1997) Nucleic Acids Res. 25:3389-3402; Zhang, J., et al., (1997) Genome Res. 7:649-656; Wootton, J.C., et al., (1993) Comput. Chem. 17:149-163; Hancock, J.M. et a/., (1994) Comput. Appl. Biosci. 10:67-70; ALIGNMENT SCORING SYSTEMS: Dayhoff, M.O., et al., "A model of evolutionary change in proteins." in Atlas of Protein Sequence and Structure, (1978) vol. 5. suppl. 3. M.O. Dayhoff (ed.), pp. 345-352, Natl. Biomed. Res. Found., Washington, DC; Schwartz, R.M., et al., "Matrices for detecting distant relationships." in Atlas of Protein Sequence and Structure, (1978) vol. 5, suppl. 3." M.O. Dayhoff (ed.), pp. 353-358, Natl. Biomed. Res. Found., Washington, DC; Altschul, S.F., (1991) J. Mol. Biol. 219:555-565; States, D.J., et al., (1991) Methods 3:66-70; Henikoff, S., et al., (1992) Proc. Natl. Acad. Sci. USA 89:10915-10919; Altschul, S.F., et al., (1993) J. Mol. Evol. 36:290-300; ALIGNMENT STATISTICS: Karlin, S., et al., (1990) Proc. Natl. Acad. Sci. USA 87:2264-2268; Karlin, S.. et al., (1993) Proc. Natl. Acad. Sci. USA 90:5873-5877; Dembo, A., et al., (1994) Ann. Prob. 22:2022-2039; and Altschul, S.F. "Evaluating the statistical significance of multiple distinct local alignments." in Theoretical and Computational Methods in Genome Research (S. Suhai, ed.), (1997) pp. 1-14, Plenum, New York.

This invention also provides expression vectors comprising various nucleic acids, wherein the nucleic acid is operably linked to control sequences that are recognized by a host cell when the host cell is transfected with the vector. Also provided are the virions comprising recombinant AAV 9 and certain AAV2 sequences, as well as nucleic acid sequences for expressing Glut-1 under the direction of chicken -β-actin promoter and a CMV enhancer. Within these constructs, the rAAV2 sequences correspond to the 5' and 3' ITR sequences, e.g. SEQ ID NOS: 2, 9, 29, 34, 36, 41 and others as described in Table 1). These sequences were packaged with the AAV9 capsid to form the virions that are therapeutic to Glut-1 deficiency in the present invention.

### Pharmaceutical Compositions and Administration

To prepare pharmaceutical or sterile compositions of the compositions of the present invention, the AAV9 vectors or related compositions may be admixed with a pharmaceutically acceptable carrier or excipient. See, *e.g.,* Remington's Pharmaceutical Sciences and U.S. Pharmacopeia: National Formulary, Mack Publishing Company, Easton, PA (1984).

Formulations of therapeutic and diagnostic agents may be prepared by mixing with acceptable carriers, excipients, or stabilizers in the form of, *e.g.,* lyophilized powders, slurries, aqueous solutions or suspensions (see, *e.g.,* Hardman, et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY).

Toxicity and therapeutic efficacy of the therapeutic compositions, administered alone or in combination with another agent, can be determined by standard pharmaceutical procedures in cell cultures or experimental animals, *e.g.,* for determining the LD₅₀ (the dose lethal to 50% of the population) and the ED₅₀ (the dose therapeutically effective in 50% of the population). The dose ratio between toxic and therapeutic effects is the therapeutic index (LD₅₀/ED₅₀). In particular aspects, therapeutic compositions exhibiting high therapeutic indices are desirable. The data obtained from these cell culture assays and animal studies can be used in formulating a range of dosage for use in human. The dosage of such compounds lies preferably within a range of circulating concentrations that include the ED₅₀ with little or no toxicity. The dosage may vary within this range depending upon the dosage form employed and the route of administration.

In an embodiment of the invention, a composition of the invention is administered to a subject in accordance with the Physicians' Desk Reference 2003 (Thomson Healthcare; 57th edition (November 1, 2002)).

The mode of administration can vary. Suitable routes of administration include oral, rectal, transmucosal, intestinal, parenteral; intramuscular, subcutaneous, intradermal, intramedullary, intrathecal, direct intraventricular, intravenous, intraperitoneal, intranasal, intraocular, inhalation, insufflation, topical, cutaneous, transdermal, or intra-arterial.

In particular embodiments, the composition or therapeutic can be administered by an invasive route such as by injection (see above). In further embodiments of the invention, the composition, therapeutic, or pharmaceutical composition thereof, is administered intravenously, subcutaneously, intramuscularly, intraarterially, intra-articularly (e.g. in arthritis joints), intratumorally, or by inhalation, aerosol delivery. Administration by non-invasive routes (*e.g*., orally; for example, in a pill, capsule or tablet) is also within the scope of the present invention.

Compositions can be administered with medical devices known in the art. For example, a pharmaceutical composition of the invention can be administered by injection with a hypodermic needle, including, e.g., a prefilled syringe or autoinjector.

The pharmaceutical compositions of the invention may also be administered with a needleless hypodermic injection device; such as the devices disclosed in U.S. Patent Nos. 6,620,135; 6,096,002; 5,399,163; 5.383.851; 5,312,335; 5,064,413; 4,941,880; 4,790,824 or 4,596,556.

Alternately, one may administer the AAV9 vector or related compound in a local rather than systemic manner. for example, via injection of directly into the desired target site, often in a depot or sustained release formulation. Furthermore, one may administer the composition in a targeted drug delivery system, for example, in a liposome coated with a tissue-specific antibody, targeting, for example, the brain. The liposomes will be targeted to and taken up selectively by the desired tissue.

The administration regimen depends on several factors, including the serum or tissue turnover rate of the therapeutic composition, the level of symptoms, and the accessibility of the target cells in the biological matrix. Preferably, the administration regimen delivers sufficient therapeutic composition to effect improvement in the target disease state, while simultaneously minimizing undesired side effects. Accordingly, the amount of biologic delivered depends in part on the particular therapeutic composition and the severity of the condition being treated.

Determination of the appropriate dose is made by the clinician, e.g., using parameters or factors known or suspected in the art to affect treatment. Generally, the dose begins with an amount somewhat less than the optimum dose and it is increased by small increments thereafter until the desired or optimum effect is achieved relative to any negative side effects. Important diagnostic measures include those of symptoms of, *e.g.,* the inflammation or level of inflammatory cytokines produced. In general, it is desirable that a biologic that will be used is derived from the same species as the animal targeted for treatment, thereby minimizing any immune response to the reagent.

As used herein, "inhibit" or "treat" or "treatment" includes a postponement of development of the symptoms associated with a disorder and/or a reduction in the severity of the symptoms of such disorder. The terms further include ameliorating existing uncontrolled or unwanted symptoms, preventing additional symptoms, and ameliorating or preventing the underlying causes of such symptoms. Thus, the terms denote that a beneficial result has been conferred on a vertebrate subject with a disorder, disease or symptom, or with the potential to develop such a disorder, disease or symptom.

As used herein, the terms "therapeutically effective amount", "therapeutically effective dose" and "effective amount" refer to an amount of a rAAV9-Glutl based compound of the invention that, when administered alone or in combination with an additional therapeutic agent to a cell, tissue, or subject, is effective to cause a measurable improvement in one or more symptoms of a disease or condition or the progression of such disease or condition. A therapeutically effective dose further refers to that amount of the compound sufficient to result at least partial amelioration of symptoms, *e.g*., treatment, healing, prevention or amelioration of the relevant medical condition, or an increase in rate of treatment, healing, prevention or amelioration of such conditions. When applied to an individual active ingredient administered alone, a therapeutically effective dose refers to that ingredient alone. When applied to a combination, a therapeutically effective dose refers to combined amounts of the active ingredients that result in the therapeutic effect, whether administered in combination, serially or simultaneously. An effective amount of a therapeutic will result in an improvement of a diagnostic measure or parameter by at least 10%; usually by at least 20%; preferably at least about 30%; more preferably at least 40%, and most preferably by at least 50%. An effective amount can also result in an improvement in a subjective measure in cases where subjective measures are used to assess disease severity.

### Kits

The present invention also provides kits comprising the components of the combinations of the invention in kit form. A kit of the present invention includes one or more components including, but not limited to, rAAV9-Glut1 based compound, as discussed herein, in association with one or more additional components including, but not limited to a pharmaceutically acceptable carrier and/or a chemotherapeutic agent, as discussed herein. The rAAV9-Glut1 based compound or composition and/or the therapeutic agent can be formulated as a pure composition or in combination with a pharmaceutically acceptable carrier, in a pharmaceutical composition.

In one embodiment, a kit includes an rAAV9-Glut1 based compound/composition of the invention or a pharmaceutical composition thereof in one container (*e.g.*, in a sterile glass or plastic vial) and a pharmaceutical composition thereof and/or a chemotherapeutic agent in another container (*e.g.*, in a sterile glass or plastic vial).

In another embodiment of the invention, the kit comprises a combination of the invention, including an rAAV9-Glutl based compound, along with a pharmaceutically acceptable carrier, optionally in combination with one or more chemotherapeutic agent component formulated together, optionally, in a pharmaceutical composition, in a single, common container.

If the kit includes a pharmaceutical composition for parenteral administration to a subject, the kit can include a device for performing such administration. For example, the kit can include one or more hypodermic needles or other injection devices as discussed above.

The kit can include a package insert including information concerning the pharmaceutical compositions and dosage forms in the kit. Generally, such information aids patients and physicians in using the enclosed pharmaceutical compositions and dosage forms effectively and safely. For example, the following information regarding a combination of the invention may be supplied in the insert: pharmacokinetics, pharmacodynamics, clinical studies, efficacy parameters, indications and usage, contraindications, warnings, precautions, adverse reactions, overdosage, proper dosage and administration, how supplied, proper storage conditions, references, manufacturer/distributor information and patent information.

### GENERAL METHODS

Standard methods in molecular biology are described Sambrook, Fritsch and Maniatis (1982 & 1989 2nd Edition, 2001 3rd Edition) Molecular Cloning, A Laboratory Manual, Cold Spring Harbor Laboratory Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA). Standard methods also appear in Ausbel, et al. (2001) Current Protocols in Molecular Biology, Vols.1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4).

Methods for protein purification including immunoprecipitation, chromatography, electrophoresis, centrifugation, and crystallization are described (Coligan, et al. (2000) Current Protocols in Protein Science, Vol. 1, John Wiley and Sons, Inc., New York). Chemical analysis, chemical modification, post-translational modification, production of fusion proteins, glycosylation of proteins are described (see, *e.g.,* Coligan, et al. (2000) Current Protocols in Protein Science, Vol. 2, John Wiley and Sons, Inc., New York; Ausubel, et al. (2001) Current Protocols in Molecular Biology, Vol. 3, John Wiley and Sons, Inc., NY, NY, pp. 16.0.5-16.22.17; Sigma-Aldrich, Co. (2001) Products for Life Science Research, St. Louis, MO; pp. 45-89; Amersham Pharmacia Biotech (2001) BioDirectory, Piscataway, N.J., pp. 384-391). Production, purification, and fragmentation of polyclonal and monoclonal antibodies are described (Coligan, et al. (2001) Current Protcols in Immunology, Vol. 1, John Wiley and Sons, Inc., New York; Harlow and Lane (1999) Using Antibodies, Cold Spring Harbor Laboratory Press, Cold Spring Harbor, NY; Harlow and Lane, *supra*). Standard techniques for characterizing ligand/receptor interactions are available (see, *e.g.,* Coligan, et al. (2001) Current Protocols in Immunology, Vol. 4, John Wiley, Inc., New York).

### Abbreviations

- AAV:: adeno-associated virus
- rAAV: recombinant adeno-associated virus or viral vector
- BBB:: blood brain barrier
- FMDV:: foot and mouth disease virus
- GFP:: green fluorescent protein
- Glut1:: Glucose transporter 1, also known as solute carrier family 2, facilitated glucose transporter member 1 (SLC2A1), is a uniporter protein that in humans is encoded by the *SLC2A1* gene. Glut1 facilitates the transport of glucose across the plasma membranes of mammalian cells. Glutl was the first glucose transporter to be characterized. Glut1 1 is highly conserved with the human Glutl protein (hGlut1) (Accession No: NP_006507.2; SEQ ID NO:79) and mouse Glut1 protein (mGlut1) (Accession No: NP_035530.2; SEQ ID NO:78) sharing 98 % homology. Glut1 exhibits 40% homology with other Gluts.
- *SLC2A1:*: Gene encoding the human glucose transporter 1 (hGlut1). Human *SLCZA1* (Accession No: NG_008232.1; gene ID - 6513).
- *Slc2a1:*: Gene encoding mouse Glut1 (mGlut1) (Accession No: Genomic #: NC_000070.6; gene ID - 20525).
- GLUT1 DS:: Glutl deficiency syndrome
- PND:: post-natal day
- PND3:: post-natal day 3

### EXAMPLES

**Table 1-Recombinant Glut1 plasmids**

| **EGFP-2A-Glut1 Constructs** | | |
|---|---|---|
| Human | pAAV CB6 PI EGFP-2A-hGIut1 (SEQ ID NO: 1) | Did not express hGlut1 1 |
| | Key Features of Construct 5'ITR (SEQ ID NO: 2) | |
| | CMV IE enhancer (SEQ ID NO:3) | |
| | CB promoter (SEQ ID NO: 4) | |
| | eGFP (SEQ ID NO: 5) | |
| | 2A-linker (SEQ ID NO: 6) | |
| | hGlut1 cDNA and 3'UTR (SEQ ID NO: 7) | |
| | Poly A signal (SEQ ID NO: 8) | |
| | 3' ITR (SEQ ID NO: 9) | |
| mouse | pAAV CB6 PI EGFP-2A-mGlut1 (SEQ ID NO: 10) | Did not express mGlut1 |
| | Key Features of Construct 5'ITR (SEQ ID NO: 11) | |
| | CMV IE enhancer (SEQ ID NO: 12) | |
| | CB promoter (SEQ ID NO: 13) | |
| | eGFP (SEQ ID NO: 14) | |
| | 2A-linker (SEQ ID NO: 15) | |
| | mGlut1 cDNA and 3'UTR (SEQ ID NO: 16) | |
| | Poly A signal (SEQ ID NO: 17) | |
| | 3' ITR (SEQ ID NO: 18) | |

| **Glut1-2A-EGFP Constructs** | | |
|---|---|---|
| human | pAAV CB6 PI hGIut1-2A-EGFP (SEQ ID NO: 19) | Did not express hGlut1 |
| | Key Features of Construct 5'ITR (SEQ ID NO: 20) | |
| | CMV IE enhancer (SEQ ID NO: 21) | |
| | CB promoter (SEQ ID NO: 22) | |
| | hGlut1 cDNA (SEQ ID NO: 23) | |
| | 2A-linker (SEQ ID NO: 24) | |
| | eGFP (SEQ ID NO: 25) | |
| | Poly A signal (SEQ ID NO: 26) | |
| | 3' ITR (SEQ ID NO: 27) | |
| mouse | *pAA V CB6 PI mGlut1-2A-EGFP SEQ ID NO:88* | Did not express mGlut1 |
| | Key Features of Construct 5'ITR (SEQ ID NO: 89) | |
| | CMV IE enhancer (SEQ ID NO:90 ) | |
| | CB promoter (SEQ ID NO:91) | |
| | eGFP (SEQ ID NO:92) | |
| | mGlut1 cDNA and 3'UTR (SEQ ID NO: 93) | |
| | Poly A signal (SEQ ID NO: 94) | |
| | 3' ITR (SEQ ID NO: 95) | |
| **Native Glut1 Constructs** | | |
| human | pAAV9-CB6 PI hGlut1 pAAV CB6 PI hGlut1 (SEQ ID NO: 28) | **Expresses hGlut1** |
| | Key Features of Construct 5'ITR (SEQ ID NO: 29) | |
| | CMV IE enhancer (SEQ ID NO: 30) | |
| | CB promoter (SEQ ID NO: 31) | |
| | hGlut1 cDNA (SEQ ID NO: 32) | |
| | Poly A signal (SEQ ID NO: 33) | |
| | 3' ITR (SEQ ID NO: 34) | |
| mouse | pAAV9-CB6 PI mGlut1 pAAV CB6 PI mGlut1 (SEQ ID NO: 35) | **Expresses mGlut1** |
| | Key Features of Construct | |
| | 5'ITR (SEQ ID NO: 36) | |
| | CMV IE enhancer (SEQ ID NO:37) | |
| | CB promoter (SEQ ID NO: 38) | |
| | mGlut1 cDNA (SEQ ID NO: 39) | |
| | Poly A signal (SEQ ID NO: 40) | |
| | 3' ITR (SEQ ID NO: 41) | |

| **Glut1-mir122** Constructs | | |
|---|---|---|
| human | pAAV CB6 PI hGlut1-out3xmiR-122 BS (SEQ ID NO: 42) | hGlut1 expression TBD |
| | Key Features of Construct 5'ITR (SEQ ID NO: 43) | |
| | CMV IE enhancer (SEQ ID NO: 44) | |
| | CB promoter (SEQ ID NO: 45) | |
| | hGlut1 cDNA (SEQ ID NO: 46) | |
| | 3'UTR (SEQ ID NO: 47) | |
| | 3xmiR-122 BS (SEQ ID NO: 48) | |
| | Poly A signal (SEQ ID NO: 49) | |
| | 3' ITR (SEQ ID NO: 50) | |
| Human | pAAV CB6 PI hGlut1-in3xmiR-122 BS (SEQ ID NO: 51) | hGlut1 expression TBD |
| | Key Features of Construct | |
| | 5'ITR (SEQ ID NO: 52) | |
| | CMV IE enhancer (SEQ ID NO: 53) | |
| | CB promoter (SEQ ID NO: 54) | |
| | hGlut1 cDNA (SEQ ID NO: 55) | |
| | 3' UTR and 3xmiR-122 (SEQ ID NO: 56) | |
| | Poly A signal (SEQ ID NO: 57) | |
| | 3' ITR (SEQ ID NO: 58) | |
| mouse | pAAV CB6 PI mGlut1-in3xmiR-122 BS (SEQ ID NO: 59) | mGlut1 expression TBD |
| | Key Features of Construct 5'ITR (SEQ ID NO: 60) | |
| | CMV IE enhancer (SEQ ID NO: 61) | |
| | CB promoter (SEQ ID NO: 62) | |
| | mGlut1 cDNA (SEQ ID NO: 63) | |
| | 3'UTR and 3x-miR122BS (SEQ ID NO: 64) | |
| | Poly A signal (SEQ ID NO: 65) | |
| | 3'ITR (SEQ ID NO: 66) | |
| mouse | pAAV CB6 PI mGlut1-out3xmiR-122 BS (SEQ ID NO: 67) | mGlut1 expression TBD |
| | Key Features of Construct 5'ITR (SEQ ID NO: 68) | |
| | CMV IE enhancer (SEQ ID NO: 69) | |
| | CB promoter (SEQ ID NO: 70) | |
| | mGlut1 cDNA (SEQ ID NO: 71) | |
| | 3'UTR (SEQ ID NO: 72) | |
| | 3xmiR-1 22BS (SEQ ID NO: 73) | |
| | Poly A signal (SEQ ID NO: 74) | |
| | 3'ITR (SEQ ID NO: 75) | |
| Human EGFP construct | | |
| | *pAAV CB6 PI hGlut1-EGFP SEQ ID NO:80* | Expresses hGlut 1 |
| | Key Features of Construct 5'ITR (SEQ ID NO: 81) | |
| | CMV IE enhancer (SEQ ID NO: 82) | |
| | CB promoter (SEQ ID NO: 83) | |
| | eGFP (SEQ ID NO: 84) | |
| | mGlut1 cDNA (SEQ ID NO: 85) | |
| | Poly A signal (SEQ ID NO: 86) | |
| | 3' ITR (SEQ ID NO: 87) | |

### Recombinant AAV Construct Development

Four DNA constructs were generated carrying either the murine or human *SLC2A1* gene linked to the nucleotide cassette encoding green fluorescent protein (GFP) reporter as shown in Figs. 1A-B. These four constructs also contain a nucleic acid sequence encoding a 16 amino-acid long 2A peptide from foot and mouth disease virus (FMDV) incorporated between the Glutl and GFP open reading frames. The 2A peptide is included in the construct to circumvent the possibility that Glutl-GFP fusion proteins might alter the structure or activity of the Glut1 protein. The 2A peptide mediates the primary cis-'cleavage' of the FMDV polyprotein in a cascade of processing events that ultimately generate the mature FMDV proteins (Donnelly, M.L. *et al.* (2001)). This strategy was expected to create constructs in which the Glut1 protein is generated in its native state. However, as described below, none of these constructs expressed Glut1 at satisfactory levels.

Six additional DNA constructs were also developed without the nucleic acid encoding the 2A peptide (Fig. 1C-D), several of which include elements which provide that the expression of the *SLC2A1* gene is selectively turned off in the liver (Fig. 1D). These constructs were developed to address the possibility that systemically augmenting the *SLC2A1* gene in future gene therapy experiments could result in high levels of the protein being expressed in the liver which could increase the process of glycogenesis and thus induce a hypoglycemic state. Finally, constructs exclusively containing the native mouse or human *SLC2A1* gene have also been generated for control purposes (Fig. 1C). pAAV9 CB6 PI hGlut1, pAAV9 CB6 PI hGlut1 out3xmiR122BS, and pAAV9 CB6 PI hGlut1 in3xmiR122BS will be utilized in validating experiments. The sequences and key features of these constructs are listed in Table 1 and in the corresponding sequence listing SEQ ID NOs:1-97.

### Initial Expression Analysis of rAAV plasmids transfected into CHO cells

Two cell lines were used to evaluate the various recombinant plasmids in cell culture. One cell line is a Chinese hamster ovary (CHO) line, the other is a fibroblast line derived from a Glutl patient (Yang et al. 2011). Cell culture experiments and subsequent Western blots indicated that plasmid constructs containing the 2A peptide expressed neither Glut1 nor GFP at satisfactory levels. While unexpected, it is possible that in the context of the *SLC2A1*/*slc2A1* gene, the 2A peptide adversely affects the expression of the protein. In contrast, the control constructs containing only the mouse or human *SLC2A1* genes were found to express robust levels of protein. None of the 2A containing Glut1 constructs (Fig.1A-B) were pursued further for restoring Glutl expression in mutant model systems.

To circumvent the expression difficulties introduced by the presence of the 2A peptide in the first four constructs (Fig. 1A-B), an hGlut1-eGFP fusion protein (referred to as phGlutl::eGFP) was tested. Cell culture experiments indicated that the fusion protein is not only expressed but is also functional in the glucose uptake assay. This construct (phGlut1::eGFP), along with constructs containing just the mouse *slc2a1* or human *SLC2A1* genes are contemplated for use in *in vivo* experiments involving gene therapy of the Glutl model mice.

### AAV9 plasmid cloning and subsequent viral vector packaging

The hGlut1-eGFP fusion protein construct (phGlut1::eGFP) was re-cloned into the AAV9 plasmid for subsequent packaging into the viral vector. To ensure that the re-cloned construct continued to express protein, it was transiently transfected it into Chinese hamster ovary (CHO) cells and protein levels were examined by western blot analysis (Fig. 2A). Analysis of protein expression in the CHO cells showed that relative to constructs expressing just the hGlut1 encoding cDNA or hGlut-eGFP fusion driven by a different promoter element, the AAV9-hGlutl-eGFP plasmid expressed lower levels of Glutl (Fig. 2A). However, the modified (AAV9-hGlut1-eGFP) fusion construct continued to express the Glut1 protein in an effective and satisfactory amount. Furthermore, the fusion protein appeared to be significantly larger than the native hGlut1 protein, as expected due to the GFP tag at the 3' end of the Glut1 cDNA (Fig. 2A). These results are consistent with glucose uptake assays in which the hGlut-eGFP protein was found to increase uptake of glucose into CHO cells (Fig. 2B). Fig. 2C shows GFP fluorescence of this construct following transfection into CHO cells. In parallel, the human *SLC2A1* and mouse *slc2a1* genes were also cloned into the AAV9 plasmid and, upon transfection into patient fibroblasts, found to drive Glut1 expression and increase glucose uptake. Accordingly, each was packaged into the AAV9 capsid and ∼10¹³ genome copies prepared for administration into Glutl DS model mice. (According to methods as described in U.S. Patent No. 8,734,809, and in Grieger and Samulski 2005 and Grieger and Samulski 2012).

### Packaging Conditions/Distribution

To optimize conditions for the administration of Glut1 expressing constructs packaged in AAV9 vectors, the distribution of an AAV9-GFP (Foust, K.D. et al. 2009) vector in wild-type mice was analyzed. (According to methods as described in Gao, G.P., and Sena-Esteves, M. (2012), In Molecular Cloning, Vol 2: A Laboratory Manual (M.R. Green and J. Sambrook eds.)).

The distribution of the AAV9-GFP construct was evaluated in different tissues in wild-type adult or neonatal mice. Bright green fluorescence was found in the tested tissues of AAV9-GFP injected mice, but not in PBS/control injected mice. Essentially, ∼4 x 10¹² genome copies of the vector were administered systemically in a volume of ∼40µl into the mice through the retro-orbital sinus and temporal vein. Results from these experiments indicate that the AAV9 virus distributes into a variety of nervous and non-nervous tissue. In particular, high levels were found to target skeletal muscle, heart and liver. However, substantial GFP fluorescence was seen in brain tissue, including in Glut1-positive endothelial cells lining the brain micro-vasculature. Importantly, these cells are the putative sites of a targeted therapy for Glut1 DS.

### Control AAV9-mGlut1 constructs

### Table 2 -

mGlut1 = pAAV9 CB6 PI mGlut1 (SEQ ID NO:35-41).

**Table 2 - Summary of Glut1 or vehicle injected mice**

| **No. of animals** | **Gender** | **Date of Injection** | **Injected with** |
|---|---|---|---|
| 4 | Female | 5/19 | mGlut1 |
| 1 | Male | 5/15 | mGlut1 |
| 2 | Male | 5/25 | mGlut1 |
| 2 | Female | 5/25 | mGblut1 |
| 1 | Female | 5/25 | PBS |
| 1 | Male | 5/31 | PBS |
| 3 | Male | 6/1 | PES |
| 2 | Male | 6/6 | PBS |

One of the constructs that expressed desired amounts of the mGlut1 protein was packaged into the AAV9 viral vector. Even though this construct does not have a labeled tag (e.g., GFP), the Glut1 expression from this construct in the model mice can be followed by assessing total mGlut1 protein by Western blot analysis and immunohistochemistry experiments.

Construct pAAV9 CB6 PI mGlut1 (SEQ ID NO:35) was introduced into postnatal day (PND) 2 mutant Glutl mouse pups through the retro-orbital sinus. Mice injected with this construct serve as controls for the AAV9-hGlut1-eGFP injected mutants (SEQ ID NO: 80; See features SEQ ID NO:81-87). Nine mutant mice have been injected with the AAV9-mGlut1 construct pAAV9 CB6 PI mGlut1 (Table 2). As additional controls, seven mutants have been injected with vehicle (PBS) alone. These mice will be tested for functional improvement of the disease phenotype. This will be carried out by determining levels of glucose uptake in brain tissue, by PET scans, and by measuring motor performance on the rotarod or vertical pole tests according to standard techniques *(See* Kariya et al, 2012).

One of the constructs used in the gene replacement experiments with the Glut1DS model mice is the AAV9-hGlutl-eGFP construct (SEQ ID NO:80). The tagged Glut1 protein produced from this construct will allow the distribution of the protein in the various organ systems to be followed, including Glut1-expressing endothelial cells of the brains of the experimental mice. This will allow for optimizing conditions for the detection of mGlut1 in the mouse brain. Robust expression of the mGlut1 protein in the brain is detectable using a specific antibody.

### Restoring Glut1 to Glut1 DS mutant mice rescues the disease phenotype, as exemplified by rescuing gait dysfunction

The ability of recombinant adeno-associated virus 9 (rAAV9) to infect multiple cell types was utilized as a feature to re-introduce the murine *Slc2a1* gene into a mouse model of the human disease. In the absence of one wild-type *Slc2a1* allele, Glutl DS mice perform poorly in the rotarod assay, an outcome measure believed to model motor behavior defects, i.e. motor phenotypes, observed in human patients. The mutant mice were either injected with ∼ 4 x 10¹¹ genome copies of AAV9-Glut1 provided by construct pAAV9 CB6 PI mGlutl(SEQ ID NO:35), or vehicle (PBS) at PND3. P values calculated using one-way ANOVA. Restoring the *Slc2a1* gene into mutant mice at PND3 resulted in a significant improvement in performance on the rotarod (carried out under standard conditions--*See* Wang et al. 2006), as early as 6 weeks of age (Fig. 3A). The enhanced performance persisted until 20 weeks of age, at which point the experiment was terminated.

In addition to the improved performance on the rotarod assay (Fig.3A), the treated mice also negotiate a vertical pole with greater agility than do their vehicle treated counterparts (Fig. 3B). The treated mutants performed indistinguishably from the wild-type control littermates when the cohorts were tested between 6 and 12 weeks of age. These results provide strong evidence that restoring Glut1 to Glut1 DS mice mitigates the motor phenotype characteristics of the human disease, indicating that restoring the *Slc2a1* gene to the mutant model mice is indicative of therapeutic value.

### Restoring Glut1 to Glut1 DS mutant mice results in enhanced expression of the gene in multiple tissues.

To explore the molecular basis of the improved performance of the AAV9-Glut1 treated animals, the expression of the murine *Slc2a1* gene in brain and liver tissue of the animals was assessed. Mutant animals treated with the pAAV9 CB6 PI mGlut1 (SEQ ID NO:35) pressed greater levels of the *Slc2a1* gene in brain and liver tissue (Figs. 4A-B). Brain and liver tissue was extracted from treated and control mice, RNA prepared and then reverse-transcribed before amplifying the *Slc2a1* transcript in a Q-PCR assay. β-actin was used to normalize *Slc2a1* gene expression. Fig. 4A shows relative *Slc2a1* gene expression in treated mutants and the relevant controls. Fig. 4B shows *Slc2a1* expression as a percent of expression in the wild-type Glut1^{+/+} mice. Primers spanning intron 1 were used to amplify the Glut1 encoding transcript by quantitative PCR in treated mutants (Glut1^{+/-}) and relevant controls (Glut1^{+/+} and PBS treated Glut1^{+/-} mutants). Mice were euthanized and tissues extracted following transcardial perfusion with PBS. RNA was prepared using the Qiagen RNAeasy kit as per the manufacturer's instructions (Qiagen, Valencia, CA). The RNA was reverse transcribed according to standard procedures and the following primers used to amplify the Glut1 encoding transcript: Glut1QPCR F1: 5' CTT GCT TGT AGA GTG ACG ATC 3' (SEQ ID NO:76) and Glut1QPCR R1: 5' CAG TGA TCC GAG CAC TGC TC 3' (SEQ ID NO:77). The expected 212bp band was quantified in an Eppendorf Realplex Cycler (Eppendorf, Germany).

Unexpectedly, expression of the gene in treated mutant liver exceeded levels in the same tissue of Glut1^{+/+} controls, consistent with prior reports (Foust et al, 2010) that the AAV9-Glut1 virus has a particular tropism for liver. In a small cohort of WT mice administered virus, this also led to hypoglycemia, likely a consequence of *Slc2a1* upregulation in this tissue and therefore removal of glucose from the blood. Accordingly, suppression of expression of *Slc2a1* is contemplated using constructs containing miRNA-122 binding sites (as shown in Fig. 1D and encompassed by SEQ ID NOs:42-75). miRNA-122 is specifically expressed in liver and suppresses expression of genes whose transcripts it binds (Xie et al, 2011). The physiological consequences of this finding will be the subject of additional investigation.

### Enhanced Glut1 brain protein and CSF glucose in mutant mice treated with AAV9-Glut1; Restoring Glutl mitigates hypoglycorrachia in Glutl DS model mice.

A defining feature of Glut1 DS is hypoglycorrhachia (low cerebrospinal fluid glucose). Glut1 DS model mice exhibit this phenotype. To determine if restoring Glut1 to model mice reversed or mitigated the hypoglycorrhachia, blood and cerebrospinal fluid (CSF) were extracted from the animals and glucose levels measured. All mice were fasted overnight before measurements were made. CSF was isolated from the cisterna magna essentially as previously described (Wang *et al*., 2006; Fleming *et al*., 1983). Briefly, an incision was made from the top of the skull to the dorsal thorax, and the musculature from the base of the skull to the first vertebrae removed to expose the meninges overlying the cisterna magna. The tissue above the cisterna magna was excised taking care not to puncture the translucent meninges. Once the surrounding area was cleaned of residual blood/interstitial fluid, a micropipette attached to a 30G needle was used to puncture the arachnoid membrane covering the cisterna magna and draw out 5-15µl of CSF. The entire procedure was completed in 5 minutes and CSF glucose measured with an Ascensia Elite XL glucose meter (Bayer Corp.) Blood glucose was similarly determined, prior to CSF extraction, by drawing ∼10µl of blood from an incision in the tail. Two readings each of the blood and CSF glucose concentrations for each mouse were assessed. The mean value will be reported.

With respect to the CSF glucose values and disease stages, the following ranges are typical: over 90% of Glut1 patients have CSF glucose values of < 40 mg/dl (2.2 mM) and the remaining patients fall in the range of 41-52 mg/dl. Thus, the normal range for CSF glucose levels is ≥ about 53 mg/dl. For the Glut1 DS model mice, the typical CSF glucose level is about 23.3±7.17 mg/dL (falling within a range of < 25.0 ±8.00 mg/dl); while for wild-type mice the level is about 74.6 ±14.1 mg/dL (falling within a range of ≥ about 70.0 ±15.0 mg/dL).

Additionally, the RBC glucose uptake function assay is often used as a surrogate for Glutl haploinsufficiency. In this assay, patient samples exhibiting Glutl DS cluster around 50% uptake, with a range of 36-73%. It is estimated that ≥75% activity is consistent with a normal range. It is noted that <25% is severe and approaching embryonic lethality at 0%.

Restoring the *slc2a1* gene to Glut1 DS mutant mice by transfection with the construct pAAV9 CB6 PI mGlut1 (SEQ ID NO:35) results in an increased expression of the Glut1 (Fig. 5A). Additionally, the treated Glutl DS mutant mice express increased levels of the Glut1 protein in brain tissue (Fig. 5B). In Fig. 5C, the CSF glucose concentrations in treated mutants were significantly greater than that of untreated mutants, but did not reach levels observed in wild-type controls. The restored *slc2a1* mutant mice exhibited increased levels of CSF/blood glucose (Fig. 5D). The sample sizes are n=8 for the untreated mutant mice and n=9 for the treated mutant mice. Additionally, the wild-type cohort is n=18. These data show that restoring Glut1 to Glut1 DS mutants mice increases CSF glucose levels and mitigates the hypoglycorrhachia of affected animals. Collectively, these results are a clear indication of the therapeutic benefits of restoring Glut1 in a Glut1 deficient subject. Preliminary results from these experiments indicate that restoring Glutl to these symptomatic, adult mice fails to rescue the disease phenotype arguing for a limited therapeutic window of opportunity in mice and, likely humans too.

### Restoring Glut1 to symptomatic mice-timing of Glut1 administration.

Restoration of Glut1 expression to model mice early during the course of the disease (exemplified by the PN3 injection of AAV9-mGlut1 constructs) in Glut1 DS mice has clear therapeutic value. To determine the timeframe of Glut1 restoration in symptomatic mice, experiments that involve injecting pAAV CB6 PI mGlut1 constructs (SEQ ID NO:35) into mutant mice at 8 weeks of age have been initiated. The Glut1 DS mice are clearly symptomatic at this point performing less well than control, wild-type littermates on the rotarod. Accordingly, a cohort of Glutl DS mice were systemically injected with either vehicle or 1 x 10¹² genome copies of AAV9-mGlut1. All of the mice tolerated the procedure indicating that virus injection in adult rodents is safe. Molecular, cellular and behavioral assays similar to those described above are being evaluated to determine time frames that will allow for treating/alleviating symptoms of Glut1 deficiency, as well as any time limits for reversing the course of the disease phenotype.

### Refining the therapeutic window of opportunity in a model of Glut1 DS

The present data demonstrate that AAV9-mediated repletion of the Glut1 (murine) protein in ***neonatal*** (PND3) Glut1 DS mice increases Glut1 expression, mitigates the hypoglycorrhachia characteristically observed in the disease, restores brain size and results in a marked improvement in motor performance. In contrast, ***Glut1 repletion at 8 weeks of age failed*** to rescue the disease phenotype. These results suggest that there is a limited therapeutic window of opportunity in Glut1 DS model mice, a finding that is likely to be reflective of the human condition. Preliminary data also indicates significantly lowered CSF glucose levels in mutant mice as early as 2 weeks of age (Mutants: 23.25±3.77mg/dL; Ctrls: 53.33±5.20mg/dL, *P <* 0.01, *t* test). Yet, it is unclear if restoring Glutl at this juncture, prior to a discernible overt phenotype, will provide therapeutic benefit.

To determine the outcome of restoring Glut1 at this early stage of the disease - *akin to treating patients that have been diagnosed in childhood but nevertheless been subject to the disease-causing effects of Glut1 deficiency during infancy,* mutant mice will be systemically transduced with the pAAV9 CB6 PI mGlut1 vector (SEQ ID NO:35). Briefly, ∼10¹² genome copies of the therapeutic vector of vehicle in a ∼50µl volume will be injected into the temporal vein of 2-week old mice. The animals will subsequently be assessed using a comprehensive battery of molecular (western blot analysis, Q-PCR assays, CSF and blood glucose levels), imaging (PET scans) and behavioral (rotarod analyses, vertical pole tests) assays to determine the outcome of restoring the functional protein at this "juvenile" stage in mice. These experiments will complement results obtained following treatment in neonates (PND3) on the one hand and in the adult model (8 weeks) on the other, and refine the therapeutic window of opportunity for Glutl DS.

### Assessing the combined effects of early treatment with the ketogenic diet and late repletion of Glut1 protein in Glut1 DS model mice

While it is clear that restoring Glutl to adult mice (8-weeks) did not mitigate the Glut1 DS phenotype, it is possible that prior treatment of these mice with a high-fat diet might have produced a more favorable outcome. Mice on such diets more accurately represent the cohort of older Glutl DS patients who may have missed the ideal therapeutic window of treatment but might nevertheless benefit from a late restoration of the Glut1 protein owing to the early protective effects of a ketogenic diet. Such diets supply the brain with ketone bodies, an alternate, albeit imperfect, source of energy that traverses the blood-brain barrier via mono-carboxylic transporters. Accordingly, in addition to our experiments in two-week old mice, we will test the effects of restoring Glut1 to adult (6-8 weeks) mutants that have received (beginning at PND7) the 7C triglyceride triheptanoin. Triheptanoin, currently in clinical trials for Glut1 DS, is not only metabolized to acetyl CoA for the TCA cycle, but is also thought to provide essential anaplerotic substrates for the cycle as nutrients are eventually broken down to supply the cell's energy requirements. In brief, this experiment will involve treating mutants with (82mg/g) or without triheptanoin until they are administered the AAV9-Glutl (pAAV9 CB6 PI mGlut1) construct. The different cohorts of mice will then be assessed as described above as a means of predicting the therapeutic outcome of restoring Glut1 expression in older patients on currently available (ketogenic diet) treatments.

### Optimization of Glut1 constructs for clinical trials

Although no untoward effects of *slc2a1* expression in Glut1 DS mice have been observed to date, preliminary studies on a small sample of wild-type mice administered the construct pAAV9 CB6 PI mGlut1 vector (SEQ ID NO:35) indicated lowered CSF and blood glucose levels. This unexpected event could result from increased *slc2a1* expression in liver, a preferred AAV9 target organ, and consequently enhanced transport of glucose into this tissue. The net result is a fall in circulating glucose which is reflected in a hypoglycemic state. In anticipation of such an event, the hGlut1 construct (pAAV CB6 PI hGlut1) will be modified to preclude its expression at high levels in liver - an organ for which AAV9 has a particularly high tropism (Zincarelli et al, 2008; Pacak et al, 2006). To do so, binding sites (BS) for miRNA - miR-122 (expressed specifically in hepatocytes) will be introduced into constructs. This strategy has been successfully implemented previously (*See* Xie et al, 2011) and takes advantage of miRNA-mediated endonucleolytic cleavage of target mRNAs, thus restricting the expression of the transcript to tissues of interest. Such constructs shown in Fig. 1 D and Table 1 (pAAV CB6 PI hGlut1-in3xmiR-122 BS, pAAV CB6 PI hGlut1-out3xmiR-122 BS, pAAV CB6 PI mGlut1-in3xmiR-122, pAAV CB6 PI mGlut1-out3xmiR-122) will be tested in a subset of mice side-by-side with the original (unmodified) Glut1 expressing vectors, examining each for Glut1 expression levels and therapeutic efficacy. An increased tendency of the original construct to cause hypoglycemia will indicate the benefit of using the new Glut1-miRNA-BS constructs in subsequent experiments and trials.

To optimize expression of the test constructs described herein not just as a means of reducing viral titers during the manufacturing process, but also to address safety concerns associated with large concentrations of the virus, the *SLC2A1* and *slc2a1* genes will be evaluated using a codon optimization process using freely available software (https://www.idtdna.com/CodonOpt). In addition, consensus Kozak sequences will be introduced into constructs as needed. Thus, any of the constructs or elements described in Table 1 may be codon optimized in this manner. Each of the modified constructs will be tested in parallel with the parental constructs in mice. Briefly, the constructs will be systemically administered through the temporal vein into PND3 mouse pups. The animals will then be euthanized either two or three weeks later and levels of protein from each of the constructs determined by Q-PCR and western blotting. Constructs delivering the most rapid and high levels of expression will be considered for eventual use in non-human primate studies and eventually in clinical trials for human patients.

### Non-human primate studies

To determine the bio-distribution, expression and toxicity of our selected construct(s) in a large mammal model, viral vector/s will be administered to a cohort of cynomolgus monkeys. Briefly, 6 animals each at PND1, PND90 and 2 years of age will be systemically administered the AAV9 vector at a dose of 5 x 10¹³ genome copies/kg. To determine acute toxicity of the construct(s), animals will be bled 1 day, 3 days and 7 days after vector administration. Additionally, the animals will be bled 2, 3 and 4 weeks after vector injection. In every case, important clinical chemistry and hematology parameters will be assessed. Titers of neutralizing antibodies to AAV9 will be monitored in serum samples by means of a transduction-based quantitative neutralizing antibody assay (Rapti et al, 2012) and determine the presence of transgene or capsid specific T cells in PBMCs using ELISPOT, intracellular cytokine staining techniques and flow cytometry (Walker et al, 2001). To complement the immunologic studies above, three animals from each cohort will be euthanized at week 4 for histopathology, vector bio-distribution studies and transgene expression analyses in all of the major organ systems. These experiments will also enable examination and comparison of B and T cell immune responses to capsid or transgene in serum, lymphocytes and PBMCs at an early versus late time following virus administration. In order to carry out a long-term safety study, the remaining 3 animals in each group will be followed over a 3 month period during which they will be bled every month for clinical chemistry and hematology studies as described above. These animals will eventually be euthanized 3 months after injections and analyzed as described in the acute toxicity studies. It is possible that the human Glutl protein despite sharing ∼99% homology with cynomolgus Glut1 elicits an aggressive immune response. To preclude this, two miRNA binding sites for miR-142-3p and miR-155 will be introduced into the test constructs. Preliminary results from an independent study indicate that these miRNAs are expressed in antigen presenting cells and, consequently, suppress the expression of proteins whose transcripts contain the binding sites for the miRNAs. Collectively, these studies will facilitate eventual use of the test Glut1 constructs described herein in human clinical trials.

### References

1. De Vivo DC, Trifiletti RR, Jacobson RI, Ronen GM, Behmand RA, Harik SI. (1991). Defective glucose transport across the blood-brain barrier as a cause of persistent hypoglycorrhachia, seizures, and developmental delay. N. Engl. J. Med., 325, 703-709.
2. Donnelly, M.L. et al. (2001). The 'cleavage' activities of foot-and-mouth disease virus 2A site directed mutants and naturally occurring '2A-like' sequences. J. Gen. Virol. 82, 1027-1041.
3. Foust, K.D., Nurre, E., Montgomery, C.L., Hernandez, A., Chan, C.M. and Kaspar, B.K. (2009). Intravascular AAV9 preferentially targets neonatal neurons and adult astrocytes. Nat. Biotech. 27, 59-65.
4. Pearson TS, Akman C, Hinton VJ, Engelstad K, De Vivo DC. (2013) Phenotypic spectrum of glucose transporter type 1 deficiency syndrome (Glut1 DS). Curr. Neurol. Neurosci. Rep. 13, 342.
5. De Giorgis V and Veggiotti, P. (2013) Glut1 deficiency syndrome 2013: Current state of the art. Seizure 22, 803-811.
6. Wang D, Pascual JM, Yang H, Engelstad K, Mao X, Cheng J, Yoo J, Noebels JL, De Vivo DC (2006) A mouse model for Glut-1 haploinsufficiency. Hum. Mol. Genet. 15, 1169-1179.
7. Mueckler M, Caruso C, Baldwin SA, Panico M, Blench I, Morris HR, Allard WJ, Lienhard GE, Lodish HF (1985) Sequence and structure of a human glucose transporter. Science 229, 941-945.
8. Seidner G, Alvarez MG, Yeh JI, O'Driscoll KR, Klepper J, Stump TS, Wang D, Spinner NB, Birnbaum MJ, De Vivo DC (1998) GLUT-1 deficiency syndrome caused by haploinsufficiency of the blood-brain barrier hexose carrier. Nat Genet. 18, 188-191.
9. Yang H, Wang D, Engelstad K, Bagay L, Wei Y, Rotstein M, Aggarwal V, Levy B, Ma L, Chung WK, De Vivo DC (2011) Glut1 deficiency syndrome and erythrocyte glucose uptake assay. Ann Neurol 70, 996-1005.
10. Grieger JC, Samulski RJ (2005) Adeno-associated virus as a gene therapy vector: vector development, production and clinical applications. Adv Biochem Eng Biotechnol. 99, 119-145.
11. Grieger JC, Samulski RJ (2012) Adeno-associated virus vectorology, manufacturing, and clinical applications. Methods Enzymol. 507, 229-254.
12. Kariya S, Re DB, Jacquier A, Nelson K, Przedborski S, Monani UR (2012) Mutant superoxide dismutase 1 (SOD1), a cause of amyotrophic lateral sclerosis, disrupts the recruitment of SMN, the spinal muscular atrophy protein to nuclear Cajal bodies. Hum Mol Genet. 21,3421-3434.
13. Foust KD, Wang X, McGovern VL, Braun L, Bevan AK, Haidet AM, Le TT, Morales PR, Rich MM, Burghes AH, Kaspar BK (2010) Rescue of the spinal muscular atrophy phenotype in a mouse model by early postnatal delivery of SMN. Nat. Biotech. 28, 271-274.
14. Fleming JO, Ting JY, Stohlman SA, Weiner LP (1983) Improvements in obtaining and characterizing mouse cerebrospinal fluid. Application to mouse hepatitis virus-induced encephalomyelitis. J Neuroimmunol. 4,129-140.
15. Gao, G.P., and Sena-Esteves, M. (2012). Introducing Genes into Mammalian Cells: Viral Vectors. In Molecular Cloning, Vol 2: A Laboratory Manual (M.R. Green and J. Sambrook eds.) pp. 1209-1313. Cold Spring Harbor Laboratory Press, New York.
16. Rapti K, Louis-Jeune V, Kohlbrenner E, Ishikawa K, Ladage D, Zolotukhin S, Hajjar RJ, Weber (2012) Neutralizing antibodies against AAV serotypes 1, 2, 6, and 9 in sera of commonly used animal models. Mol. Ther. 20, 73-83.
17. Goulder PJ, Addo MM, Altfeld MA, Rosenberg ES, Tang Y, Govender U, Mngqundaniso N, Annamalai K, Vogel TU, Hammond M, Bunce M, Coovadia HM, Walker BD (2001) Rapid definition of five novel HLA-A*3002-restricted human immunodeficiency virus-specific cytotoxic T-lymphocyte epitopes by elispot and intracellular cytokine staining assays. J. Virol. 75, 1339-1347.

## Claims

1. A recombinant adeno-associated vector (rAAV) comprising a nucleic acid sequence comprising a transgene encoding Glut1 operably linked to a chicken Beta-actin promoter and wherein the rAAV is capable of crossing the blood-brain barrier (BBB), and wherein the AAV is AAV8 or AAV9.

2. The recombinant AAV of claim 1, wherein the rAAV comprising the transgene targets the endothelial cells lining the brain microvasculature, thereby allowing the transgene to be expressed in endothelial cells lining the brain microvasculature.

3. The recombinant AAV of claim 1, wherein the chicken Beta-actin promoter is selected from the group consisting of SEQ ID NO:31, 38, 45, 54, 62, and 70.

4. The recombinant AAV of claim 1, wherein the Glut1 comprises SEQ ID NO:78 or 79.

5. The recombinant AAV of claim 1, further comprising miRNA elements selected from the group consisting of SEQ ID NO:48, 56, 59, 64, and 73.

6. The recombinant AAV of claim 1, wherein the recombinant vector further comprises inverted terminal repeats (ITRs) flanking the miRNA elements.

7. A composition comprising the recombinant AAV of any of claims 1 to 6 and further comprising a pharmaceutical carrier.

8. A kit comprising a container housing comprising the composition of claim 7.

9. The kit of claim 8, wherein the container is a syringe.

10. The recombinant AAV of any one of claims 1-6 or the composition of claim 7 for use in treating Glut1 deficiency syndrome in a subject in need thereof, wherein the use comprises administering to the subject an effective amount of the recombinant AAV vector of any one of claims 1-6 or the composition of claim 7.

11. The recombinant AAV of any one of claims 1-6 or the composition of claim 7 for use alleviating in a subject, at least one of the symptoms associated with Glut1 deficiency syndrome selected from the group consisting of hypoglycorrhachia, acquired microcephaly, ataxic and dystonic motor dysfunction, wherein the use comprises administering to the subject an effective amount of the recombinant AAV vector of any one of claims 1-6 or the composition of claim 7.

## Patentansprüche

1. Rekombinanter Adeno-assoziierter Vektor (rAAV), umfassend eine Nukleinsäuresequenz, umfassend ein Transgen, das für Glut1 codiert, das funktionsfähig mit einem Huhn-Beta-Actin-Promotor verbunden ist und wobei der rAAV die Blut-Hirn-Schranke (BBB) überschreiten kann und wobei der AAV AAV8 oder AAV9 ist.

2. Rekombinanter AAV nach Anspruch 1, wobei der das Transgen umfassende rAAV auf die Endothelzellen abzielt, die die Mikrovaskulatur des Gehirns auskleiden, wobei dadurch ermöglicht wird, dass das Transgen in Endothelzellen exprimiert wird, die die Mikrovaskulatur des Gehirns auskleiden.

3. Rekombinanter AAV nach Anspruch 1, wobei der Huhn-Beta-Actin-Promotor ausgewählt ist aus der Gruppe bestehend aus SEQ ID NO: 31, 38, 45, 54, 62 und 70.

4. Rekombinanter AAV nach Anspruch 1, wobei das Glut1 SEQ ID NO: 78 oder 79 umfasst.

5. Rekombinanter AAV nach Anspruch 1, ferner umfassend miRNA-Elemente, ausgewählt aus der Gruppe bestehend aus SEQ ID NO: 48, 56, 59, 64 und 73.

6. Rekombinanter AAV nach Anspruch 1, wobei der rekombinante Vektor ferner invertierte terminale Wiederholungen (ITRs) umfasst, die die miRNA-Elemente flankieren.

7. Zusammensetzung, umfassend den rekombinanten AAV nach einem der Ansprüche 1 bis 6 und ferner umfassend einen pharmazeutischen Träger.

8. Kit, umfassend ein Behältergehäuse, umfassend die Zusammensetzung nach Anspruch 7.

9. Kit nach Anspruch 8, wobei der Behälter eine Spritze ist.

10. Rekombinanter AAV nach einem der Ansprüche 1 bis 6 oder Zusammensetzung nach Anspruch 7 für die Verwendung bei der Behandlung des Glut1-Mangel-Syndroms bei einem Subjekt, das dies benötigt, wobei die Verwendung das Verabreichen einer wirksamen Menge des rekombinanten AAV-Vektors nach einem der Ansprüche 1 bis 6 oder der Zusammensetzung nach Anspruch 7 an das Subjekt umfasst.

11. Rekombinanter AAV nach einem der Ansprüche 1 bis 6 oder Zusammensetzung nach Anspruch 7 für die Verwendung für die Linderung wenigstens eines der Symptome, die mit dem Glut1-Mangel-Syndrom verbunden sind, in einem Subjekt, ausgewählt aus der Gruppe bestehend aus Hypoglykorrhachie, erworbener Mikrozephalie, ataxischer und dystonischer motorischer Funktionsstörung, wobei die Verwendung das Verabreichen einer wirksamen Menge des rekombinanten AAV-Vektors nach einem der Ansprüche 1 bis 6 oder der Zusammensetzung nach Anspruch 7 an das Subjekt umfasst.

## Revendications

1. Vecteur adénoassocié recombinant (rAAV) comprenant une séquence d'acides nucléiques comprenant un transgène codant pour Glut1 lié de manière opérationnelle à un promoteur de bêta-actine de poulet et dans lequel le rAAV est capable de traverser la barrière hématoencéphalique (BBB), et dans lequel l'AAV est l'AAV8 ou l'AAV9.

2. AAV recombinant selon la revendication 1, dans lequel le rAAV comprenant le transgène cible les cellules endothéliales tapissant la microvascularisation cérébrale, permettant ainsi au transgène d'être exprimé dans les cellules endothéliales tapissant la microvascularisation cérébrale.

3. AAV recombinant selon la revendication 1, dans lequel le promoteur de bêta-actine de poulet est choisi dans le groupe constitué par la SEQ ID NO : 31, 38, 45, 54, 62, et 70.

4. AAV recombinant selon la revendication 1, dans lequel le Glut1 comprend la SEQ ID NO : 78 ou 79.

5. AAV recombinant selon la revendication 1, comprenant en outre des éléments d'ARNmi choisis dans le groupe constitué par la SEQ ID No : 48, 56, 59, 64, et 73.

6. AAV recombinant selon la revendication 1, dans lequel le vecteur recombinant comprend en outre des répétitions terminales inversées (ITR) flanquant les éléments d'ARNmi.

7. Composition comprenant l'AAV recombinant selon l'une quelconque des revendications 1 à 6 et comprenant en outre un support pharmaceutique.

8. Kit comprenant un boîtier de récipient comprenant la composition selon la revendication 7.

9. Kit selon la revendication 8, dans lequel le récipient est une seringue.

10. AAV recombinant selon l'une quelconque des revendications 1 à 6 ou composition selon la revendication 7 destiné à être utilisé dans le traitement du syndrome de carence en Glut1 chez un sujet en ayant besoin, dans lequel l'utilisation comprend l'administration au sujet d'une quantité efficace de vecteur recombinant AAV selon l'une quelconque des revendications 1 à 6 ou composition selon la revendication 7.

11. AAV recombinant selon l'une quelconque des revendications 1 à 6 ou composition selon la revendication 7 destiné à être utilisé dans le soulagement d'un sujet, au moins l'un des symptômes associés au syndrome de carence en Glut1 est choisi dans le groupe constitué par l'hypoglycorrhachie, la microcéphalie acquise, le dysfonctionnement moteur ataxique et dystonique, dans lequel l'utilisation comprend l'administration au sujet d'une quantité efficace de vecteur recombinant AAV selon l'une quelconque des revendications 1 à 6 ou composition selon la revendication 7.
